# EUROPEAN PATENT SPECIFICATION

(11) **EP 3 060 924 B1**
(45) Date of publication and mention of the grant of the patent: **01.06.2022**
(21) Application number: 14855381.1
(22) Date of filing: 22.10.2014
(51) Int. Cl.: G01N 33/68, C07K 7/06, A61K 38/00

(54) **PIF-TRANSFECTED CELLS AND METHODS OF USE**
PIF-TRANSFIZIERTE ZELLEN UND VERFAHREN ZUR VERWENDUNG
CELLULES TRANSFECTÉES PAR UN PIF ET PROCÉDÉS D'UTILISATION

(30) Priority: 22.10.2013 US 201361894274 P; 24.10.2013 US 201361895088 P
(43) Date of publication of application: 31.08.2016
(73) Proprietor: Bioincept LLC, Cherry Hill, NJ 08003 (US)
(72) Inventor: BARNEA, Eytan R., Cherry Hill, NJ 08003 (US); HAYRABEDYAN, Soren Bohos, 1784 Sofia (BG)
(74) Representative: Leonard, Thomas Charles
(86) International application number: PCT/US2014/061814
(87) International publication number: WO 2015/061483

(56) References cited:
- WO-A2-03/033644
- WO-A2-2005/040196
- WO-A2-2012/119072
- US-A1- 2007 231 310
- US-A1- 2008 003 178
- US-A1- 2011 112 016
- US-A1- 2012 301 921
- US-A9- 2012 107 318
- CHRISTOPHER W STAMATKIN ET AL: "PreImplantation Factor (PIF) correlates with early mammalian embryo development-bovine and murine models", REPRODUCTIVE BIOLOGY AND ENDOCRINOLOGY, BIOMED CENTRAL LTD, GB, vol. 9, no. 1, 15 May 2011 (2011-05-15), page 63, XP021100711, ISSN: 1477-7827, DOI: 10.1186/1477-7827-9-63
- HAYRABEDYAN SOREN B ET AL: "Structural design-based preimplantation factor (PIF*) fusion peptide synthetic DNA cloning and eukaryote expression aimed for functional proteomic studies and possible chronic immune disorders therapy", JOURNAL OF REPRODUCTIVE IMMUNOLOGY, vol. 101, 1 March 2014 (2014-03-01), page 60, XP028627922, ISSN: 0165-0378, DOI: 10.1016/J.JRI.2013.12.074
- SHAINER, R ET AL.: 'Immune Regulation And Oxidateive Stress Reduction By Preimplantation Factor Following Syngeneic Or Allogeneic Bone Marrow Transplantation.' CONFERENCE PAPERS IN MEDICINE. vol. 2013, 11 March 2013, pages 1 - 8, XP055334794
- WEISS, L ET AL.: 'Preimplantation Factor (PIF*) Reverses Neuroinflammation While Promoting Neural Repair In EAE Model.' JOURNAL OF THE NEUROLOGICAL SCIENCES. vol. 312, 13 October 2011, pages 146 - 157, XP028394165

### SEQUENCE LISTING

<110> BioIncept, LLC
<120> PIF-Transfected Cells and Methods of Use
<130> 120785.02302
<160> 28
<170> PatentIn version 3.5
<210> 1
   <211> 9
   <212> PRT
   <213> Artificial Sequence
<220>
   <223> PIF-1 (9)
<400> 1
   Met Val Arg Ile Lys Pro Gly Ser Ala
   1               5
<210> 2
   <211> 13
   <212> PRT
   <213> Artificial Sequence
<220>
   <223> PIF-1 (13)
<400> 2
   Met Val Arg Ile Lys Pro Gly Ser Ala Asn Lys Pro Ser
   1               5                   10
<210> 3
   <211> 15
   <212> PRT
   <213> Artificial Sequence
<220>
   <223> PIF-1 (15)
<400> 3
   Met Val Arg Ile Lys Pro Gly Ser Ala Asn Lys Pro Ser Asp Asp
   1               5                   10                  15
<210> 4
   <211> 14
   <212> PRT
   <213> Artificial Sequence
<220>
   <223> PIF-1 (14)
<400> 4
   Met Val Arg Ile Lys Tyr Gly Ser Tyr Asn Lys Pro Ser Asp
   1               5                   10
<210> 5
   <211> 18
   <212> PRT
   <213> Artificial Sequence
<220>
   <223> PIF-3 (18)
<400> 5
   Ser Gly Ile Val Ile Tyr Gln Tyr Met Asp Asp Arg Tyr Val Gly Ser
   1               5                   10                  15
   Asp Leu
<210> 6
   <211> 9
   <212> PRT
   <213> Artificial Sequence
<220>
   <223> PIF-4 (9)
<400> 6
   Val Ile Ile Ile Ala Gln Tyr Met Asp
   1               5
<210> 7
   <211> 10
   <212> PRT
   <213> Artificial Sequence
<220>
   <223> PIF-2 (10)
<400> 7
   Ser Gln Ala Val Gln Glu His Ala Ser Thr
   1               5                   10
<210> 8
   <211> 13
   <212> PRT
   <213> Artificial Sequence
<220>
   <223> PIF-2 (13)
<400> 8
   Ser Gln Ala Val Gln Glu His Ala Ser Thr Asn Met Gly
   1               5                   10
<210> 9
   <211> 5
   <212> PRT
   <213> Artificial Sequence
<220>
   <223> PIF-1 (5)
<400> 9
   Met Val Arg Ile Lys
   1               5
<210> 10
   <211> 4
   <212> PRT
   <213> Artificial Sequence
<220>
   <223> PIF-1 (4A)
<400> 10
   Pro Gly Ser Ala
   1
<210> 11
   <211> 4
   <212> PRT
   <213> Artificial Sequence
<220>
   <223> PIF-1 (4B)
<400> 11
   Arg Ile Lys Pro
   1
<210> 12
   <211> 27
   <212> DNA
   <213> Artificial Sequence
<220>
   <223> PIF-1 (9)
<400> 12
   atggtcagga ttaagcctgg ctccgcc 27
<210> 13
   <211> 39
   <212> DNA
   <213> Artificial Sequence
<220>
   <223> PIF-1 (13)
<400> 13
   atggtcagga ttaagcctgg ctccgccaac aagccttcc 39
<210> 14
   <211> 45
   <212> DNA
   <213> Artificial Sequence
<220>
   <223> PIF-1 (15)
<400> 14
   atggtcagga ttaagcctgg ctccgccaac aagccttccg acgac 45
<210> 15
   <211> 15
   <212> DNA
   <213> Artificial Sequence
<220>
   <223> PIF-1 (5)
<400> 15
   atggtcagga ttaag 15
<210> 16
   <211> 12
   <212> DNA
   <213> Artificial Sequence
<220>
   <223> PIF-1 (4A)
<400> 16
   cctggctccg cc 12
<210> 17
   <211> 12
   <212> DNA
   <213> Artificial Sequence
<220>
   <223> PIF-1 (4B)
<400> 17
   aggattaagc ct 12
<210> 18
   <211> 8
   <212> PRT
   <213> Artificial Sequence
<220>
   <223> FLAG
<400> 18
   Asp Tyr Lys Asp Asp Asp Asp Lys
   1 5
<210> 19
   <211> 9
   <212> PRT
   <213> Artificial Sequence
<220>
   <223> HA
<400> 19
   Tyr Pro Tyr Asp Val Pro Asp Tyr Ala
   1 5
<210> 20
   <211> 24
   <212> DNA
   <213> Artificial Sequence
<220>
   <223> FLAG
<400> 20
   gattacaaag atgacgacga caag 24
<210> 21
   <211> 27
   <212> DNA
   <213> Artificial Sequence
<220>
   <223> HA
<400> 21
   tatccttacg atgtgcctga ttatgct 27
<210> 22
   <211> 37
   <212> PRT
   <213> Artificial Sequence
<220>
   <223> FLAG-(Gly)2-PIF-(Gly)2-HA
<400> 22
   Asp Tyr Lys Asp Asp Asp Asp Lys Gly Gly Gly Met Val Arg Ile Lys
   1               5                   10                  15
   Pro Gly Ser Ala Asn Lys Pro Ser Asp Asp Gly Gly Tyr Pro Tyr Asp
               20                  25                  30
   Val Pro Asp Tyr Ala
           35
<210> 23
   <211> 42
   <212> PRT
   <213> Artificial Sequence
<220>
   <223> FLAG-HA-(Gly)6-PIF
<400> 23
   Ala Thr Met Asp Tyr Lys Asp Asp Asp Asp Lys Gly Tyr Pro Tyr Asp
   1               5                   10                  15
   Val Pro Asp Tyr Ala Gly Gly Gly Gly Gly Gly Met Val Arg Ile Lys
               20                  25                  30
   Pro Gly Ser Ala Asn Lys Pro Ser Asp Asp
           35                  40
<210> 24
   <211> 41
   <212> PRT
   <213> Artificial Sequence
<220>
   <223> PIF-(Gly)2-FLAG-HA
<400> 24
   Ala Thr Met Val Arg Ile Lys Pro Gly Ser Ala Asn Lys Pro Ser Asp
   1               5                   10                  15
   Asp Gly Gly Gly Gly Gly Gly Asp Tyr Lys Asp Asp Asp Asp Lys Gly
               20                  25                  30
   Tyr Pro Tyr Asp Val Pro Asp Tyr Ala
           35                  40
<210> 25
   <211> 111
   <212> DNA
   <213> Artificial Sequence
<220>
   <223> FLAG-(Gly)2-PIF-(Gly)2-HA
<400> 25
   gattacaaag atgacgacga caagggaggc ggtatggtca gaatcaagcc tggtagtgct    60
   aacaagccat ccgatgacgg ggggtatcct tacgatgtgc ctgattatgc t            111
<210> 26
   <211> 129
   <212> DNA
   <213> Artificial Sequence
<220>
   <223> FLAG-HA-(Gly)6-PIF
<400> 26
   gccaccatgg actataaaga tgacgatgat aagggatacc catacgacgt ccctgattat    60
   gcgggcgggg gagggggcgg tatggttcgg ataaagcctg gctctgcgaa caaaccctct   120
   gatgattaa                                                           129
<210> 27
   <211> 126
   <212> DNA
   <213> Artificial Sequence
<220>
   <223> PIF-(Gly)2-FLAG-HA
<400> 27
   gccaccatgg tgagaattaa acctgggtcc gcaaacaagc ctagtgacga cggtggcggc        60
   ggaggaggcg actacaagga cgacgacgac aagggctacc cctacgacgt gcccgactac       120
   gcctaa                                                                  126
<210> 28
   <211> 14
   <212> PRT
   <213> Artificial Sequence
<220>
   <223> Linearized representation of the active site of a receptor
<220>
   <221> MISC_FEATURE
   <222> (2)..(2)
   <223> Xaa is any amino acid.
<220>
   <221> MISC_FEATURE
   <222> (4)..(4)
   <223> Xaa is any amino acid.
<220>
   <221> MISC_FEATURE
   <222> (5)..(5)
   <223> Xaa is any amino acid.
<220>
   <221> MISC_FEATURE
   <222> (6)..(6)
   <223> Xaa is any amino acid.
<220>
   <221> MISC_FEATURE
   <222> (11)..(11)
   <223> Xaa is any amino acid.
<220>
   <221> MISC_FEATURE
   <222> (12)..(12)
   <223> Xaa is any amino acid.
<220>
   <221> MISC_FEATURE
   <222> (13)..(13)
   <223> Xaa is any amino acid.
<400> 28
   Trp Xaa Trp Xaa Xaa Xaa Arg Glu Trp Phe Xaa Xaa Xaa Trp
   1               5                   10

## Description

### B. Background

WO 03/033644 A2 describes peptides and polynucleotides, and their use for immunomodulation, immunotherapy and vaccine particularly for anti-cancer therapy, and for diagnosis purposes. Stamatkin et al. 2011 Rep Bio Endo 9(1) describes Prelmplantation Factor (PIF) correlating with early mammalian embryonic development in bovine and murine models. US 2008/003178 A1 describes peptides and peptidomimetics capable of enhance endometrial receptivity, blocking activated but not basal immunity, inhibiting cell proliferation and creating a TH2 type cytokine bias. WO 2005/040196 A2 describes PIF peptide biologic activities, site of action, and the antibody to detect PIF. US 2011/112016 A1 describes PIF peptides that were generated synthetically and were tested on peripheral blood immune cells and shown to block activated but not basal immunity, inhibiting cell proliferation and creating a TH2 type cytokine bias.

### C. Brief summary of the invention

The invention is as set out in the claims. In one aspect, the invention provides a cell comprising an exogenous deoxyribonucleic acid (DNA) sequence encoding a preimplantation factor (PIF) peptide, wherein the PIF peptide consists of the sequence of MX₁RIKPX₂X₃A, wherein X₁, X₂ and X₃ are independently any amino acid, provided that said PIF peptide is not SEQ ID NO: 1, wherein the cell is obtained by a method of modifying the cell to express the exogenous DNA. In another aspect, the invention provides a pharmaceutical composition comprising the cell of the invention and a pharmaceutically acceptable carrier. In another aspect, the invention provides the cell of the invention for use as a medicament.

The present disclosure relates to cells transfected with DNA sequences encoding for a Prelmplantation Factor (PIF) or a PIF and one or more fusion tag(s), DNA sequences encoding for synthetic PIFs, a PIF fusion peptide made of a PIF and one or more fusion tags, methods of treating subjects using the transfected cells that express a PIF, an R-I-K-P peptide, compositions containing the R-I-K-P peptide, and methods of identifying a compound that binds to an active site of the amino acid residue signature WX₁WX₂X₃X₄REWFX₅X₆X₇W (Trp-X-Trp-X-X-X-Arg-Glu-Trp-Phe-X-X-X-Trp). It also provides a novel general method for the seamless expression of other peptides.

### D. Description of Drawings

For a fuller understanding of the nature and advantages of the present invention, reference should be had to the following detailed description taken in connection with the accompanying drawings, in which:
**Figure 1** illustrates PIF sequence codon mapping over H. sapiens BLASTP queried similar proteins sequence alignment.
**Figure 2** shows the amino acid sequences used for the design of peptide fusion variants.
**Figure 3** illustrates a PIF fusion peptides design scenario, peptide nucleotide sequence, peptide diagram, average GC content (DNA2), and GenScript analysis data for Rare Codon Usage Analysis, including Codon Adaptation Index (CAI), the GC content curve and average GC content, and Codon Frequency Distribution.
**Figure 4** illustrates the *ab initio* strategy for assessing fusion protein binding.
**Figure 4A** illustrates a modeling strategy scheme wherein PIF fusion construct amino acids are modeled using several *de novo* approaches, followed by submission to servers for assessment and detection of molecular fluctuations, deep residues and residue accessibility, and the potential molecular locations undergoing readjustment.
**Figure 4B** illustrates the native PIF *ab initio* tertiary structure prediction using the PEP FOLD server.
**Figure 4C** illustrates the hinge region assessment, wherein a molecular surface map of PIF fusion hinged model conformations was created using the I-Tasser server-generated PDBs, and analyzed using the HINGE server.
**Figure 4D** illustrates a protein residue accessibility and structure flexibility assessment.
**Figure 4E** shows a protein residue's accessibility and structure flexibility assessment: *Left:* I-Tasser model of PIF fusion was used to predict amino acid depth and accessibility. The surface model contains water molecules showing the deep hydrophobic pockets. *Right:* The same PDB was used for DEPTH modeling, and the obtained backbone structure was reversed to full model using ModRefiner to conform the subsequent CABS-Flex modeling of the putative flexibility structures. The R-I-K-P sequence is predicted as deeply situated inside the molecule in the FLAG-HA-PIF and PIF-FLAG-HA models.
**Figure 5** illustrates models generated using the method described herein (HINGEProt model submitted to the DEPTH server).
**Figure 6** illustrates a PIF fusion peptide and vector linearization primers design, In-Fusion cloning and in vitro validation.
**Figure 6A** shows the fusion peptide transcript structure, encoding FLAG short linker PIF short linker HA variant, as entered in Vector NTI.
**Figure 6B** illustrates the original Invitrogen pCEP4 vector diagram, depicting MCS, as entered in Vector NTI for *in silico* cloning.
**Figure 6C** shows the vector NTI screen, showing the original MCS and the chosen MCS flanking restriction sites used for the design of linearization primers.
**Figure 6D** illustrates *in silico* reverse PCR: Vector NTI screen, showing 100% alignment between original vector and restriction site sequences of -20bp upstream of Kpn I and +20bp downstream of BamH I in AddGenep sequenced pCEP4-HAHA, where MCS contains two successive HA tags.
**Figure 6E** shows the agarose gel electrophoresis of the synthesized *in silico* designed fusion insert, and the initial pCEP4-HAHA cloning vector.
**Figure 6F** shows the agarose gel electrophoresis of cloned products.
**Figure 6G** shows the confocal immunofluorescence validation of PIF fusion transfection expression.
**Figure 6H** shows the flow cytometry validation of PIF fusion transfection expression.
**Figure 7** shows the primers used for the validation expression of the selected *in silico* optimized model (FLAG-(Gly)₂-PIF-(Gly)₂-HA).
**Figure 8** shows the PIF targets identified by ProtoArray^{®} Human Protein Microarrays v5.0.
**Figure 9** shows the protein structures used for the examination of PIF binding sites.
**Figure 10** shows the bioinformatics deduction of the PIF peptide core sequence that is most likely to participate in binding interfaces. Taverna automated query to PepSite for predicting putative binding amino acids in PF and protein targets derived from experimental ProteoArray protein chip screening are shown. 200 PDBs corresponding to positive hits and more than 2500 PDBs corresponding to negative hits were scored for interaction amino acids pattern search.
**Figure 11** shows statistical overrepresentation of the amino acid position in the PIF sequence.
**Figure 12** shows the naive Bayesian assessment of each amino acid influence for distinguishing between PIF binders and non-binders.
**Figure 13** shows PIF targets structures A-D, wherein cartoon ribbon and surface rendering of the four most prominent PIF targets were identified using ProtoArray^{®} Human Protein Microarrays v5.0.
**Figure 13A** shows the IDE-free format.
**Figure 13B** shows IDE complexed with insulin.
**Figure 13C** shows PDGFb.
**Figure 13D** shows Kvl.3b.
**Figure 14** shows that the R-I-K-P consensus sequence of the PIF peptide is involved in its binding interactions.
**Figure 14A** shows two synthetic images of PIF PDB structures modeled using PepSite, superimposed in transparent mode emphasizing the solid beta sheet PIF structure. The green beta sheet corresponds to a conserved consensus PIF sequence (aligned).
**Figure 14B** shows a table representing PIF amino acid residue configuration with highest probability for four targets binding out of 11 putative targets.
**Figure 15** shows PIF binding surfaces represented by amino acid residues identified using peptide-to-protein binding modeling by the PepSite server of the top 4 protein targets identified using ProtoArray^{®} Human Protein Microarrays v5.0.
**Figure 16** shows PIF alignment with protein targets.
**Figure 16A** shows multiple protein sequence alignment after multiple protein structural alignment and superposition of the Kvl.3 potassium channel, IDE and PDEGF models (Chimera 1.8).
**Figure 16B** shows (from left to right) the multiple protein structural alignment of all four targets imaged as cartooned ribbons colored by protein model ID; only matching structures are in opaque, while non-matching structures are colored dark-gray and transparent; the surface rendering of the superimposed protein structures are colored by model.
**Figure 16C** shows (from left to right) superimposed atom chains of the structures with the highest level of structural alignment; the same structures are superimposed by the surfaces of those residues that match and are colored by the level of conservation, blue being the lowest; the same structures are colored by the RMSD, blue being the lowest distance, and red the highest distance.

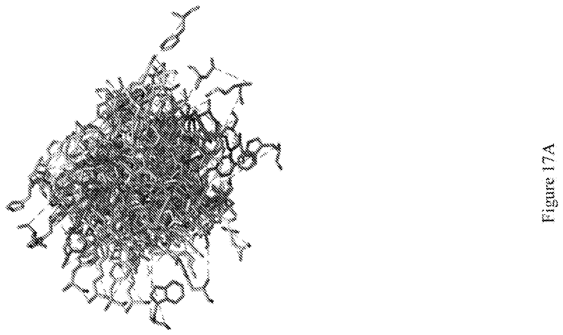

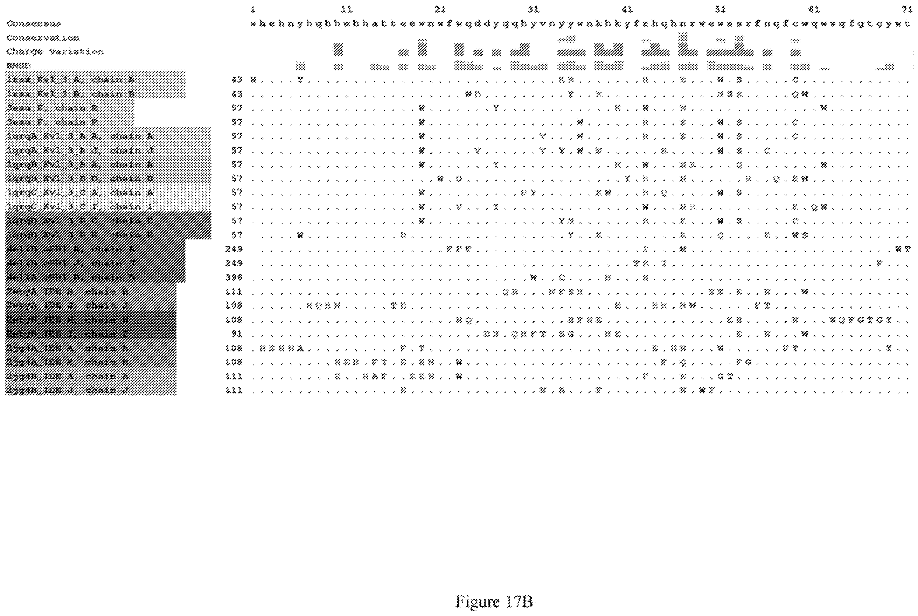

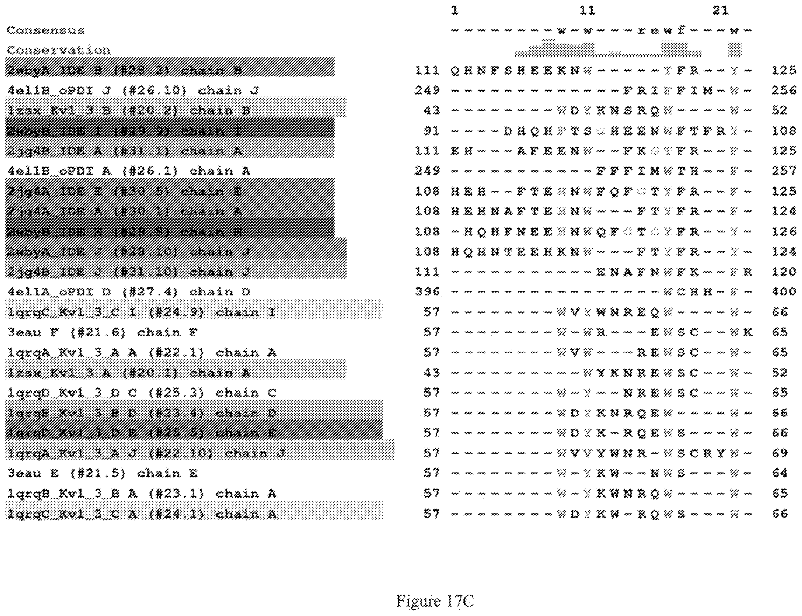

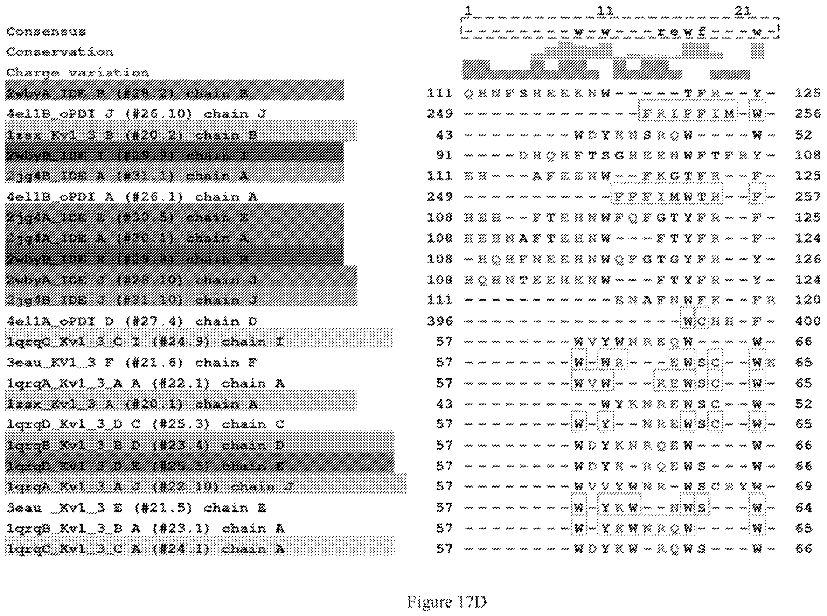

**Figures 17** **A and B** show structural alignment using a Smith-Waterman algorithm with the same parameters over the entire set of models representing all the targets was done using Match-Maker algorithm of UCSF Chimera 1.9. The structural representation is too diverse as it resembled distinct non-homologous proteins that do not share the same amino acid sequences, but rather have similar binding pocket properties in terms of hydrophobicity/electrostatic potential/H-bonds formation/van der Waals forces, etc. **Figures 17C** **and** D show the following signature for the maximally 23 amino acids participating in the PIF binding pocket: (1)--------w-w---rewf---w-(23), where their constellation could be represented at the following relative linear positions: Trp-9, Trp-11, Arg-15, Glu-16, Trp-17, Phe-18, Trp-22.
**Figure 18** shows a PIF binding validation scenario.
**Figure 19** shows a PIF binding validation scenario, with flexible peptide docking using FlexPepDock.
**Figure 20** shows the most affected amino acids that would cause binding interface disruption based on an *in silico* mutagenesis of the PIF peptide-protein targets interface.
**Figure 21** shows the macropain gene upregulation involved in insulin degradation.

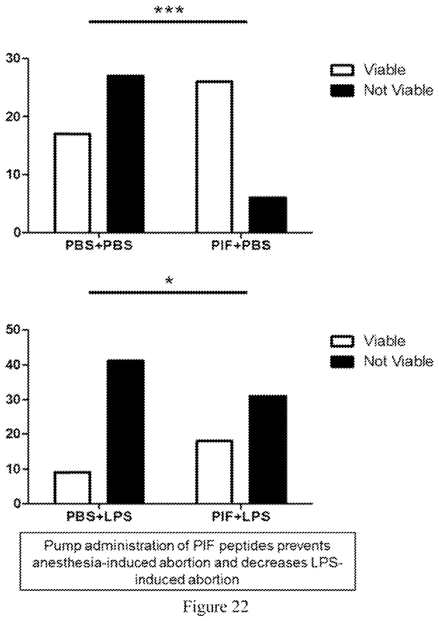

**Figure 22** illustrates that pump administration of PIF peptides prevents anesthesia-induced abortion and decreases LPS-induced abortion. 20 female Swiss mice were successfully inseminated, and were anesthetized at days 1-3 post-insemination (pi). An Alzet pump filled with either PBS or PIF was inserted subcutaneously into each mouse. At days 6-8 pi, the mice were treated with either LPS or PBS. At days 14-16 pi, the mice were euthanized and investigated for vital fetuses and aborts. Mice with no vital fetuses nor signs of abortion were considered to carry 10 aborts for statistical purposes.

### E. Detailed Description

Before the present compositions and methods are described, it is to be understood that this invention is not limited to the particular processes, compositions, or methodologies described, as these may vary. It is also to be understood that the terminology used in the description is for the purpose of describing the particular versions or embodiments only, and is not intended to limit the scope of the present invention which will be limited only by the appended claims.

### F. Definitions

Unless defined otherwise, all technical and scientific terms used herein have the same meanings as commonly understood by one of ordinary skill in the art. Although any methods and materials similar or equivalent to those described herein can be used in the practice or testing of embodiments of the present invention, the preferred methods, devices, and materials arc now described. Nothing herein is to be construed as an admission that the invention is not entitled to antedate such disclosure by virtue of prior invention.

It must also be noted that as used herein and in the appended claims, the singular forms "a," "an," and "the" include plural reference unless the context clearly dictates otherwise. Thus, for example, reference to a "peptide" is a reference to one or more peptides and equivalents thereof known to those skilled in the art, and so forth.

As used herein, the term "about" means plus or minus 10% of the numerical value of the number with which it is being used. Therefore, about 50% means in the range of 45%-55%.

When used in conjunction with a therapeutic, "administering" means to administer a therapeutic directly into or onto a target tissue or to administer a therapeutic to a patient whereby the therapeutic positively impacts the tissue to which it is targeted. The cells of the present invention can be administered in the conventional manner by any method in which they are active.

"Autoimmune" refers to an adaptive immune response directed at self-antigens. "Autoimmune disease" refers to a condition wherein the immune system reacts to a "self antigen that it would normally ignore, leading to destruction of normal body tissues.

As used herein, the term "fusion tag" refers to commonly used "Epitope tag" meaning a short peptide sequence that can be genetically grafted onto a target protein such as a PIF peptide, for which highly specific antibodies exist.

By "tag," we refer herein to an amino acid sequence introduced to the original PIF sequence conferring molecular designation and extra functionality means.

"Immune disorder" refers to abnormal functioning of the immune system.

As used herein, "immune-modulating" refers to the ability of a compound of the present invention to alter (modulate) one or more aspects of the immune system.

"Inflammatory response" or "inflammation" is a general term for the local accumulation of fluid, plasma proteins, and white blood cells initiated by physical injury, infection, or a local immune response.

In some embodiments, the compounds and methods disclosed herein can be utilized with or on a subject in need of treatment, which can also be referred to as "in need thereof." As used herein, the phrase "in need thereof means that the subject has been identified as having a need for the particular method or treatment and that the treatment has been given to the subject for that particular purpose.

As used herein, the term "linker" means short peptide sequences that occur between protein domains.

As used herein, the term "optimal" means the best or most favorable.

As used herein, the terms "peptide," "polypeptide" and "protein" are used interchangeably and refer to two or more amino acids covalently linked by an amide bond or non-amide equivalent.

As used herein, the term "peptide bond" means a covalent chemical bond formed between two molecules when the carboxyl group of one molecule reacts with the amino group of the other molecule.

By "pharmaceutically acceptable," it is meant the carrier, diluent or excipient must be compatible with the other ingredients of the formulation or composition and not deleterious to the recipient thereof.

The term "subject" as used herein includes, but is not limited to, humans and non-human vertebrates and mammals such as wild, domestic and farm animals. Preferably, the term "subject" refers to mammals. More preferably, the term "subject" refers to humans.

As used herein, the term "therapeutic" means an agent utilized to treat, combat, ameliorate, prevent or improve an unwanted condition or disease of a patient. In part, the present disclosure also relates to the treatment of cancer, immune disorders, inflammatory disorders, vascular diseases, transplant, radiation-induced injury, intracellular damage, infection, pregnancy, or the decrease in proliferation of cells.

A "therapeutically effective amount" or "effective amount" of a composition is a predetermined amount calculated to achieve the desired effect, *i.e*., to inhibit, block, or reverse the activation, migration, or proliferation of cells. The activity contemplated by the present methods includes both medical therapeutic and/or prophylactic treatment, as appropriate. The specific dose of a compound administered according to this invention to obtain therapeutic and/or prophylactic effects will, of course, be determined by the particular circumstances surrounding the case, including, for example, the compound administered, the route of administration, and the condition being treated. The compounds are effective over a wide dosage range. It will be understood that the effective amount administered will be determined by the physician in the light of the relevant circumstances including the condition to be treated, the choice of compound to be administered, and the chosen route of administration, and therefore the above dosage ranges are not intended to limit the scope of the invention in any way. A therapeutically effective amount of compound of this invention is typically an amount such that when it is administered in a physiologically tolerable excipient composition, it is sufficient to achieve an effective systemic concentration or local concentration in the tissue.

As used herein, "transfection" and "transfecting" mean delivering a free nucleic acid to a cell by means of transfecting agent.

The terms "treat," "treated," or "treating" as used herein refer to both therapeutic treatment and prophylactic or preventative measures, wherein the object is to prevent or slow down (lessen) or entirely reverse (eradicate) an undesired physiological condition, disorder or disease, or to obtain beneficial or desired clinical results. For the purposes of this invention, beneficial or desired clinical results include, but are not limited to, alleviation of symptoms; diminishment of the extent of the condition, disorder or disease; stabilization (*i.e*., not worsening) of the state of the condition, disorder or disease; delay in onset or slowing of the progression of the condition, disorder or disease; amelioration of the condition, disorder or disease state; remission (whether partial or total), whether detectable or undetectable, or enhancement or improvement of the condition, disorder, or disease; and eradication of the condition, disorder, or disease. Treatment includes eliciting a clinically significant response without excessive levels of side effects. Treatment also includes prolonging survival as compared to expected survival if not receiving treatment.

### PreImplantation Factor Peptides

Mammalian pregnancy is a unique physiological event in which the embryo/fetus interacts with the maternal immune system in a very efficient manner that benefits both parties. The mammalian embryo/allograft may be considered a "perfect transplant" creating a "perfectly homeostatic immune balance." The mother/host tolerates the invading embryo and subsequent fetoplacental unit while preserving maternal immunity, ability to fight infection, and ability to control autoimmune processes. Pregnancy is thus an immune paradox, displaying no graft vs. host (GVH) or host vs. graft (HVG) effect, and regulating immunity, inflammation, and transplantation. Multiple factors have to act synergistically to allow this pregnancy-specific immune paradox. PIF peptides have been discovered to be paramount to this process.

PIF peptides are secreted only by viable embryos, are absent in nonviable embryos, are detected in the circulation of several species of pregnant mammals, and are expressed in the placenta. Natural PIF peptides, synthetic PIF peptides, PIF peptide fragments, and PIF analogs (collectively referred to herein as "PIF peptides") modulate peripheral immune cells and create a favorable immune environment shortly after fertilization. PIF peptides regulate uterine natural killer cell toxicity *in vivo* and allow the implantation of an embryo to the womb without rejection. PIF peptides are thus instrumental in pregnancy maintenance and success, and also have added essential immune-regulatory effects on the body. Without PIF peptides, or when PIF peptide concentrations are low, pregnancy fails.

The following PIF peptides are described in the present disclosure: Met-Val-Arg-Ile-Lys-Pro-Gly-Ser-Ala (SEQ ID NO:1); Met-Val-Arg-Ile-Lys-Pro-Gly-Ser-Ala-Asn-Lys-Pro-Ser (SEQ ID NO:2); Met-Val-Arg-Ile-Lys-Pro-Gly-Ser-Ala-Asn-Lys-Pro-Ser-Asp-Asp (SEQ ID NO:3); Met-Val-Arg-Ile-Lys-Tyr-Gly-Ser-Tyr-Asn-Lys-Pro-Ser-Asp (SEQ ID NO:4); Ser-Gly-Ile-Val-Ile-Tyr-Gln-Tyr-Met-Asp-Asp-Arg-Tyr-Val-Gly-Ser-Asp-Leu (SEQ ID NO:5); Val-Ile-Ile-Ile-Ala-Gln-Tyr-Met-Asp (SEQ ID NO:6); Ser-Gln-Ala-Val-Gln-Glu-His-Ala-Ser-Thr (SEQ ID NO:7); Ser-Gln-Ala-Val-Gln-Glu-His-Ala-Ser-Thr-Asn-Met-Gly (SEQ ID NO: 8); Met-Val-Arg-Ile-Lys (SEQ ID NO:9); Pro-Gly-Ser-Ala (SEQ ID NO: 10); and Arg-Ile-Lys-Pro (SEQ ID NO: 11).

A list of PIF peptide amino acid sequences is provided below in Table 1.

| **TABLE 1. PIF PEPTIDE AMINO ACID SEQUENCES** | | |
|---|---|---|
| **SEQ ID NO** | **Peptide** | **Amino Acid Sequence** |
| SEQ ID NO: 1 Synthetic | PIF-1₍₉₎ | MVRIKPGSA |
| SEQ ID NO:2 Synthetic | PIF-1₍₁₃₎ | MVRIKPGSANKPS |
| SEQ ID NO:3 Synthetic | PIF-1₍₁₅₎ | MVRIKPGSANKPSDD |
| SEQ ID NO:4 Synthetic | PIF-1₍₁₄₎ | MVRIKYGSYNKPSD |
| SEQ ID NO:5 Synthetic | PIF-3₍₁₈₎ | SGIVIYQYMDDRYVGSDL |
| SEQ ID NO:6 Synthetic | PIF-4₍₉₎ | VIIIAQYMD |
| SEQ ID NO:7 Synthetic | PIF-2₍₁₀₎ | SQAVQEHAST |
| SEQ ID NO:8 Synthetic | PIF-2₍₁₃₎ | SQAVQEHASTNMG |
| SEQ ID NO:9 Synthetic | PIF-1₍₅₎ | MVRIK |
| SEQ ID NO:10 synthetic | PIF-1_{(4A)} | PGSA |
| SEQ ID NO:11 synthetic | PIF-1_{(4B)} | RIKP |

| | | |
|---|---|---|
| ()= number of AA | | |

A list of PIF peptide nucleotide sequences is provided below in Table 2.

| **TABLE 2. PIF PEPTIDE NUCLEOTIDE SEQUENCES** | | |
|---|---|---|
| **SEQ ID NO** | **Peptide** | **Nucleotide Sequence** |
| SEQ ID NO:12 synthetic | PIF-l₍₉₎ | ATGGTCAGGATTAAGCCTGGCTCCGCC |
| SEQ ID NO:13 synthetic | PIF-1₍₁₃₎ | |
| SEQ ID NO:14 synthetic | PIF-1₍₁₅₎ | |
| SEQ ID NO:15 synthetic | PIF-1₍₅₎ | ATGGTCAGGATTAAG |
| SEQ ID NO:16 synthetic | PIF-1_{(4A)} | CCTGGCTCCGCC |
| SEQ ID NO: 17 synthetic | PIF-1(4B) | AGGATTAAGCCT |

PIF peptides within the scope of the invention consist of the following sequence: MX₁RIKPX₂X₃A, wherein X₁, X₂ and X₃ are independently any amino acid, provided that said PIF peptide is not SEQ ID NO: 1.

The peptides of the invention include 1- and d-isomers, and combinations of 1- and d-isomers. The peptides can include modifications typically associated with post-translational processing of peptides, for example, cyclization (e.g., disulfide or amide bond), phosphorylation, glycosylation, carboxylation, ubiquitination, myristylation, or lipidation.

### Mechanism of Action of PIF Peptides

The mechanism of action of PIF peptides includes, but is not limited to, reduction of oxidative stress, reduction of protein misfolding, regulation of macrophage migration, regulation of macrophage access to some arteries and/or veins, reduction in macrophage-induced inflammation, prevention of macrophage activation, reduction of macrophage CD68 expression, control of chemotaxis, targeting of CD 14+ cell flow cytometry, targeting CD 14+ cells directly, activation of APC blocks activated-T-cells proliferation (3HThymidine), reduction in NO secretion by LPS-activated macrophages, reduction of macrophage activity and lipid attachment to the aorta in atherosclerosis, prevention of macrophage migration-LPS peritonitis, reduction of neutrophil extravasation-chemical peritonitis, prevention of neutrophil adhesion and rolling in TNFalpha-induced testicular inflammation, modification of MTb-activated macrophage cytokine secretion and/or nitrate release, promotion of B7H1 expression link to T cells, and combinations thereof.

The PIF peptide mechanism of action is unique, not exerted through calcium mobilization or binding to GPCR/Gq receptors seen with immune suppressive drugs (cyclosporine, FK506). PIF peptides have no effect alone or PBMC activated by PHA or PMA/Ionomycin. However, PIF peptides synergize with anti-CD3 mAb to stimulate IL-2 a major product of calcineurin signaling, while also promoting FK506 binding protein upregulation, suggesting a downstream involvement in this pathway. Recent data together with current study implicates K+ channels in the PIF peptide-induced observed effects. PIF peptides bind to Kvl.3 beta-channels that modulate the ion pore and gene expression data demonstrates modulation of K+ genes.

Beyond peripheral immunity, local uterine immunity may also need to be controlled to achieve successful reproduction. Embryo- secreted PIF peptides may be localized within granules of murine uNK cells at the maternal-fetal interface, possibly, targeting intra cellular binding sites. PIF peptides, derived from the surrounding trophoblastic cells, rapidly diffuse into uNK cells. Thus, PIF peptides may downregulate uNK cytotoxic functions or modulate cytokine secretion to support the conceptus' survival. At the point when direct embryo-maternal interaction takes place during implantation, PIF peptides play a significant role in concert with other pro-implantation elements.

PIF peptides may have an essential orchestrating role in creating a perfect immune balance, through a contradictory "immune paradox" profile that is only observed in pregnancy. The increase in TH2 cytokines coupled with binding to Treg cells may protect the embryo while the increase in TH1 cytokines under challenge may protect against infection. PIF peptide expression shortly post-fertilization may reflect its dominant role in early pregnancy events.

### Example of Embodiments of the Invention - Transfected Cell

Some embodiments of the invention are directed to a transfected cell as set out in the claims. A cell is transfected with a deoxyribonucleic acid (DNA) sequence encoding for a PIF peptide. In some embodiments, the cell is a mammalian cell. In one embodiment, the cells are human embryonic kidney 293 cells (HEK293).. PIF SEQ ID:3 was used for experimental validation. In the invention, the peptide is MX₁RIKPX₂X₃A, wherein X₁, X₂, and X₃ are independently any amino acid, provided that said peptide is not MVRIKPGSA (SEQ ID NO: 1).

In other embodiments, the DNA sequence encodes for a PIF peptide with one or more fusion tags attached. A fusion tag is used for the purposes of affinity purification techniques (affinity tags), mediated isolation of a specific protein target from a crude biological source, solubilization of proteins expressed in chaperone-deficient hosts (solubilization tags), enhancement of chromatographic separation resolution of proteins (chromatography tags), in conditions when antibody-mediated protein detection is required, but no specific antibody exists, and epitope tagging is used to follow the protein expression and localization. A fusion tag may be selected for use by the purpose of its application, the hydrophobicity properties of the fusion target and the tag, and by experimental validation of its fusion product expression. The validation requires testing for toxicity, solubility, significant conformational changes detected by specific antibody and fusion protein binding to a known existing target, and/or functional validation of the maintenance of the biological effect after adding the tag.

Examples of fusion tags include, but are not limited to, FLAG^{™}, Human influenza hemagglutinin (HA), myc tag, which is a ten amino acid peptide tag derived from the c-myc gene product, and Strep-tag^{®}, which is an eight amino acid peptide tag binding Strep-Tactin, an engineered for high affinity version of streptavidin. All tags listed here can be N- or C- terminally fused.

A list of amino acid sequences for the fusion tags FLAG and HA, used in some embodiments of this invention, is shown in Table 3:

| **TABLE 3. FUSION TAG AMINO ACID SEQUENCES** | | |
|---|---|---|
| **SEQ ID NO** | **Fusion Tag** | **Amino Acid Sequence** |
| SEQ ID NO: 18 | FLAG | DYKDDDDK |
| SEQ ID NO: 19 | HA | YPYDVPDYA |

A list of nucleotide sequences for the fusion tags FLAG and HA is shown in Table 4:

| **TABLE 4. FUSION TAG NUCLEOTIDE SEQUENCES** | | |
|---|---|---|
| **SEQ ID NO** | **Fusion Tag** | **Nucleotide Sequence** |
| SEQ ID NO:20 | FLAG | GATTACAAAGATGACGACGACAAG |
| SEQ ID NO:21 | HA | TATCCTTACGATGTGCCTGATTATGCT |

In some embodiments, for the purposes of proteomics-based PIF peptide signaling studies, there is a first fusion tag and a second fusion tag. In other embodiments, the first fusion tag and the second fusion tag have amino acids sequence independently selected from the following: SEQ ID NO: 18 and SEQ ID NO: 19. In another embodiment, the first fusion tag and the second fusion tag have nucleotide sequences independently selected from the following: SEQ ID NO:20 and SEQ ID NO:21. In another embodiment, the DNA sequence encodes for a peptide having a structure selected from the following: (a) N-terminally fused first fusion tag - PIF - C-terminally second fusion tag, (b) N-terminally fused first fusion tag - second fusion tag - PIF, (c) N-terminally fused second fusion tag -first fusion tag - PIF, and (d) PIF - first fusion tag - C-terminally fused second fusion tag. In a preferred embodiment, the first fusion tag has an amino acid sequence of SEQ NO: 18 and the corresponding nucleotide sequence of SEQ NO:20, and the second fusion tag has an amino acid sequence of SEQ NO: 19 and the corresponding nucleotide sequence of SEQ NO:21. In some embodiments, the fusion tag(s) are connected to a PIF peptide by a linker. Linkers are often composed of flexible residues like glycine and serine so that the adjacent protein domains are free to move relative to one another. Longer linkers are used when it is necessary to ensure that two adjacent domains do not sterically interfere with one another. Linkers could be also linear or helical. In some embodiments, the linker is one of: a glycine linker having a structure of (Gly)ₙ, where n is an integer of 1 to 8, inclusive, or a glycine-serine linker. The linker, in some embodiments, is a short linker - two repeat Glycine linker (Gly₂), or long linker - six repeat Glycin linker (Gly6). Other options include, but are not limited to Glycine-Serine linker (Gly-Gly-Gly-Gly-Ser)ₙ and α-helix-forming peptide linkers, such as Ala(Glu-Ala-Ala-Ala-Lys)nAla, (n = 2-5), providing structure flexibility, improved protein stability, or increased biological activity. The linker for PIF fusion was chosen to be flexible, stable and most importantly, short. The linkers are covalently linked to the PIF peptide core sequence by means of the peptide bond.

A list of amino acid sequences and nucleotide sequences for some exemplary PIF fusion peptides is shown in Table 5:

| **TABLE 5. PIF FUSION PEPTIDE SEQUENCES** | | |
|---|---|---|
| **PIF Fusion Peptide Construct** | **Amino Acid Sequence** | **Nucleotide Sequence** |
| **FLAG-(Gly)₂-PIF-(Gly)₂-HA** | | |
| **FLAG-HA-(Gty)₆-PIF** | | |
| **PIF-(Gly)₂-FLAG-HA** | | |

### Exemplary Embodiments of the Invention - DNA Sequence Encoding for a Synthetic PIF Peptide (for reference only)

The present disclosure also relates to a DNA sequence encoding for a synthetic PIF peptide. In one embodiment, the synthetic PIF peptide has an amino acid sequence selected from the following: SEQ ID NO:1, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO: 10, and SEQ ID NO: 11. In another embodiment, the synthetic PIF peptide has a nucleotide sequence selected from the following: SEQ ID NO: 12, SEQ ID NO: 13, SEQ ID NO: 14, SEQ ID NO: 15, SEQ ID NO: 16, and SEQ ID NO: 17. In other embodiments, the DNA encodes for a PIF peptide with one or more fusion tags attached. In some embodiments, the fusion tag(s) have an amino acid sequence selected from the following: SEQ ID NO: 18 and SEQ ID NO: 19. In some embodiments, the fusion tag(s) have a nucleotide sequence selected from the following: SEQ ID NO:20 and SEQ ID NO:21. In some embodiments, there is a first fusion tag and a second fusion tag. In some embodiments, the first fusion tag and the second fusion tag have an amino acid sequence independently selected from the following: SEQ ID NO: 18 and SEQ ID NO: 19. In some embodiments, the first fusion tag and the second fusion tag have nucleotide sequences independently selected from the following: SEQ ID NO:20 and SEQ ID NO:21. In another embodiment, the DNA sequence encodes for a peptide having a structure selected from the following: (a) N-terminally fused first fusion tag - PIF - C-terminally fused second fusion tag, (b) N-terminally fused first fusion tag - second fusion tag - PIF, (c) N-terminally fused second fusion tag - first fusion tag - PIF, and (d) PIF - first fusion tag - C-terminally fused second fusion tag. In a preferred embodiment, the first fusion tag has an amino acid sequence of SEQ ID NO: 18 and/or a nucleotide sequence of SEQ ID NO:20, and the second fusion tag has an amino acid sequence of SEQ ID NO: 19 and/or the corresponding nucleotide sequence of SEQ ID NO:21. In some embodiments, the fusion tag(s) are connected to the PIF peptide by a linker. In some embodiments, the linker is one of: a glycine linker having a structure of (Gly)ₙ, where n is an integer of 1 to 8, inclusive, or a glycine-serine linker. The linker, in some embodiments, is a short linker - two repeat Glycine linker (Gly₂), or long linker - six repeat Glycin linker (Gly₆). Other options include, but are not limited to Glycine-Serine linker (Gly-Gly-Gly-Gly-Ser)ₙ and α-helix-forming peptide linkers, such as Ala(Glu-Ala-Ala-Ala-Lys)nAla, (n = 2-5), providing structure flexibility, improved protein stability, or increased biological activity. The linker for PIF fusion was chosen to be flexible, stable and most importantly short. The linkers are covalently linked to the PIF peptide core sequence by the means of peptide bond.

### Exemplary aspects - Prelmplantation Factor Fusion Peptide (for reference only)

The present disclosure also relates to a PIF fusion peptide. The PIF fusion peptide consists of a PIF peptide with one or more fusion tags attached. In one embodiment, the PIF peptide of the PIF fusion peptide has an amino acid sequence selected from the following: SEQ ID NOT, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO: 10, and SEQ ID NO:11. In some embodiments, the PIF peptide of the PIF fusion peptide has a nucleotide sequence selected from the following: SEQ ID NO: 12, SEQ ID NO: 13, SEQ ID NO: 14, SEQ ID NO:15, SEQ ID NO: 16, and SEQ ID NO:17. In some embodiments, the fusion tag(s) have an amino acid sequence selected from the following: SEQ ID NO: 18 and SEQ ID NO: 19. In some embodiments, the fusion tag(s) have a nucleotide sequence selected from the following: SEQ ID NO:20 and SEQ ID NO:21. In some embodiments, there is a first fusion tag and a second fusion tag. In some embodiments, the first fusion tag and the second fusion tag have an amino acid sequence independently selected from the following: SEQ ID NO: 18 and SEQ ID NO: 19. In some embodiments, the first fusion tag and the second fusion tag have a nucleotide sequence independently selected from the following: SEQ ID NO:20 and SEQ ID NO:21. In another embodiment, the PIF fusion peptide has a structure selected from the following: (a) N-terminally fused first fusion tag - PIF - C-terminally fused second fusion tag, (b) N-terminally fused first fusion tag - second fusion tag - PIF, (c) N-terminally fused second fusion tag - first fusion tag - PIF, and (d) PIF - first fusion tag - C-terminally fused second fusion tag. In a preferred embodiment, the first fusion tag has an amino acid sequence of SEQ ID NO: 18 and the corresponding nucleotide sequence of SEQ ID NO:20, and the second fusion tag has an amino acid sequence of SEQ ID NO: 19 and the corresponding nucleotide sequence of SEQ ID NO:21. In some embodiments, the fusion tag(s) are connected to the PIF peptide by a linker. In some embodiments, the linker is one of: a glycine linker having a structure of (Gly)ₙ, where n is an integer of 1 to 8, inclusive, or a glycine-serine linker. The linker, in some embodiments, is a short linker - two repeat Glycine linker (Gly₂), or long linker - six repeat Glycine linker (Gly₆). Other options include, but are not limited to Glycine-Serine linker (Gly-Gly-Gly-Gly-Ser)ₙ and α-helix-forming peptide linkers, such as Ala(Glu-Ala-Ala-Ala-LysnAla, (n = 2-5), providing structure flexibility, improved protein stability, or increased biological activity. The linker for PIF fusion was chosen to be flexible, stable, and most importantly, short. The linkers are covalently linked to the PIF peptide core sequence by peptide bonds.

### Exemplary aspects - Methods of Expressing a PIF Peptide in One or More Cells (for reference only)

The present disclosure also relates to a method of expressing a PIF peptide in one or more cells by transfecting the cell(s) with a DNA sequence encoding for a PIF peptide. In one embodiment, the DNA sequence encodes for a PIF peptide with at least one fusion tag. In some embodiments, the one or more fusion tag(s) do not influence the function, structure, and/or immunogenicity of the PIF peptide.

The present disclosure also relates to a method of expressing a PIF peptide in one or more cells by transfecting the one or more cells with a DNA sequence encoding for a PIF peptide. In other embodiments, the method of expressing a PIF peptide in one or more cells by transfecting the one or more cells with a DNA sequence includes where the DNA sequence encodes for a PIF peptide with at least one fusion tag attached. In some embodiments, the fusion tag(s) does not influence the peptide's function, structure, or immunogenicity.

The present disclosure also relates a method of expressing a PIF peptide in one or more cells includes the following steps: selecting a PIF peptide for expression in one or more cells; translating the amino acid sequence of the PIF peptide to determine one or more corresponding nucleotide sequences encoding for the PIF peptide; analyzing the nucleotide sequence(s) encoding for the PIF peptide to determine one or more optimal nucleotide sequence(s); analyzing the physical structure of the optimal nucleotide sequence(s) of the PIF peptide to determine the one optimal PIF peptide nucleotide sequence; producing a DNA sequence of the one optimal PIF peptide nucleotide sequence; and transfecting one or more cells with the DNA sequence of the one optimal PIF peptide nucleotide sequence.

The present disclosure also relates to a method of expressing a PIF peptide in one or more cells includes the following steps: selecting a PIF peptide for expression in one or more cells; translating the amino acid sequence of the PIF peptide to determine one or more corresponding nucleotide sequences encoding for the PIF peptide; analyzing the nucleotide sequence(s) encoding for the PIF peptide to determine an optimal nucleotide sequence; creating one or more PIF fusion peptides, wherein the PIF fusion peptide(s) consist of a PIF peptide and at least one fusion tag; analyzing the physical structure of the PIF fusion peptide(s) to determine an optimal PIF fusion peptide; producing a DNA sequence encoding for the optimal PIF fusion peptide, wherein the DNA sequence includes the optimal nucleotide sequence; and transfecting one or more cells with the DNA sequence encoding for the optimal PIF fusion peptide. These steps are discussed more in detail below:

**Select a PIF peptide for expression in one or more cells** - A PIF peptide is selected based on the desired characteristic(s), for instance, but not limited to, physiologic activity, receptor binding, and structure.

**Translate the amino acid sequence of the PIF peptide to determine one or more corresponding nucleotide sequences encoding for the PIF peptide** - The amino acid sequence of the PIF peptide is back-translated using the known nucleic acid codon to amino-acid correspondence.

**Analyze the nucleotide sequence(s) encoding for the PIF peptide to determine an optimal nucleotide sequence** - The amino acid codon(s) optimal for H. sapiens translation is selected from all possible codons encoding particular amino acids. There is an H. sapiens-specific tRNA represented codon pool. Codon usage is optimized to prevent tRNA depletion. Local GC content is then optimized.

**Create one or more PreImplantation Factor fusion peptides, wherein the PreImplantation Factor fusion peptide(s) consist of a PIF peptide and at least one fusion tag** - A fusion gene *in silico* design using DNA 2.0 for amino acid arrangement is used, followed by selection of subsequent alternative corresponding codons.

Analyze the physical structure of the **PreImplantation** Factor fusion peptide(s) to determine an optimal **PreImplantation** Factor fusion peptide - The physical structure of the PIF fusion peptide is analyzed using a combination of *ab intio* molecular structural simulation and subsequent *in silico* molecular fluctuability (HINGEProt and CABS Flex servers) and solubility assessment (DEPTH server).

**Produce a DNA sequence encoding for the optimal PreImplantation Factor fusion peptide, wherein the DNA sequence includes the optimal nucleotide sequence** - A software-mediated generation of an end-nucleotide fusion sequence and necessary primers for reverse PCR vector linearization is completed, followed by In-Fusion^{™} gene cloning and PCR fusion product amplification using In-Fusion Clontech design software (online), and the Vector NTI software package.

**Transfect one or more cells with the DNA sequence encoding for the optimal PreImplantation Factor fusion peptide** - One or more cells are transfected using an optimized transfection protocol for Atractene (Qiagen) transfection.

### Exemplary aspects - Methods of Treating a Subject (for reference only)

The present disclosure also relates to a method of treating a condition by administering a therapeutically effective amount of cells modified to express a PIF peptide to a subject in need thereof, where the subject has the condition to be treated. In one embodiment, the PIF peptide has an amino acid sequence selected from the following: SEQ ID NOT, SEQ ID NO:2, SEQ ID NO:3, SEQ ID NO:4, SEQ ID NO:5, SEQ ID NO:6, SEQ ID NO:7, SEQ ID NO:8, SEQ ID NO:9, SEQ ID NO: 10, and SEQ ID NO:11. In one embodiment, the PIF peptide has a nucleotide sequence selected from the following: SEQ ID NO: 12, SEQ ID NO: 13, SEQ ID NO: 14, SEQ ID NO: 15, SEQ ID NO: 16, and SEQ ID NO: 17.

The cells modified to express a PIF peptide can be administered to a subject in need thereof by any method known in the art. Administration may be systemic or local. Some cells may be administered via stereotactic implantation, a minimally invasive form of surgical intervention which makes use of a three-dimensional coordinate system to locate small targets inside the body. Administration may also be by intravenous implantation. Other parenteral routes may be used to administer cells modified to express a PIF peptide, such as injection through subcutaneous or intramuscular methods. Administration may be accomplished intravenously, intraarterially, intramuscularly, subcutaneously, transdermally, intratracheally, intrathecally, intraperitoneally, intranasally, or into spinal fluid, and by other routes of administration. Cells modified to express a PIF peptide may be administered at the desired site of action or proximal to the desired site of action. Cells may also be contained in a matrix, such as but not limited to a collagen gel, that is administered in any of the above ways.

### Pregnancy-Related Methods of Treatment

### Embryo, Endometrial Receptivity, and Trophoblast Invasion

Embryo-maternal cross talk is essential for the establishment of normal pregnancy, and as such is critical at the earliest stages post-fertilization. At that time, contact between the embryo (segregated in the zona-pellucida) and the maternal environment is still limited. Thus, the pre-implantation period could be perceived as silent, since the maternal system would still be unaware of the embryo's presence. However, data indicates that after fertilization and before implantation, embryo-maternal dialogue, which is critical for the impending pregnancy, develops.

PIF peptides are embryo-specific proteins secreted by viable mammalian embryos starting at the two-cell stage and continuing throughout a viable pregnancy, and are absent in non-viable embryos. PIF levels correlate with embryo viability. PIF peptides exhibit a direct autotrophic effect, promote embryo development in culture, and negate maternal serum-induced toxicity derived from patients with a history of recurrent pregnancy loss (RPL). PIF peptides are expressed by the placenta and are present in maternal circulation until the pregnancy is full-term. PIF peptides act mainly on activated immunity, creating a Th2/Thl bias and thus modulating the maternal immune system without immune suppression. PIF peptides condition the intrauterine environment, particularly the sex steroid-primed human endometrial stromal cells and the first-trimester decidua, by enhancing embryo implantation and providing support for the embryo. PIF's effect is exerted by modulating local immunity and adhesion, and controlling apoptosis as evidenced by expression of the affected genes and associated tissue's proteome.

PIF peptides are secreted by the embryo shortly after fertilization, and a 5-7 day delay exists until implantation occurs. PIF peptide administration can prime the endometrium during that critical period. PIF peptides promote trophoblast invasion, which is essential for proper placental development independent of epidermal growth factor. PIF peptides play a critical role in creating a receptive environment for the embryo prior to and during implantation.

PIF peptides, through autocrine effects, support embryo development and survival, beginning before implantation. In addition, PIF promotes embryo receptivity, creating a favorable uterine environment by acting on decidualized stromal cells (HESC) and first-trimester decidua. PIF has been demonstrated to have an evolving coordinated pro-receptive effect on the uterine environment, favoring embryo implantation and development. PIF's effect on HESC supports implantation by increasing amphiregulin, epiregulin, and FGFs, while reducing proliferation promoter and betacellulin expression. Mechanistically, PIF's modulating effect on growth factors involves p-MAPK inhibition coupled with increased phosphatase expression.

The present disclosure also relates to a method of enhancing the implantation of one or more embryos in a subject by administering a therapeutically effective amount of cells modified to express a PIF peptide to a subject in need thereof.

The present disclosure also relates to a method of increasing endometrial receptivity in a subject by administering a therapeutically effective amount of cells modified to express a PIF peptide to a subject in need thereof.

The present disclosure also relates to a method of promoting trophoblast invasion by administering a therapeutically effective amount of cells modified to express a PIF peptide to a subject in need thereof.

### In-Vitro Fertilization (IVF) and Repeat Pregnancy Loss (RPL)

There is a continuous quest to develop targeted and safe treatments for patients with a history of immune-based in-vitro fertilization (IVF) failures and immune-based RPL, particularly for those patients who lose chromosomally normal embryos. Overactive circulating NK cells with elevated cytotoxicity may lead to IVF failures and RPL. PIF peptides block NK cell cytotoxicity and inhibit NK cell activation marker CD69 expression. Thus, PIF peptides act as a rescue factor and block serum toxicity to embryos by increasing blastocyst rates and lowering embryo demise. PIF may protect the embryo against maternally induced hostility.

The present disclosure also relates to a method of in vitro fertilization of a subject involving administering a therapeutically effective amount of cells modified to express a PIF peptide to a subject in need thereof.

The present disclosure also relates to a method of decreasing the incidence of spontaneous miscarriage in a subject by administering a therapeutically effective amount of cells modified to express a PIF peptide to a subject in need thereof.

The present disclosure also relates to a method of maintaining a pregnancy through term in a subject comprising administering a therapeutically effective amount of cells modified to express a PIF peptide to a subject in need thereof.

### Immunity-Related Methods of Treatment - Non-Pregnant Subjects

Some immune disorders are ameliorated during pregnancy, despite the presence of an "immune inflammatory" environment. PIF peptides have been found to be effective in the treatment of immune-related diseases and disorders in non-pregnant subjects. When exposed to pathogens or foreign material, the immune system responds in a rapid and aggressive manner, but no immune response is initiated in cases of anergy, immune-suppressive diseases, such as AIDS, or exposure to immune-suppressive agents. In contrast, in pregnancy the (semi-foreign or foreign) embryo is accepted, nurtured, and earned to term, corresponding to an entirely different type of immune response: embryo/allograft acceptance without maternal/host immune suppression. Such a uniquely balanced state of immune tolerance - acceptance without suppression - is required to protect the embryo/fetus from being rejected while preserving the well-being of both host and offspring. It has been determined that pregnancy is not an immune-suppressed state, but rather a unique environment in which a regulated and controlled state of inflammation prevails.

Specific embryo signaling is critical for the development of maternal tolerance of the embryo/fetus. Autoimmune diseases follow a unique course during pregnancy, and this signaling has an important role in mitigating autoimmune diseases. PIF peptides have been shown to have unique immune-modulatory properties beyond pregnancy, for the prevention of autoimmune and other immune/inflammation-related diseases.

Immune disorders caused by an impaired or compromised immune system can produce a deficient immune response that leaves the body vulnerable to various viral, bacterial, or fungal opportunistic infections. Causes of immune deficiency can include various illnesses such as viruses, chronic illness, or immune system illnesses. Diseases characterized by an impaired immune system include, but are not limited to, HIV/AIDS and severe combined immunodeficiency syndrome (SCIDS).

Immune disorders can be caused by an excessive response by the immune system. This excessive response can be an excessive response to one or more antigens on a pathogen, or to an antigen that would normally be ignored by the immune system. Diseases characterized by an overactive immune system include, but are not limited to, arthritis, allergies, asthma, pollinosis, atopy, and autoimmune diseases. PIF peptides are immune-modulating and can be used to treat various immune disorders.

The immune system functions to protect the organism from infection and from foreign antigens by cellular and humoral mechanisms involving lymphocytes, macrophages, and other antigen-presenting cells that regulate each other by means of multiple cell-cell interactions and by elaborating soluble factors, including lymphokines and antibodies, that have autocrine, paracrine, and endocrine effects on immune cells.

Immune disorders can be caused by deficient immune responses (for instance, but not limited to, HIV/AIDS) or overactive immune responses (e.g., allergies, autoimmune disorders). Immune disorders can result in the uncontrolled proliferation of immune cells, the uncontrolled response to foreign antigens or organisms leading to allergic or inflammatory diseases, aberrant immune responses directed against host cells leading to autoimmune organ damage and dysfunction, or generalized suppression of the immune response leading to severe and recurrent infections. Immune disorders refer to disorders of the innate immune system (innate immunity) and the adaptive immune system (adaptive immunity). Innate immunity refers to an early system of defense that depends on invariant receptors recognizing common features of pathogens. The innate immune system provides barriers and mechanisms to inhibit foreign substances, particularly through the action of macrophages and neutrophils. The inflammatory response is considered part of innate immunity. The innate immune system is involved in initiating adaptive immune responses and removing pathogens that have been targeted by an adaptive immune response. However, innate immunity can be evaded or overcome by many pathogens, and does not lead to immunological memory. Adaptive immunity refers to the ability to recognize pathogens specifically and to provide enhanced protection against reinfection due to immunological memory based on clonal selection of lymphocytes bearing antigen-specific receptors. A process of random recombination of variable receptor gene segments and the pairing of different variable chains generates a population of lymphocytes, each bearing a distinct receptor, forming a repertoire of receptors that can recognize virtually any antigen. If the receptor on a lymphocyte is specific for a ubiquitous self-antigen, the cell is normally eliminated by encountering the antigen early in its development. Adaptive immunity is normally initiated when an innate immune response fails to eliminate a new infection, and antigen and activated antigen-presenting cells are delivered to draining lymphoid tissues. When a recirculating lymphocyte encounters its specific foreign antigen in peripheral lymphoid tissues, its proliferation is induced and its progeny then differentiate into effector cells that can eliminate the infectious agent. A subset of these proliferating lymphocytes differentiates into memory cells, capable of responding rapidly to the same pathogen if it is encountered again.

The present disclosure also relates to a method of treating a condition by administering a therapeutically effective amount of cells modified to express a PIF peptides to a subject in need thereof, wherein the condition to be treated is an immune disorder.

The immune disorder may be selected from: HIV, AIDS, SCIDS, bacterial peritonitis, chemical peritonitis, allergy, asthma, pollinosis, and atopy.

### Autoimmune Disorders

Autoimmune disorders are considered to be caused, at least in part, by a hypersensitivity reaction similar to allergies, because in both cases the immune system reacts to a substance that it normally would ignore. Autoimmune disorders include, but are not limited to, Hashimoto's thyroiditis, pernicious anemia, autism, Alzheimer's disease, Addison's disease, type I (insulin-dependent) diabetes, arthritis, rheumatoid arthritis, systemic lupus erythematosus, dermatomyositis, Sjogren's syndrome, lupus erythematosus, multiple sclerosis, myasthenia gravis, chronic neurogenerative disease, Reiter's syndrome, Grave's disease, alopecia areata, anklosing spondylitis, antiphospholipid syndrome, autoimmune hemolytic anemia, autoimmune hepatitis, autoimmune inner-ear disease, autoimmune lymphoproliferative syndrome (ALPS), autoimmune thrombocytopenic purpura (ATP), Behcet's disease, bullous pemphigoid, cardiomyopathy, celiac sprue dermatitis, chronic fatigue syndrome immune deficiency syndrome (CFIDS), chronic inflammatory demyelinating polyneuropathy, cicatricial pemphigoid, cold agglutinin disease, CREST syndrome, Crohn's disease, Dego's disease, dermatomyositis, dermatomyositis, discoid lupus, essential mixed cryoglobulinemia, fibromyalgia-fibromyositis, Guillain-Barre syndrome, idiopathic pulmonary fibrosis, idiopathic thrombocytopenia purpura (ITP), IgA nephropathy, juvenile arthritis, Meniere's disease, mixed connective tissue disease, pemphigus vulgaris, neurodegenerative syndromes, polyarteritis nodosa, polychondritis, polyglancular syndromes, polymyalgia rheumatica, polymyositis, primary agammaglobulinemia, primary biliary cirrhosis, psoriasis, Raynaud's phenomenon, rheumatic fever, sarcoidosis, scleroderma, stiff-man syndrome, Takayasu arteritis, temporal arteritis/giant cell arteritis, ulcerative colitis, uveitis, vasculitis, vitiligo, Wegener's granulomatosis, collagen disease, and connective tissue disease.

The present disclosure also relates to a method of treating a condition comprising administering a therapeutically effective amount of cells modified to express a PIF peptide, wherein the condition is an autoimmune disease.

The autoimmune disease may be selected from: Hashimoto's thyroiditis, pernicious anemia, Alzheimer's disease, Addison's disease, type I (insulin-dependent) diabetes, arthritis, rheumatoid arthritis, systemic lupus erythematosus, dermatomyositis, Sjogren's syndrome, lupus erythematosus, multiple sclerosis, myasthenia gravis chronic neurogenerative disease, Reiter's syndrome, Grave's disease, alopecia areata, anklosing spondylitis, antiphospholipid syndrome, autoimmune hemolytic anemia, autoimmune hepatitis, autoimmune inner-ear disease, autoimmune lymphoproliferative syndrome (ALPS), autoimmune thrombocytopenic purpura (ATP), Behcet's disease, bullous pemphigoid, cardiomyopathy, celiac sprue dermatitis, chronic fatigue syndrome immune deficiency syndrome (CFIDS), chronic inflammatory demyelinating polyneuropathy, cicatricial pemphigoid, cold agglutinin disease, CREST syndrome, Crohn's disease, Dego's disease, dermatomyositis, discoid lupus, essential mixed cryoglobulinemia, fibromyalgia-fibromyositis, Guillain-Barre syndrome, idiopathic pulmonary fibrosis, idiopathic thrombocytopenia purpura (ITP), IgA nephropathy, juvenile arthritis, Meniere's disease, mixed connective tissue disease, neurodegenerative syndromes, pemphigus vulgaris, polyarteritis nodosa, polychondritis, polyglandular syndromes, polymyalgia rheumatica, polymyositis, primary agammaglobulinemia, primary biliary cirrhosis, psoriasis, Raynaud's phenomenon, rheumatic fever, sarcoidosis, scleroderma, stiff-man syndrome, Takayasu arteritis, temporal arteritis/giant cell arteritis, ulcerative colitis, uveitis, vasculitis, vitiligo, Wegener's granulomatosis, collagen disease, and connective tissue disease.

### Inflammatory Disorders

Inflammatory responses can be triggered by injury, for example to the skin, muscle, tendons, brain or nerves. Inflammatory responses can also be triggered as part of an immune response. Inflammatory responses can also be triggered by infection, where pathogen recognition and tissue damage can initiate an inflammatory response at the site of infection. Generally, infectious agents induce inflammatory responses by activating innate immunity. Inflammation combats infection by delivering additional effector molecules and cells to augment the killing of invading microorganisms by the front-line macrophages, by providing a physical barrier preventing the spread of infection, and by promoting repair of injured tissue. "Inflammatory disorder" is sometimes used to refer to chronic inflammation, regardless of its cause.

Diseases characterized by inflammation of the skin, often characterized by skin rashes, include but are not limited to dermatitis, atopic dermatitis (eczema, atopy), contact dermatitis, dermatitis herpetiformis, generalized exfoliative dermatitis, seborrheic dermatitis, drug rashes, erythema multiforme, erythema nodosum, granuloma annulare, poison ivy, poison oak, toxic epidermal necrolysis and rosacea.

Inflammation triggered by various kinds of injuries to muscles, tendons or nerves caused by repetitive movement of a part of the body is generally referred to as repetitive strain injury (RSI). Diseases characterized by inflammation triggered by RSI include, but are not limited to, bursitis, carpal tunnel syndrome, Dupuytren's contracture, epicondylitis (e.g. "tennis elbow"), "ganglion" (inflammation in a cyst that has formed in a tendon sheath, usually occurring on the wrist) rotator cuff syndrome, tendinitis (e.g., inflammation of the Achilles tendon), tenosynovitis, and "trigger finger" (inflammation of the tendon sheaths of fingers or thumb accompanied by tendon swelling).

Inflammation is an aspect of many diseases and disorders, including but not limited to diseases related to immune disorders, viral infections, arthritis, autoimmune diseases, collagen diseases, allergies, asthma, pollinosis, and atopy. Inflammation is characterized by rubor (redness), dolor (pain), calor (heat) and tumor (swelling), reflecting changes in local blood vessels leading to increased local blood flow which causes heat and redness, migration of leukocytes into surrounding tissues (extravasation), and the exit of fluid and proteins from the blood and their local accumulation in the inflamed tissue, which results in swelling and pain, as well as the accumulation of plasma proteins that aid in host defense. These changes are initiated by cytokines produced by activated macrophages. Inflammation is often accompanied by loss of function due to the replacement of parenchymal tissue with damaged tissue (e.g., in damaged myocardium), reflexive disuse due to pain, and mechanical constraints on function, e.g., when a joint swells during acute inflammation, or when scar tissue bridging an inflamed joint contracts as it matures into a chronic inflammatory lesion.

It is understood that the terms "immune disorder" and "inflammatory response" are not exclusive. It is understood that many immune disorders include acute (short-term) or chronic (long-term) inflammation, as well as delayed inflammation onset. It is also understood that inflammation can have immune aspects and non-immune manifestations. The role(s) of immune and non-immune cells in a particular inflammatory response may vary with the type of inflammatory response, and may vary during the course of an inflammatory response. Immune aspects of inflammation and diseases related to inflammation can involve both innate and adaptive immunity. Certain diseases related to inflammation represent an interplay of immune and non-immune cell interactions, for example intestinal inflammation, pneumonia (lung inflammation), or glomerulonephritis.

It is further understood that many diseases are characterized by both an immune disorder and an inflammatory response, such that the use of discrete terms "immune disorder" or "inflammatory response" is not intended to limit the scope of use or activity of the compounds of the present disclosure with respect to treating a particular disease. For example, arthritis is considered an immune disorder characterized by inflammation of joints, but arthritis is likewise considered an inflammatory disorder characterized by immune attack on joint tissues. Thus, the observation that a compound of the invention reduces the inflammation seen in an animal model of arthritis, does not limit the observed activity of the compound to anti-inflammatory activity. In a disease having both immune and inflammatory aspects, merely measuring the effects of a compound of the present invention on inflammation does not exclude the possibility that the compound may also have immune-modulating activity in the same disease. Likewise, in a disease having both immune and inflammatory aspects, merely measuring the effects of a compound of the present invention on immune responses does not exclude the possibility that the compound may also have anti-inflammatory activity in the same disease.

to the present disclosure provides a method of treating a condition comprising administering a therapeutically effective amount of cells modified to express a PIF peptide to a subject in need thereof, wherein the condition is an inflammatory disorder.

The inflammatory disorder may be selected from: breast disease, skin disorders, dermatitis, atopic dermatitis (eczema, atopy), contact dermatitis, dermatitis herpetiformis, generalized exfoliative dermatitis, seborrheic dermatitis, drug rashes, erythema multiforme, connective tissue disorders, erythema nodosum, granuloma annulare, poison ivy, poison oak, toxic epidermal necrolysis, rosacea, bursitis, carpal tunnel syndrome, Dupuytren's contracture, epicondylitis, ganglion, rotator cuff syndrome, tendinitis, tenosynovitis, and trigger finger.

### Neuroinflammation

Neuroinflammatory disorders represent a large spectrum of chronic diseases ranging from multiple sclerosis to brain disorders, Alzheimer's disease and/or autism. Activated myelin-specific T-cells that migrate into the nervous system, leading to progressive inflammation, are viewed as an important etiological factor throughout the spectrum of neuroinflammatory disorders.

It is known that autoimmune disorder symptoms may improve during pregnancy. However, this improvement is short-lived, since the disorder frequently flares post-partum. Following delivery, fetal cells can persist for several years, creating microchimerism and possibly promoting autoimmunity. Consequently, pregnancy-related compounds such as PIF peptides are possible therapies for autoimmune disorders, and particularly for neuroinflammatory disorders. PIF peptides have been shown to reverse severe paralysis while promoting neurogenesis.

Beyond genetics, it is not fully elucidated whether neurodevelopmental disorders are initiated at the prenatal/neonatal period, the post-natal period, or whether they are initiated on a continuum. Identifying embryo-protective mechanisms that persist post-delivery is critical. PIF peptides exert autotrophic protective effects by regulating oxidative stress, protein misfolding, and macrophages. In non-pregnant models, through similar mechanisms, PIF peptides reversed severe neuroinflammation (reversed severe paralysis) up to total resolution. The human implantation model (HESC stromal cells) PIF peptides promote receptivity and neuroprotective proteins. PIF peptide-induced protective maternal pathways can prevent damage to the notochord, which is the embryo's earliest structure.

In HESC, PIF peptides affected angiotensin, epithelial-mesenchymal transition and cytoskeletal signaling pathways. Improved nerve impulse and guidance were coupled with ATBF1, NK3 PlexinA4, HIPK2, SYNNJ2BP and FUS 1 genes expression. PIF peptides enriched genes affecting the central nervous system and neonates while reducing autism and other developmental disorders. In FITC, PIF peptides affected angiotensin, xenobiotic and hormone biosynthesis pathways. Improved axon guidance and transmission were coupled with PEA3, STAT3, c-Rel, CDK1, UGT1A6/A1 and beta-fodrin. PIF peptides enriched genes affecting brain infarctions, neoplasms, and developmental disorders, reducing connective disorders. By regulating uterine gene pathways, PIF peptides may decrease the embryo's vulnerability to neurological diseases. Such protection may represent a long-term preventive strategy against the development of post-natal pathologies.

A neurodevelopmental disorder, or disorder of neural development, may be any impairment of the growth and development of the brain and/or central nervous system. Such disorders may affect emotion, communication skills, speech, language skills, learning ability and memory and may increasingly present as the individual ages, Neurodevelopmental disorders may be associated with widely varying degrees of mental, emotional, physical and economic burdens to individuals, families and society in general. There are many causes of neurodevelopmental disorders, ranging from deprivation to genetic and metabolic diseases, immune disorders, infectious diseases, nutritional factors, physical trauma, toxic and environmental factors or a combination of these factors.

The present disclosure also relates to a method of treating a condition comprising administering a therapeutically effective amount of cells modified to express a PIF peptide, wherein the condition is a caused by neuroinflammation.

The condition may be selected from the following: autism, Alzheimer's disease, neurodevelopmental disorders, brain disorders, child developmental disorders, mood disorders, stress, mental disorders, mental disorders diagnosed at childhood, craniomandibular disorders, temporomandibular disorders, mandibular disorders, schizophrenia, sleep initiation disorders, sleep maintenance disorders, epilepsy, Angelman Syndrome, dystonia, dystonic disorders, and anxiety disorders.

### Diabetes

Insulin-dependent type 1 (juvenile) diabetes (TIDM) leads to the destruction of insulin-producing pancreatic islet beta cells causing a life-long dependence on insulin administration. The etiology of TIDM is complex and involves a combination of genetic, environmental, and immunological influences. TIDM is characterized by a progressive, asymptomatic decline in beta cell function until hyperglycemia develops. The disease process begins with infiltration of mononuclear cells around the islets, and proceeds with destruction of the insulin-producing beta cells. Islet destruction is mediated by CD4⁺ and CD8⁺ T-cells, activated by specific pancreatic antigens. In fulminant TIDM, macrophage infiltration dominates, coupled with increased TOLL receptor expression. The important role of protein misfolding and oxidative stress leading to islet cell apoptosis in TIDM has also been documented. The immune modulatory properties of PIF peptides are useful in the treatment of TIDM. PIF peptides prevent diabetes while maintaining pancreatic function.

The present disclosure also relates to a method of treating a condition comprising administering a therapeutically effective amount of cells modified to express a PIF peptide, wherein the condition is diabetes.

### Radiation-Induced Disease

Presently, no definite therapies are in use for patients suffering from radiation sickness or exposure to radiation, either from a nuclear attack or accident, or from cancer treatment. The current treatment options are epidemiologically based and not based on individual diagnosis/treatment. Ideal treatment would be individualized, rapid, nontoxic and easy to administer. It is well accepted that radiation injury combined with trauma, burn and sepsis would be the most likely scenario after a nuclear attack. In such cases, the most suitable view of Acute Radiation Sickness (ARS) is multi-organ dysfunction due to exacerbated immune response.

PIF presents broad immunomodulatory features and control of inflammation and engraftment properties; thus, PIF can be used for ARS management. PIF can be used as a protector to counteract radiation injury if given before exposure, as well as a safe self-administration countermeasure for military personnel, in the few minutes post-detonation but before exposure to fallout.

Radiation-induced liver damage (RILD) is a major complication of liver irradiation characterized by the development of parenchyma liver cell damage, venous occlusive disease, fibrosis formation and high mortality. RILD may manifest 4 to 8 weeks after radiation exposure. Significant RILD may develop in patients exposed to an excess of 30 Gy of radiation, depending on irradiated liver volume and hepatic functional reserve. Patients may present with a triad of ascites, hepatomegaly, and elevated liver enzymes. Radiation-induced endothelial damage exposes the subendothelial basement membrane, leading to platelet activation and aggregation, and stimulation of dormant hepatic stellate cells. Fibrin thrombus causes venous occlusion, panlobular congestion, diffuse hemorrhagic and necrotic foci, and distention of hepatic sinusoids. Prolonged obstruction and activation of hepatic stellate cells may result in hepatocyte loss and fibrosis mediated by transforming growth factor-pi release. Radiologically, RILD presents with a "straight-border" sign, which is defined as any hepatic attenuation difference bordered by straight lines. Fatty infiltration, fibrosis, and vascular abnormalities can all present similarly. RILD presents as demarcated areas of hypo- or hyperattenuation in a nonanatomic distribution, contrasting with vascular lesions.

Acute radiation syndrome (ARS), also known as radiation sickness, develops after whole-body or partial-body high-dose irradiation. ARS injuries may result in damage to hematopoiesis, leading to immune suppression. In addition, acute toxicity to the skin, gut, and central nervous system may also be prevalent. Although individual organ damage can be monitored, a more suitable view for ARS is the concept of multiple organ dysfunction syndrome caused by a systemic inflammatory response. A number of reports show that radiation-induced production of pro-inflammatory cytokines contributes to radiotherapy-associated disorders in the blood and peripheral lymphoid tissues.

Despite advances in understanding of the pathophysiology of radiation injury, very little information is available on possible therapeutic approaches to radiation sickness. The current clinical treatment approach is to inhibit the production of inflammatory mediators and suppress the initiation of the inflammatory response. These treatments cause generalized immunosuppression and place patients in danger of opportunistic infections.

The present disclosure also relates to a method of treating a condition comprising administering a therapeutically effective amount of cells modified to express a PIF peptide, wherein the condition is exposure to radiation.

The exposure to radiation may cause ARS, radiation poisoning, radiation-induced organ damage, or intracellular damage.

The present disclosure also relates to a method of preventing the development of ARS by administering a therapeutically effective amount of cells modified to express a PIF peptide to a subject.

The present disclosure also relates to a method of preventing or reversing changes in chemokines, cytokines, and other inflammatory agents after exposure to radiation by administering a therapeutically effective amount of cells modified to express a PIF peptide to a subject.

The present disclosure also relates to a method of improving immune cell function and recovery following exposure to radiation by administering a therapeutically effective amount of cells modified to express a PIF peptide to a subject.

The present disclosure also relates to a method of normalizing hepatic, colon, and other organ function following exposure to radiation by administering a therapeutically effective amount of cells modified to express a PIF peptide to a subject.

The present disclosure also relates to a method of preventing a stochastic effect of radiation by administering a therapeutically effective amount of cells modified to express a PIF peptide to a subject.

### Brain Inflammation

PIF peptides have been shown to reverse brain inflammation while restoring brain/nerve function.

The present disclosure also relates to a method of treating brain inflammation by administering a therapeutically effective amount of cells modified to express a PIF peptide to a subject.

The present disclosure also relates to a method of reversing advanced brain ischemia and inflammation following a stroke by targeting brain microglia cells comprising administering a therapeutically effective amount of cells modified to express a PIF peptide to a subject.

### Chronic Tuberculosis Infection

Tuberculosis (TB), caused by bacilli *mycobacterium tuberculosis* (Mtb), is a serious and life-threatening disease which infects about 8 million and kills about 3 million people annually worldwide. Mtb invades and replicates within the endosomes of alveolar macrophages, altering their activity. Localized immune suppression within mononuclear cell granulomas develops following Mtb infection. Macrophages, T lymphocytes, B lymphocytes and fibroblasts are among the cells that aggregate to form a granuloma, with lymphocytes surrounding the infected macrophages. The granuloma not only prevents dissemination of the mycobacteria, but also provides a local environment for the communication of immune cells. Within the granuloma, T lymphocytes secrete cytokines such as interferon (IFN)-gamma, which activates macrophages to destroy the bacteria with which they are infected. Cytotoxic T cells can also directly kill infected cells by secreting perforin and granulysin. Importantly, Mtb may survive in granulomas by going into a latent state.

During pregnancy, the vertical transmission of TB infection from the mother/host to the newborn/allograft is low, analogous to the pregnancy-related protection observed against HIV and various immune disorders such as multiple sclerosis. Without wishing to be bound by theory, it is believed that activation of the host's immune system could be instrumental in the treatment of TB, and embryo-specific protective compounds could have such a pivotal protective role against TB. Successfully transposing pregnancy's immune-modulatory effects to the non-pregnant immune state may result in a powerful, effective, nontoxic tool to control TB. Thus, having a potent immune-modulatory agent that may act in synergy with anti-TB agents may represent a critical supportive element in the protection against this serious disease.

PIF peptides create immune tolerance and acceptance while playing an essential role in responding as needed to pathogen challenges. Localized immune suppression within mononuclear cell granulomas develops following Mtb infection. Accordingly, PIF peptides may prove to be a useful immunomodulator of TB. Without wishing to be bound by theory, PIF peptides revert tuberculin-induced disease, resorting to local (intracellular) as well as to global immune modulation without suppression. Therefore, it is believed that PIF peptides may target the Kvl.3 potassium channel, an anti-TB target.

The present disclosure also relates to a method of reversing chronic intracellular infection by tuberculosis and other intracellular pathogens by administering a therapeutically effective amount of cells modified to express a PIF peptide to a subject.

### Intracellular Damage

Embodiments described herein are directed to the use of PIF as a useful immune-modulator for the treatment of intracellular damage.

The present disclosure also relates to a method of treating intracellular damage by administering a therapeutically effective amount of cells modified to express a PIF peptide to a subject.

The present disclosure also relates to a method of regulating cytokine, chemokine, and inflammatory mediator secretion in response to intracellular damage comprising administering a therapeutically effective amount of cells modified to express a PIF peptide to a subject.

### Transplant-Related Methods of Treatment - Non-Pregnant Subjects Bone Marrow Transplantation (BMT)

Bone Marrow Transplantation (BMT) may be used as a treatment for hematological malignancies and inherited blood cell disorders, such as, but not limited to, lymphomas, lymphocytic leukemias, myeloma, leukemia, anemia, hemophilia, thalassemias, sickle cell disease, multiple sclerosis, scleroderma, myelodysplasia syndromes and myeloproliferative diseases. There are three types of bone marrow transplantation: autologous/syngeneic (self), allogeneic (donor), and semi-allogeneic.

An autologous transplant may be possible if the disease afflicting the bone marrow is in remission or if the condition being treated does not involve the bone marrow. In autologous bone marrow transplantation, the bone marrow is extracted from the patient prior to transplant and may be "purged" to remove lingering malignant cells (if the disease has afflicted the bone marrow). After the patient undergoes chemotherapy or radiation, the stem cells are transplanted back into the patient. Autologous bone marrow transplant allows the patient to receive high doses of chemotherapy **and** radiation. High doses of chemotherapy or radiation therapy with bone marrow or peripheral blood transplants have improved cure rates for leukemia and lymphoma. Once the patient has gone through chemotherapy or radiation, the patient may have a limited ability to produce blood cells. An autologous transplant would allow the patient to "jump start" the production of blood cells and platelets.

The present disclosure also relates to a method of decreasing the immune response in a subject receiving a transplantation by administering a therapeutically effective amount of cells modified to express a PIF to a subject.

The present disclosure also relates to a method of increasing engraftment of solid organs, such as the heart, liver, bowel, lung, and kidney, comprising administering a therapeutically effective amount of cells modified to express a PIF peptide to a subject.

The present disclosure also relates to a method of increasing engraftment of transplanted bone marrow by administering a therapeutically effective amount of cells modified to express a PIF peptide to a subject.

The present disclosure also relates to a method of improving acceptance of transplanted bone marrow without deleterious graft-versus-host disease (GVHD) while maintaining the beneficial graft-versus-leukemia (GVL) effect by administering a therapeutically effective amount of cells modified to express a PIF to a subject.

The present disclosure also relates to a method of improving acceptance and function of transplanted stem cells by administering a therapeutically effective amount of cells modified to express a PIF peptide to a subject.

### Graft-versus-Host Disease (GVHD)

Mature donor T cells are the main mediators of the beneficial graft-versus-leukemia effect that prevents relapse of malignancy. However, these same cells also induce GVHD, which is a major cause of morbidity and mortality in BMT recipients.

Acute GVHD is characterized by widespread damage, acutely affecting mainly target organs such as the skin, liver, and gastrointestinal tract and chronically producing multi-organ damage and failure. Protecting these vital organs while preventing the return of malignancy after immunosuppressive drug administration remains a difficult clinical challenge. Treating GVHD poses serious challenges. Standard GVHD prophylaxis and therapy involve the use of drugs that cause generalized immune suppression, placing patients in danger of opportunistic infections and tumor relapse. PIF peptides may be used to prevent alloactivation and acute GVHD while maintaining the GVL effect.

The present disclosure also relates to a method of preserving the GVL effect of transplanted bone marrow while reducing GVHD by administering a therapeutically effective amount of cells modified to express a PIF peptide to a subject.

### Atherosclerosis and Circulatory System Diseases

Atherosclerosis is a chronic disease that may remain asymptomatic for decades. Atherosclerotic lesions are separated into two broad categories: stable and unstable (also called vulnerable). The pathobiology of atherosclerotic lesions is complicated, but generally, stable atherosclerotic plaques, which tend to be asymptomatic, may be rich in smooth muscle cells, while unstable plaques may be rich in macrophages and foam cells. The extracellular matrix separating the lesion from the arterial lumen (also known as the fibrous cap) is usually weak and prone to rupture. Ruptures of the fibrous cap expose thrombogenic material, such as collagen, to the circulation and eventually induce thrombus formation in the lumen. Upon formation, intraluminal thrombi can occlude arteries outright (i.e. coronary occlusion), but more often they detach, move into the circulation and eventually occlude smaller downstream branches, causing thromboembolism. A stroke is often caused by thrombus formation in the carotid arteries. Apart from thromboembolism, chronically expanding atherosclerotic lesions may cause complete closure of the lumen. Interestingly, chronically expanding lesions are often asymptomatic until lumen stenosis is so severe that blood supply to downstream tissue(s) is insufficient, resulting in ischemia.

These complications of advanced atherosclerosis are chronic, slowly progressive and cumulative. Unstable plaque may suddenly rupture, causing the formation of a thrombus that will rapidly slow or stop blood flow, leading to death of the tissues fed by the artery in approximately 5 minutes. This catastrophic event is called an infarction. One of the most common recognized examples is called coronary thrombosis, causing myocardial infarction (a heart attack). The same process in an artery to the brain is commonly called stroke. Another common scenario in very advanced disease is claudication from insufficient blood supply to the legs, typically due to a combination of both stenosis and aneurysmal segments. Since atherosclerosis occurs throughout the body, similar events may also occur in the arteries to the brain, intestines, kidneys, legs, etc. Other circulatory system diseases may also be treated by administering cells modified to express a PIF peptide.

The present disclosure also relates to a method of treating atherosclerosis by administering a therapeutically effective amount of cells modified to express a PIF peptide to a subject.

The present disclosure also relates to a method of inhibiting or preventing complications of atherosclerosis and plaque formation by administering a therapeutically effective amount of cells modified to express a PIF peptide to a subject.

The present disclosure also relates to a method of preventing or reducing atheromatous plaque hemorrhage or rupture by administering a therapeutically effective amount of cells modified to express a PIF peptide to a subject.

The present disclosure also relates to a method of inhibiting or preventing complications of aortic diseases or vascular aneurysms by administering a therapeutically effective amount of cells modified to express a PIF to a subject.

The present disclosure also relates to a method of inhibiting or preventing vascular stenosis or occlusion by administering a therapeutically effective amount of cells modified to express a PIF peptide to a subject. Cancer

The present disclosure also relates to a method of treating cancer by administering a therapeutically effective amount of cells modified to express a PIF peptide to a subject, wherein the PIF peptide enhances the anti-cancer activity of pharmaceutical agents.

### R-I-K-P Polypeptide

Another embodiment of this invention is directed to a synthetic polypeptide having an amino acid sequence of R-I-K-P (SEQ ID NO: 11).

Another embodiment of this invention is directed to a synthetic polypeptide having a nucleotide sequence of SEQ ID NO: 17.

Another embodiment of this invention is directed to a composition of a synthetic polypeptide having an amino acid sequence of R-I-K-P (SEQ ID NO: 11) and an excipient.

Another embodiment of this invention is directed to a composition of a synthetic polypeptide having a nucleotide sequence of SEQ ID NO: 17 and an excipient.

In another embodiment, a composition is made of a polypeptide having an amino acid sequence of SEQ ID NO: 11 bonded to a label. In some embodiments, the label is selected from the following: FITC, biotin, rhodamine, radioactive isotopes, and fluorescent nanocrystals.

In another embodiment, a composition is made of a polypeptide having a nucleotide sequence of SEQ ID NO: 17 bonded to a label. In some embodiments, the label is selected from the following: FITC, biotin, rhodamine, radioactive isotopes, and fluorescent nanocrystals.

In another embodiment, a pharmaceutical composition is made of a polypeptide having an amino acid sequence of R-I-K-P of SEQ ID NO: 11 and a pharmaceutically acceptable excipient.

In another embodiment, a pharmaceutical composition is made of a polypeptide having a nucleotide sequence of SEQ ID NO: 17 and a pharmaceutically acceptable excipient.

### Methods of Identifying Compounds that Bind to the WX₁WX₂X₃X₄REWFX₅X₆X₇W (Trp-X-Trp-X-X-X-Arg-Glu-Trp-Phe-X-X-X-Trp) consensus target interacting site (for reference only).

The present disclosure also relates to a method of identifying a compound that binds to WX₁WX₂X₃X₄REWFX₅X₆X₇W (Trp-X-Trp-X-X-X-Arg-Glu-Trp-Phe-X-X-X-Trp; SEQ ID NO:28) active site of a receptor, wherein each X may independently be any amino acid, comprising the steps of providing the WX₁WX₂X₃X₄REWFX₅X₆X₇W receptor and a cognate ligand of the WX₁WX₂X₃X₄REWFX₅X₆X₇W receptor, combining the cognate ligand and the WX₁WX₂X₃X₄REWFX₅X₆X₇W receptor in the presence of a test compound under conditions wherein, in the absence of the test compound, a pre-determined quantity of a peptide would bind the WX, WX₁WX₂X₃X₄REWFX₅X₆X₇W receptor, and determining if the quantity of the peptide bound to the WX₁WX₂X₃X₄REWFX₅X₆X₇W receptor is decreased in the presence of the test compound, the decrease indicating that the test compound binds to the WX₁WX₂X₃X₄REWFX₅X₆X₇W receptor. In some embodiments, the test compound is a small organic molecule, a peptide, or a peptidomimetic.

The present disclosure also relates to a method of identifying a compound that is an agonist of PIF expression comprising the steps of contacting a WX₁WX₂X₃X₄REWFX₅X₆X₇W receptor with a test compound and determining if the test compound increases WX₁WX₂X₃X₄REWFX₅X₆X₇W receptor activity, wherein an increase in WX₁WX₂X₃X₄REWFX₅X₆X₇W receptor activity indicates that the test compound is an agonist of PIF expression. In some embodiments, the test compound is a small organic molecule, a peptide, or a peptidomimetic.

This invention and embodiments illustrating the method and materials used may be further understood by reference to the following non-limiting examples.

### EXAMPLE 1

### Determination of PIF peptide cDNA sequence

A BLASTP search for the PIF peptide amino acid sequence (SEQ ID NO:3) revealed that PIF peptide (SEQ ID NO:3) has relatively close similarity to the TSP-1 domain (Uniprot: P02893), part of *P. falciparum* protein (residue range: 344 to 389), and to lesser extent to a number of human proteins like neurexins, cyclophilin E, zinc finger domain-containing proteins and acyl-CoA synthetase medium-chain family member 3. Since *blastp* (NCBI) and *tblastn* (NCBI) queries couldn't find an exact match neither in protein nor in nucleotide sequence databases and therefore, no cDNA could be isolated, a back-translation from amino acid to nucleotide sequence for eukaryote exogenous expression was undertaken.

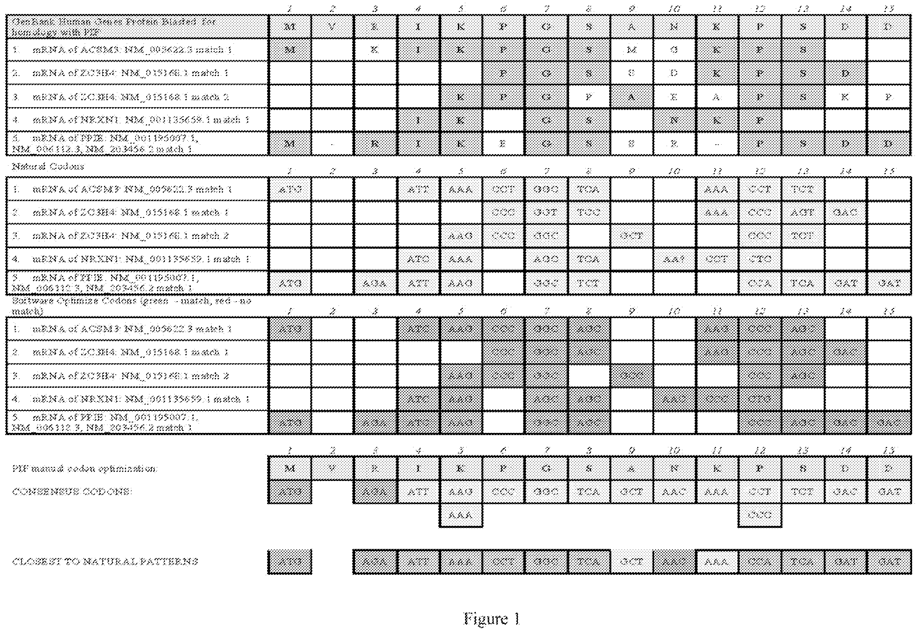

DNA 2.0 GeneDesigner back-translated PIF peptide sequence (SEQ ID NO:3) using Human codon optimized probability table for optimal host expression. As an alternative optimization approach, sequence alignment mapping of a native PIF peptide (SEQ ID NO:3) and BLASTP selected sequence-similar proteins was created. **Figure 1** shows PIF peptide sequence (SEQ ID NO:3) codon mapping over H. sapiens BLASTP queried similar proteins sequence alignment - the corresponding codons of similar AA sequences were used for PIF peptide (SEQ ID NO:3) codon optimization. Retrieving the actual nucleotide sequences of these proteins and mapping them to GeneDesigner-solely optimized codon sequence, produced color-coded labels of matches and mismatches. Then, using the positional codon consensus the *"close to natural pattern sequence* " was created and further used as an alternative codon encoding of PIF peptide (SEQ ID NO:3) for subsequent peptide expression probability analysis. Thus, the lack of definition of native PIF peptide (SEQ ID NO:3) cDNA was resolved by using known similar protein sequences codon encoding retrieved from the human genome or by software codon optimization.

### Codon Optimization

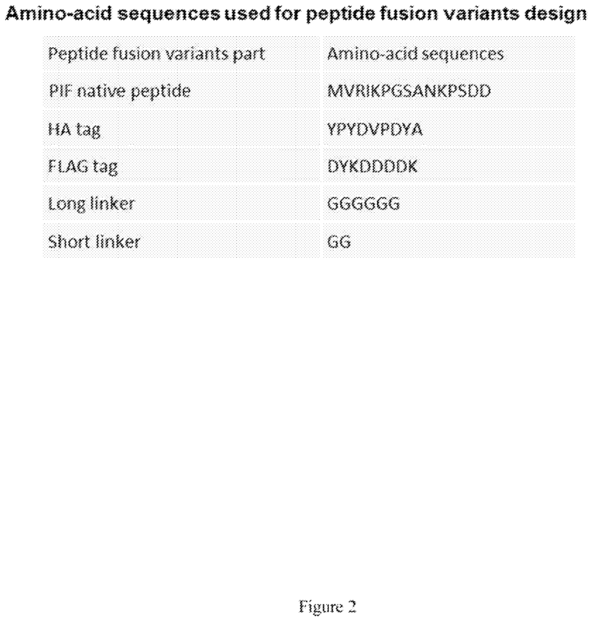

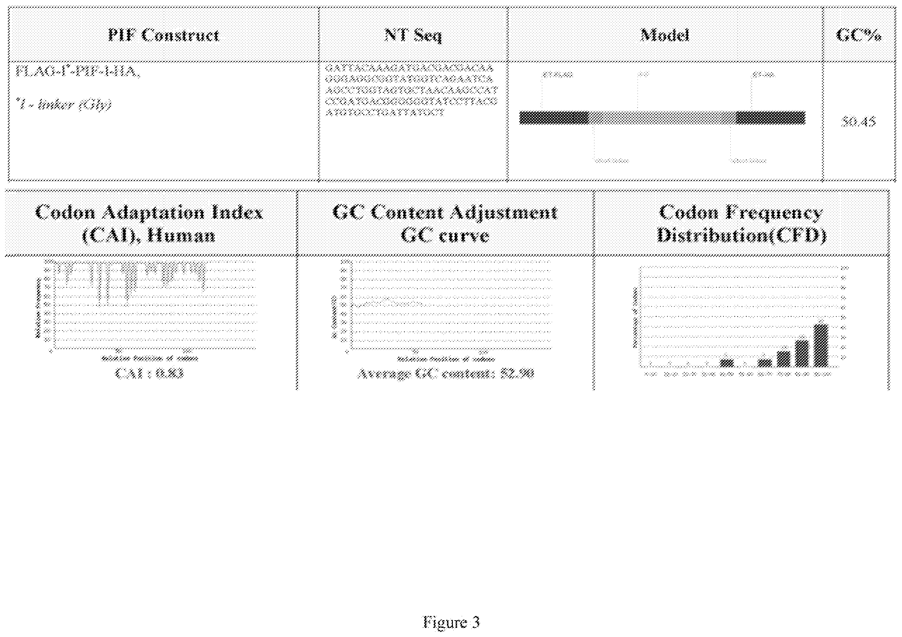

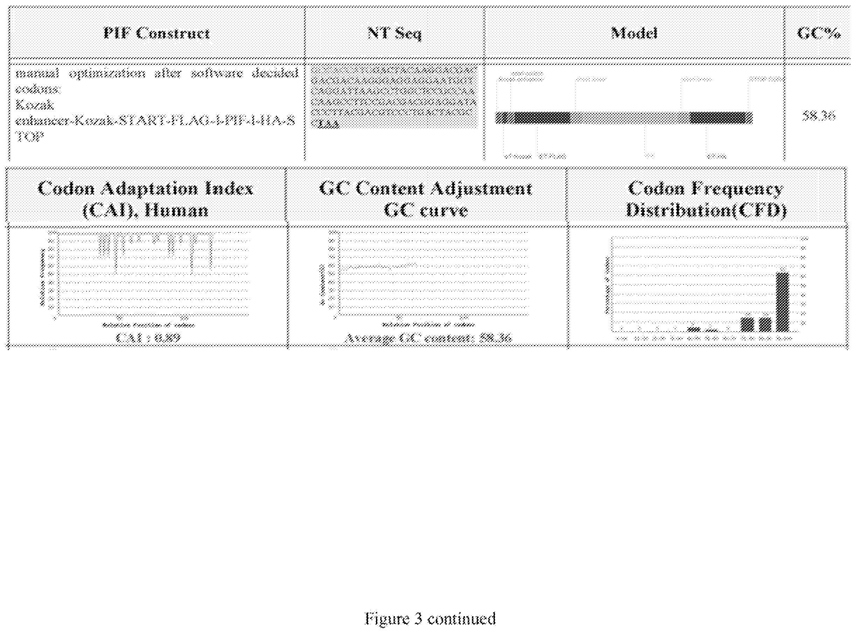

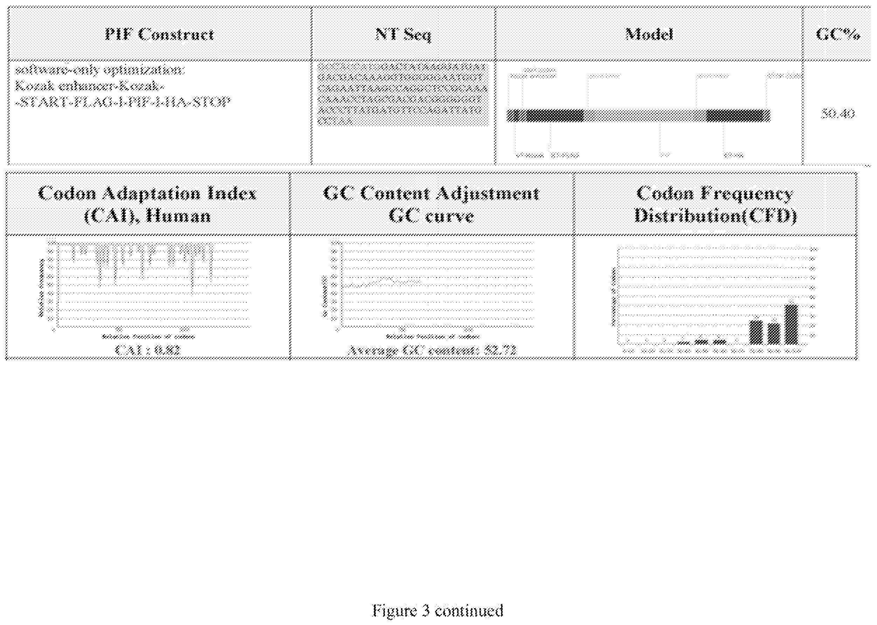

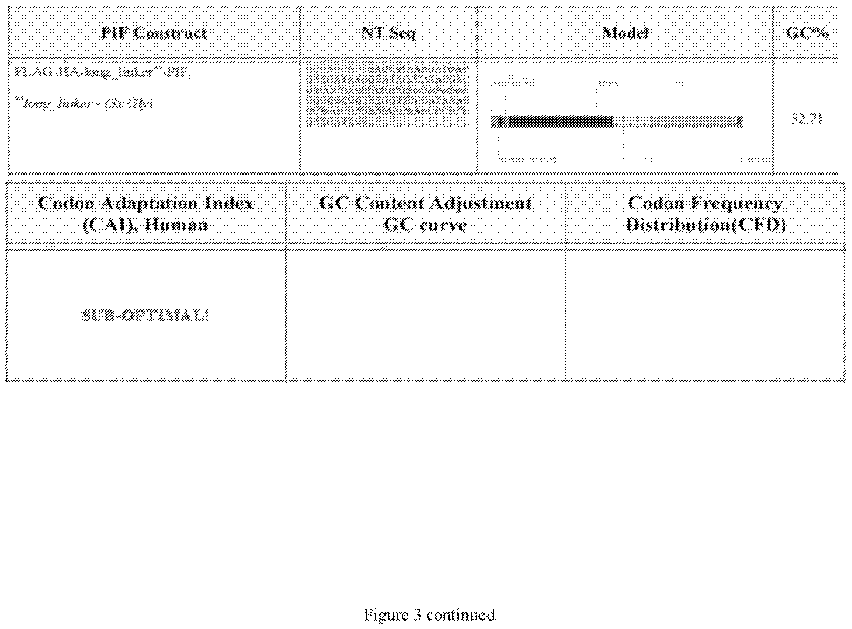

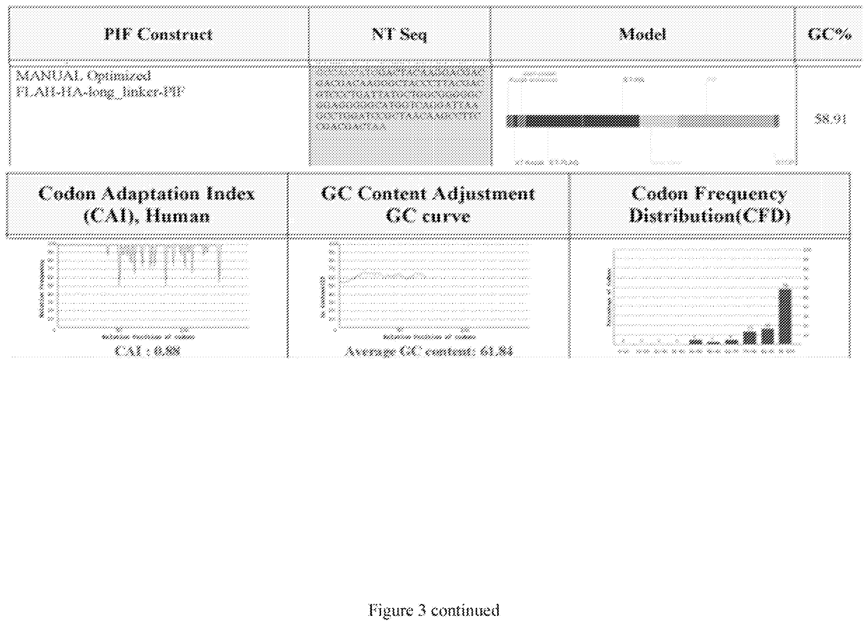

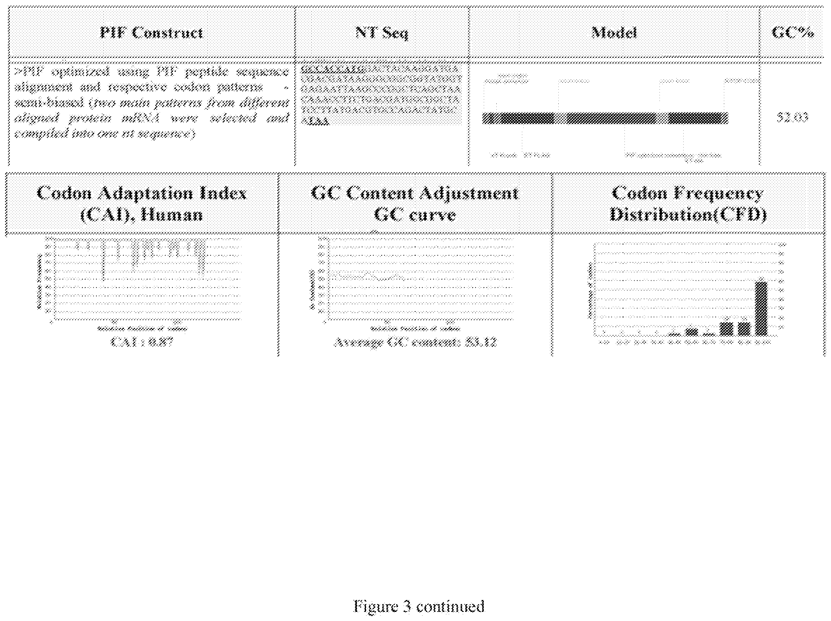

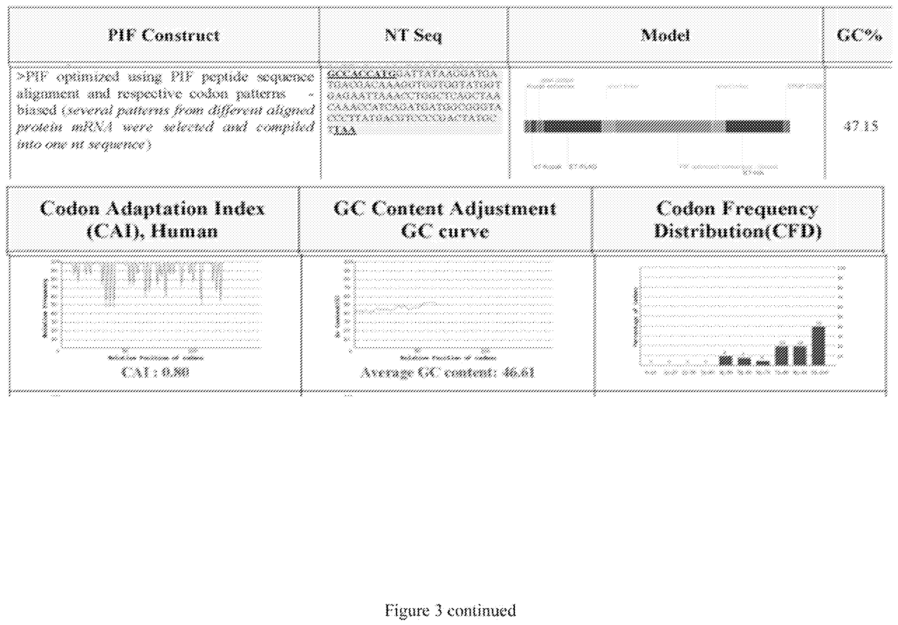

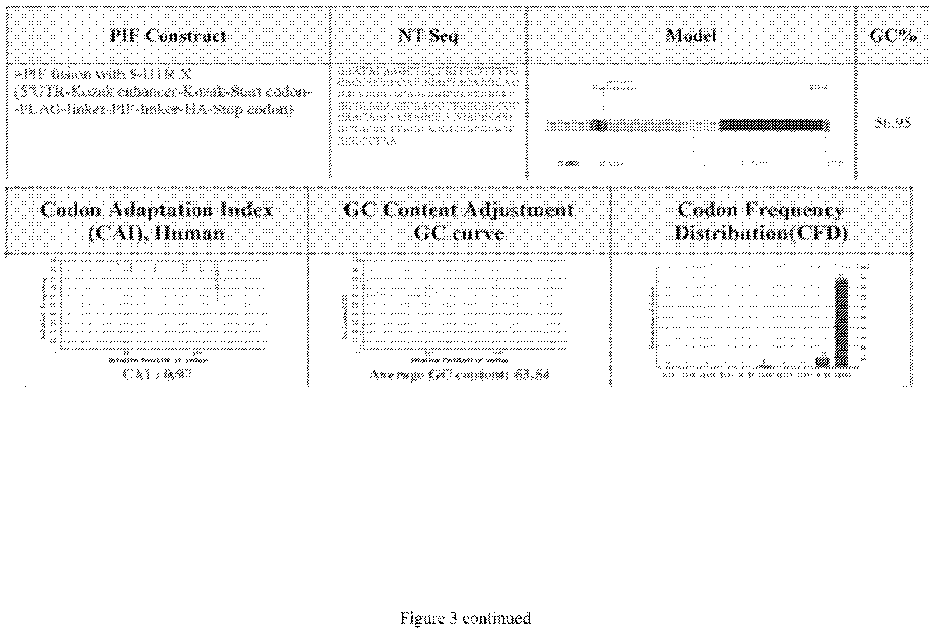

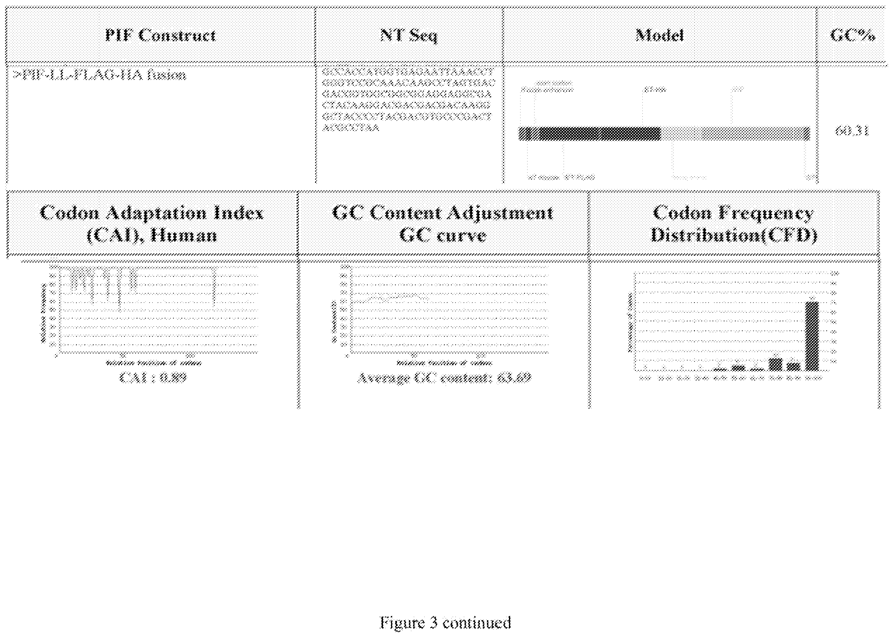

Tagged PIF peptides design scenario was implemented by using two fusion tags, FLAG and HA, following three possible permutations: tags at N-terminal, flanking and tags at C-terminal of PIF peptide, in tag order - FLAG followed by HA. The tags were connected to PIF peptide (SEQ ID NO:3; SEQ ID NO: 14) using either short or long linkers of glycine (Gly). All sequences used as well as the diagram of the peptides are shown in **Figures 2** and **3****.** Using DNA2 GeneDesigner functionality, codon sequences were optimized by eliminating possible 5'-secondary structures preventing ribosomal expression, and Kozak sequence and translation enhancer sequence were added as well. The full insert sequence targeted for seamless cloning was assessed for average and positional GC content and Rare Codon Usage, Codon Adaptation Index (CAI) and Codon Frequency Distribution, using the GenScript Rare Codon Analysis Tool. **Figure 3** shows PIF fusion peptides design scenario, peptide nucleotide sequence, peptide diagram, average GC content (DNA2), and GenScript analysis data for Rare Codon Usage Analysis, including Codon Adaptation Index (CAI), the GC content curve and average GC content, and Codon Frequency Distribution. Rare Codon Usage Analysis evaluates the distribution of codon usage frequency along the length of the coding sequence. A CAI value above 0.8 correlates with the possibility of a high protein expression level in the desired host expression system. Codon Frequency Distribution assesses the percentage distribution of codons across the computed codon quality groups, where the codon with the highest usage frequency for a given amino acid in H. sapiens would have a value of 100. All codons with values lower than 30 are considered likely to hamper the expression efficiency.

In some cases the distribution of codon usage frequency along with the length of the coding sequence was assessed through CAI, which was below 0.8, or the percent distribution of codons across the computed codon quality groups assessed using Codon Frequency had a distribution of <30. In such cases, the probability for hampering or severely reducing the protein expression in the host system was high, therefore an alternative, lower-frequency codons-based nucleotide sequence was devised, while preserving the local GC content below 60%. Human and close to natural pattern optimization in FLAG-PIF-HA peptide enhanced the CAI from 0.82 to 0.87 and even 0.89, without exceeding the GC% above 60%. Similarly, the PIF-FLAG-HA peptide had CAI of suboptimal value suggesting no expression at all, and required human codon optimization that resulted in CAI of 0.88.

Thus, host protein expression was ensured by using software-based GC, CAI, and frequency-optimized codon tables derived from large-scale multispecies expression experimental data. Further, a *close-to-natural pattern sequence-derived codon optimization* (human-manipulated) was applied here. Both contributed to enhanced protein expression in the host due to optimized tRNA pool utilization.

### Conformational Assessment

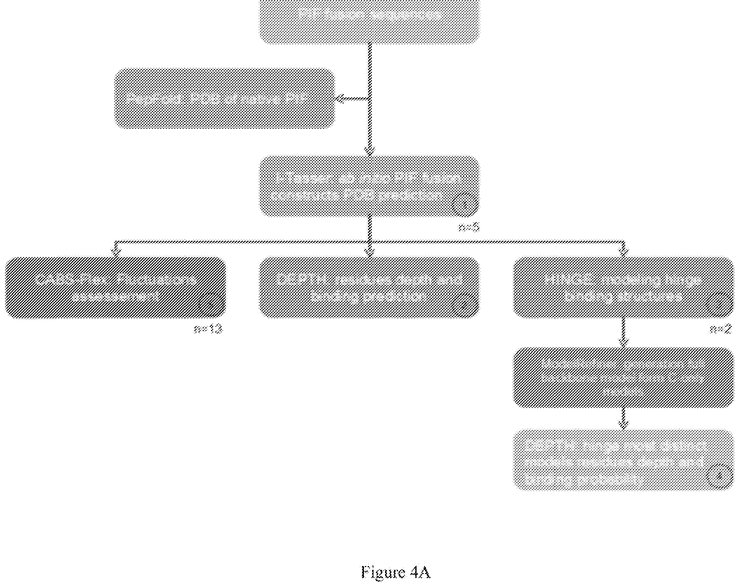

Following codon optimization, to select for the most likely peptide scenario that would yield closer-to-physiological binding and avoid sterical interference, computer-aided conformational assessment was applied. **Figure 4A** shows the modeling strategy scheme: PIF fusion peptide amino acids are modeled using several *de novo* approaches and further submitted to the server for molecular fluctuations assessment, and to the server for detecting deep residues and residues accessibility. Later, a hinge regions prediction server was used to assess potential molecular locations undergoing readjustment that were submitted for depth and accessibility assessment. Sterical interference can develop when fusion tags are added, especially to such a small peptide sequence.

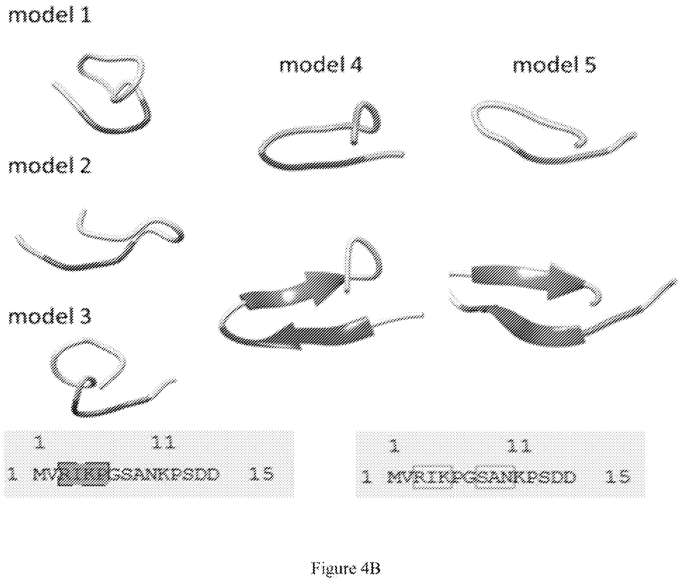

A *de novo* model of the native PIF peptide (SEQ ID NO:3) was first generated by using the PEP FOLD server. The created 5 top probability PDB encoded for tertiary structures, some of which were predicted to eventually form p-sheet or P-bulge-like structures. **Figure 4B** shows the native PIF peptide (SEQ ID NO:3) *ab initio* tertiary structure prediction using PEP FOLD server. The first 5 models have the highest probabilities. In red is the most likely binding consensus sequence of PIF peptides, namely R-I-K-P. The models are situated so the R-I-K-P sequence faces downside, towards the place of possible binding. Models 4 and 5 are predicted to have additional conformations, showing ribbon structure with two beta sheets facing each other.

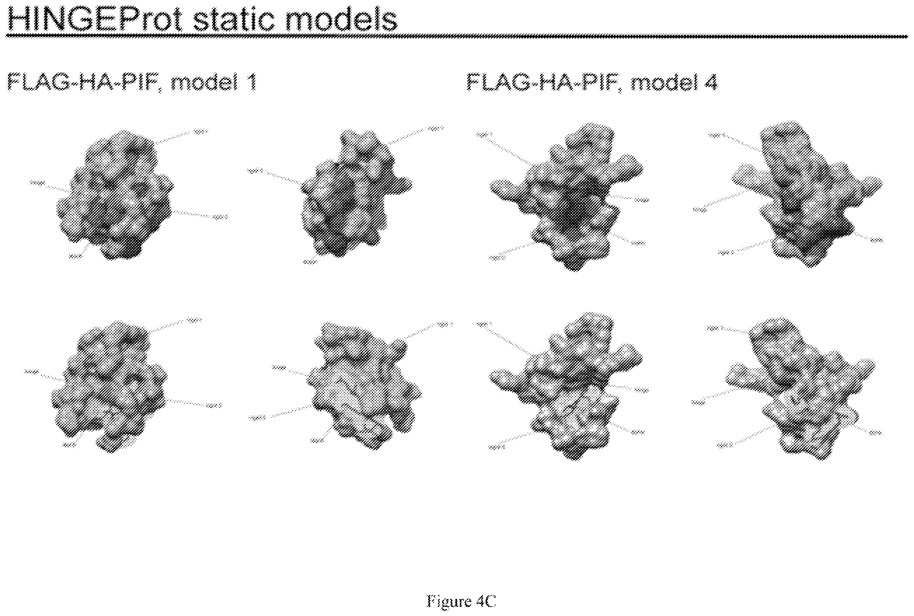

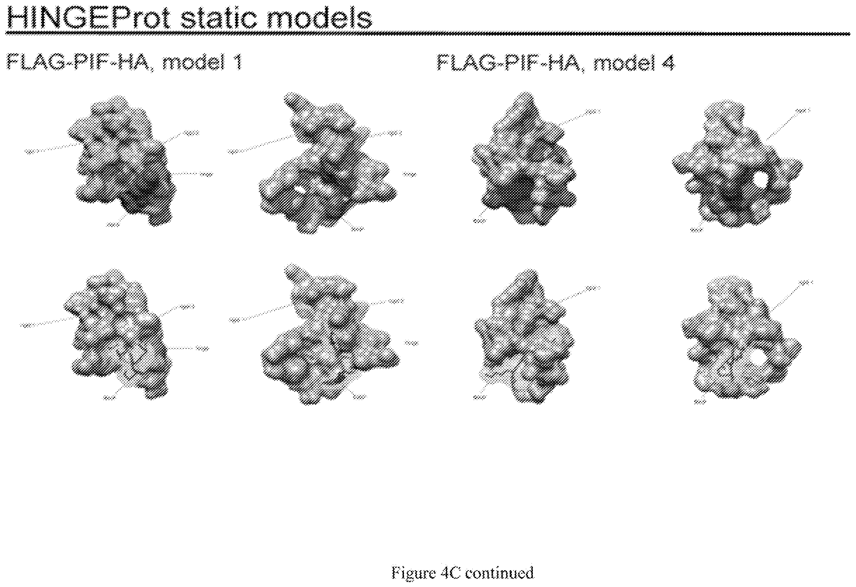

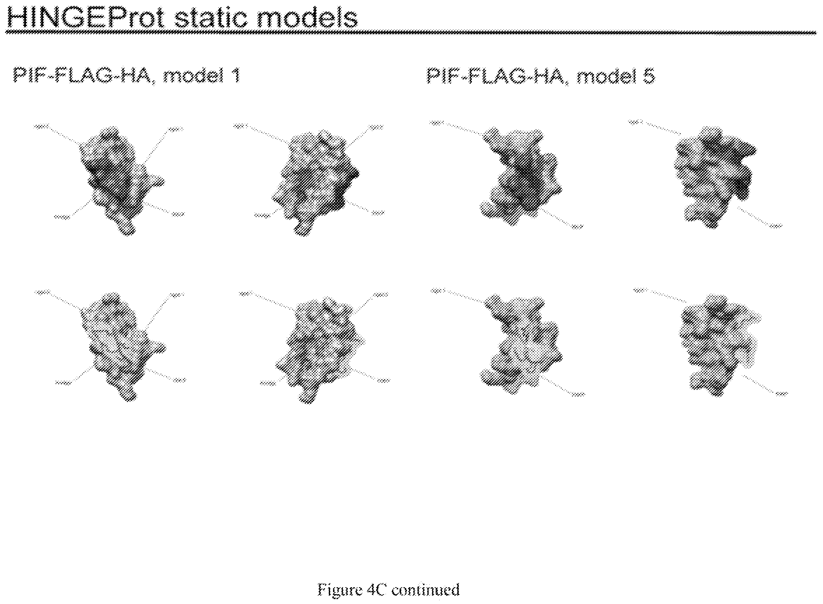

Subsequently, using I-Tasser, *ab initio* PDBs encoding the steric tertiary structure of the three PIF fusion peptide scenarios, each represented by the 5 most probable conformations were generated. To evaluate these models and select the one that would still expose the native PIF peptide physiological loop containing R-I-K-P, multiple structural alignments of the models within each of the peptide scenarios were employed. The two most distinct models within each group (model-1 and model-4, for FLAG-HA-PIF and FLAG-PIF-HA, and model-1 and model-5 for PIF-FLAG-HA) were subjected to the hinge analysis using the HingeProt server. Since proteins are highly flexible molecules, they can be regarded as moving parts using shear and hinge motion. While shear motions are very limited, hinge motions are similar to rotations around a joint, and can be very large. Therefore, it was necessary to evaluate whether the peptides could assume an open/close conformation that would hide or expose the R-I-K-P containing loop. The server generated 2 variants for FLAG-HA-PIF, having two or one rigid part(s) respectively, both having a hinge residue at amino acid 28. FLAG-PIF-HA had two different variants, one with 2 rigid parts, and one with 3 rigid parts and a hinge at 35 or at 11 and 29 amino-acid position, respectively. The PIF-FLAG-HA scenario had the same variants - 2 and 3 rigid parts, respectively. Using the Chimera software package the I-Tasser PIF fusion peptide full models were visualized, using ribbon diagram and overlaid molecular surface, showing the binding loops and possible grooves, which were colored based on rigid/hinge part classification. **Figure 4C** shows the hinge region assessment: Molecular Surface Map of PIF fusion hinged model conformations using I-Tasser server-generated PDBs, analyzed using HINGEProt server: in red, the R-I-K-P sequence, in orange and in blue, two rigid regions, and in magenta, the predicted hinge. The FLAG-HA-PIF had its R-I-K-P sequence deeply buried and only some of the sequence residues were externally exposed. The PIF-FLAG-HA and FLAG-PIF-HA had their R-I-K-P sequence better accessible. In FLAG-HA-PIF and PIF-FLAG-HA variants, even if rigid parts sterically move, there was still another coiled coil of the fusion molecule next to it, preventing an open-loop formation.

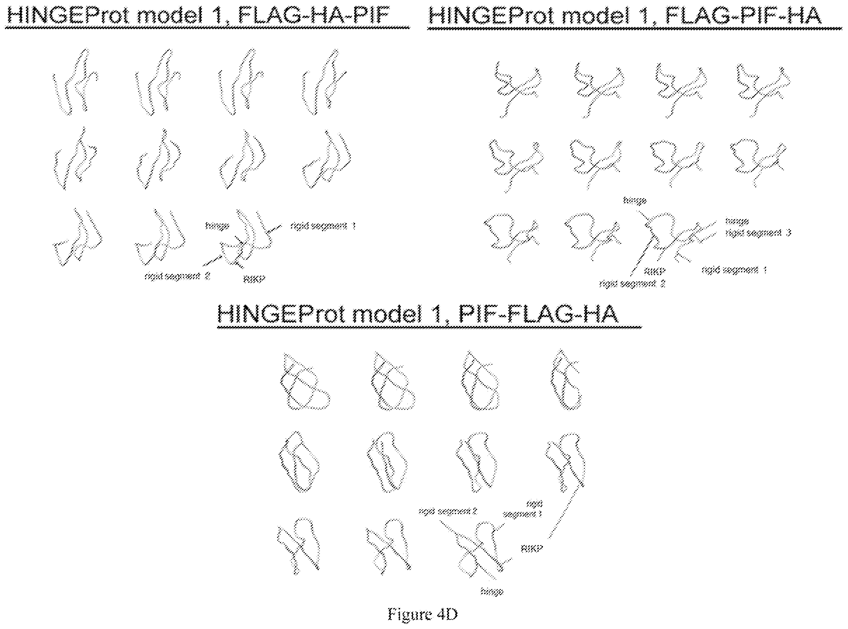

Creating a tiled C-only backbone set of models representing the conformation change, delivered from the HingeProt server, we found that FLAG-HA-PIF and PIF-FLAG-HA have less accessible R-I-K-P loops, since both have long and short rigid parts such that even if they sterically move, there is still another coiled coil of the fusion molecule next to them, preventing open-loop formations. **Figure 4D** illustrates HINGEProt modeled PIF fusion scenarios tiled from mode 1 to mode 10, showing the peptide bending. Conversely, in FLAG-PIF-HA peptide, the hinge is relatively centered in the molecule and allows for a high range of rigid segment movement, thus allowing for easier R-I-K-P loop accessibility.

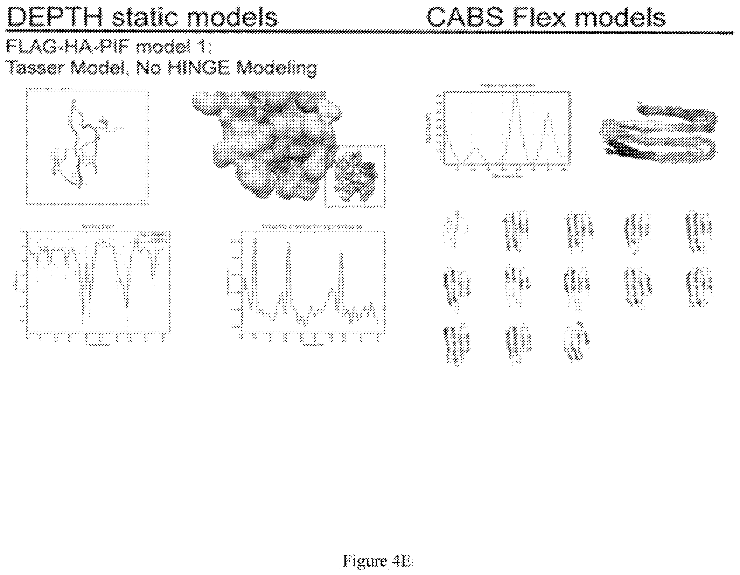

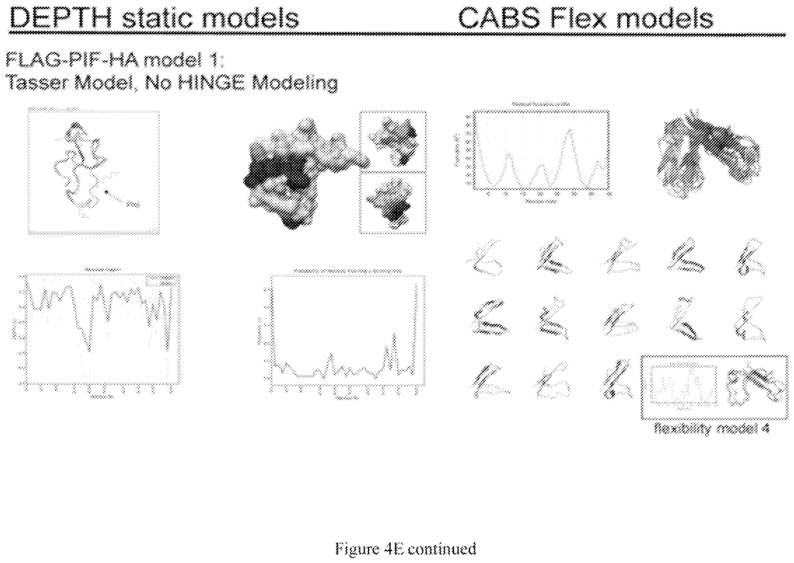

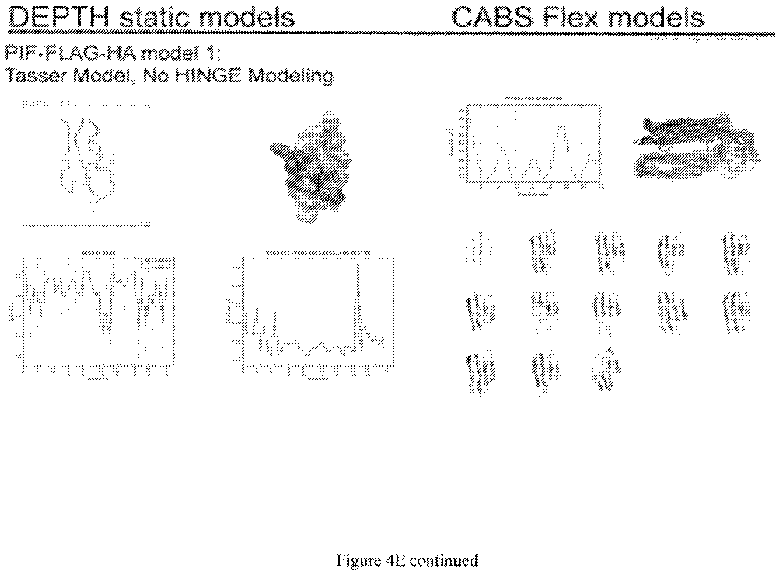

Considering HingeProt prediction accuracy, the depth of amino acid residues and their accessibility to outer molecules and water containing pockets formation was further evaluated as a more direct approach. The DEPTH server provided such information about the I-Tasser generated static PIF fusion models. See **Figure 4E****,** which shows a protein residue's accessibility and structure flexibility assessment: *Left:* I-Tasser model of PIF fusion was used to predict amino acid depth and accessibility. The surface model contains water molecules showing the deep hydrophobic pockets. *Right:* The same PDB was used for DEPTH modeling, and the obtained backbone structure was reversed to full model using ModRefiner to conform the subsequent CABS-Flex modeling of the putative flexibility structures. The R-I-K-P sequence is predicted as deeply situated inside the molecule in the FLAG-HA-PIF and PIF-FLAG-HA models.

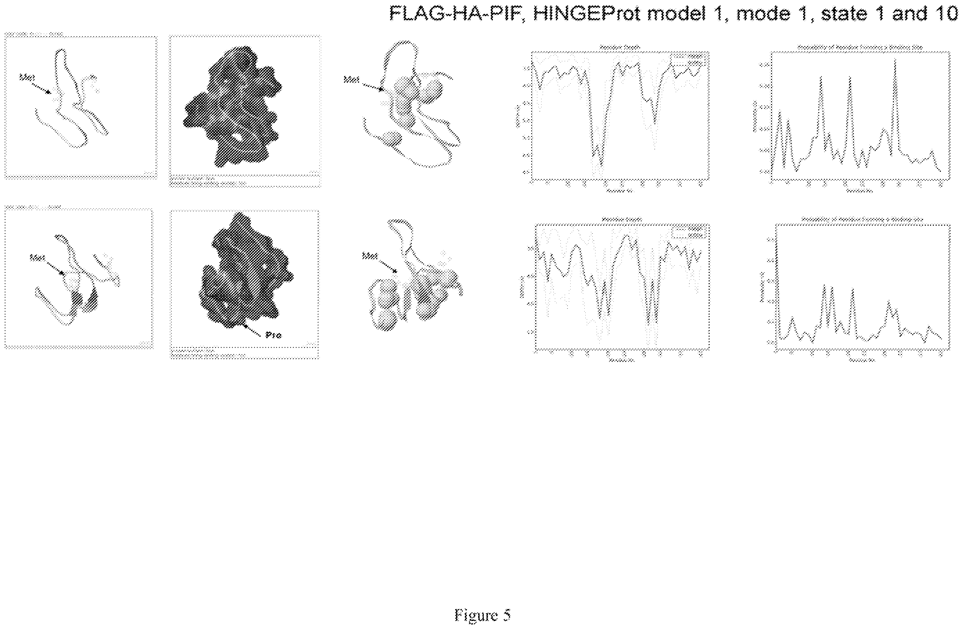

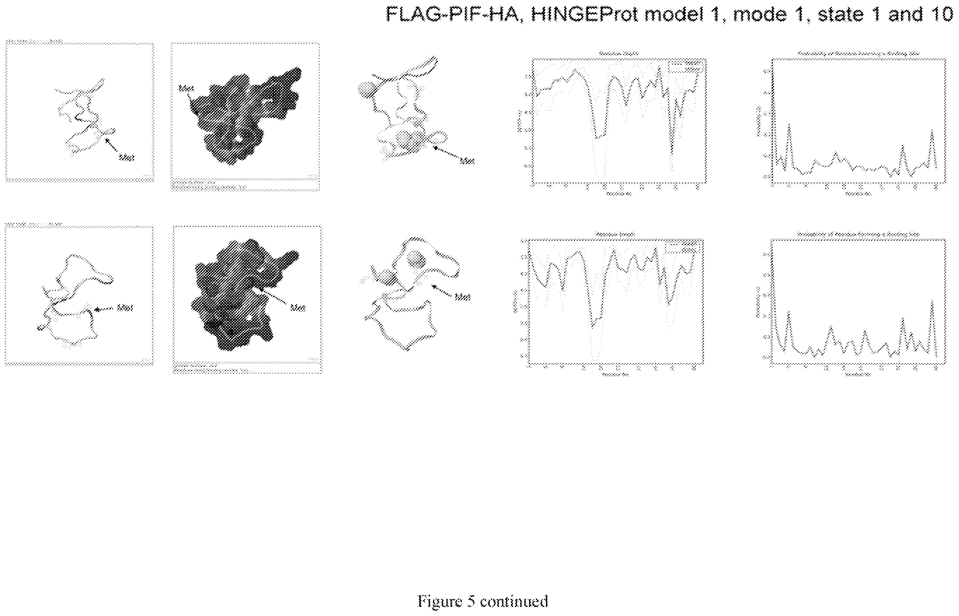

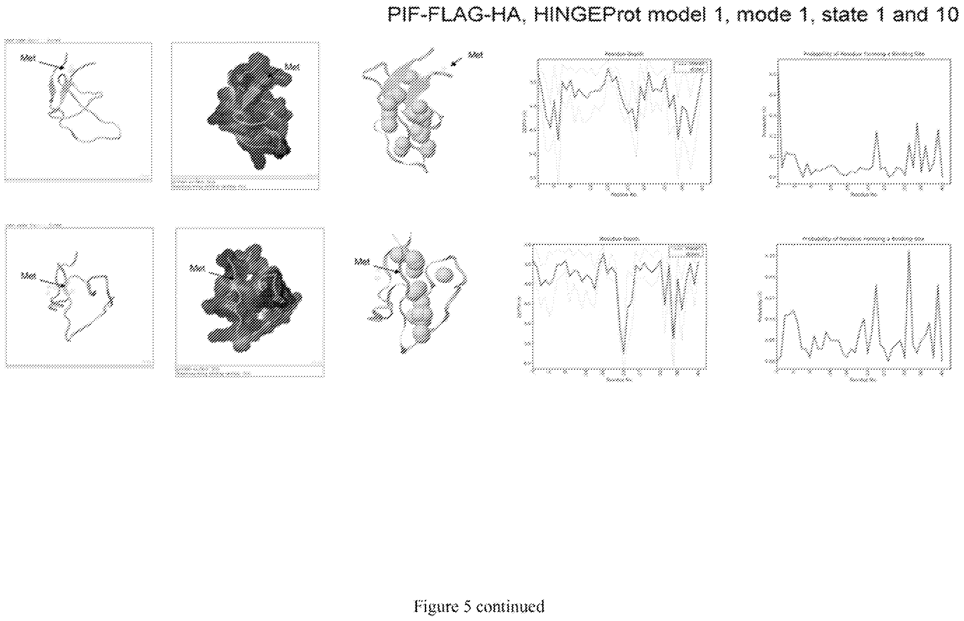

To evaluate if the depth and accessibility undergo changes during molecular conformation movement, ModRefiner-generated stabilized full-atomic models of HingeProt acquired C-only backbones were submitted to DEPTH server for analysis. The generated models, shown in **Figure 5****,** further supported the R-I-K-P steric interference in N- and C-tagged models.

Using a different approach for molecular fluctuation evaluation implemented by the CABS Flex server, the impact of weak forces on the molecular conformation was assessed (see **Figure 4E****).** The H-H bonds formed by the strands of FLAG-HA-PIF and PIF-FLAG-HA suggested more stable structures that integrate the R-I-K-P sequence within beta-sheet (or bulge)-like structures, while FLAG-PIF-HA was having its R-I-K-P externalized even when the molecule was packed by these weak forces. The fluctuation curves generated also backed the notion of flanking tag strategy as being better in terms of R-I-K-P accessibility.

This way, several lines of *in silico* PIF fusion scenarios structural assessment suggest that flanking fusion tags could be a better solution to the only N- or C- double fusion tag alternatives. The latter scenarios would most likely create unfavorable conditions for the PIF fusion peptide folding, causing deeper relocation within the molecular core, low solvent and interacting binding partner residue accessibility, stabilized by weak H-H bonds.

### Encoding of the PreImplantation Factor Fusion Peptide

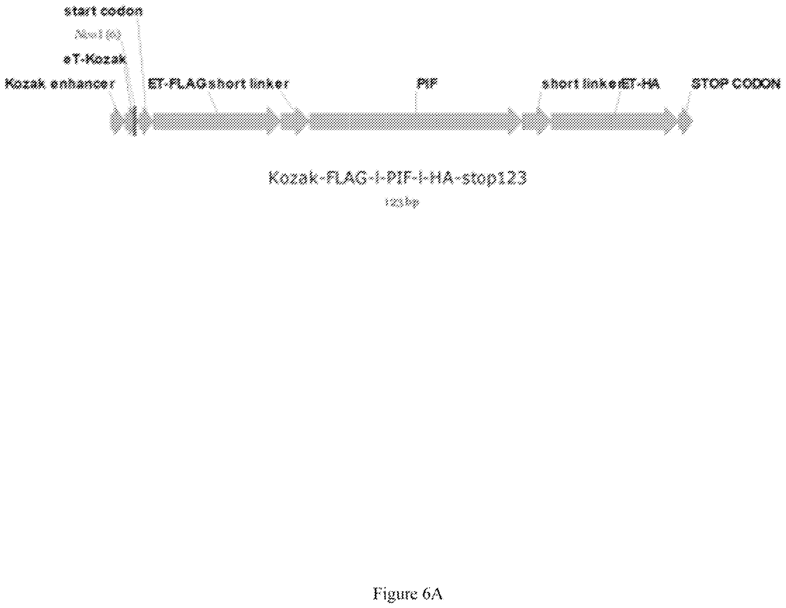

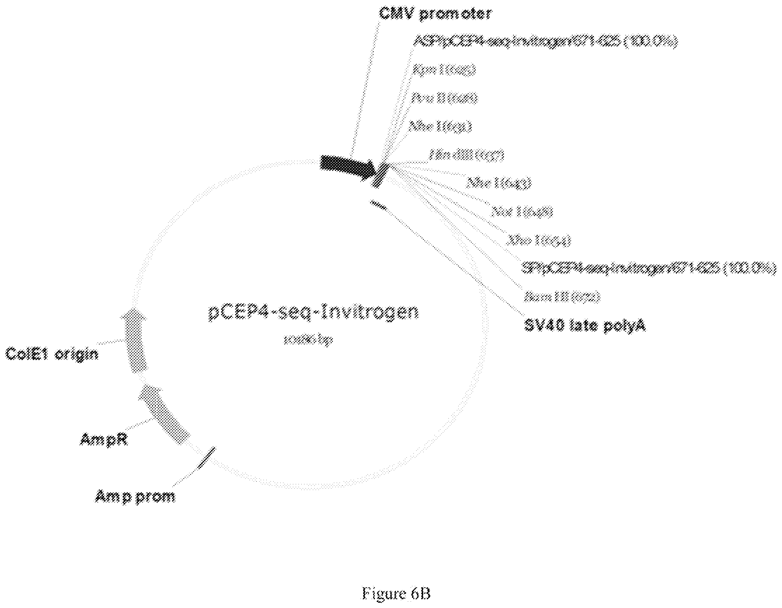

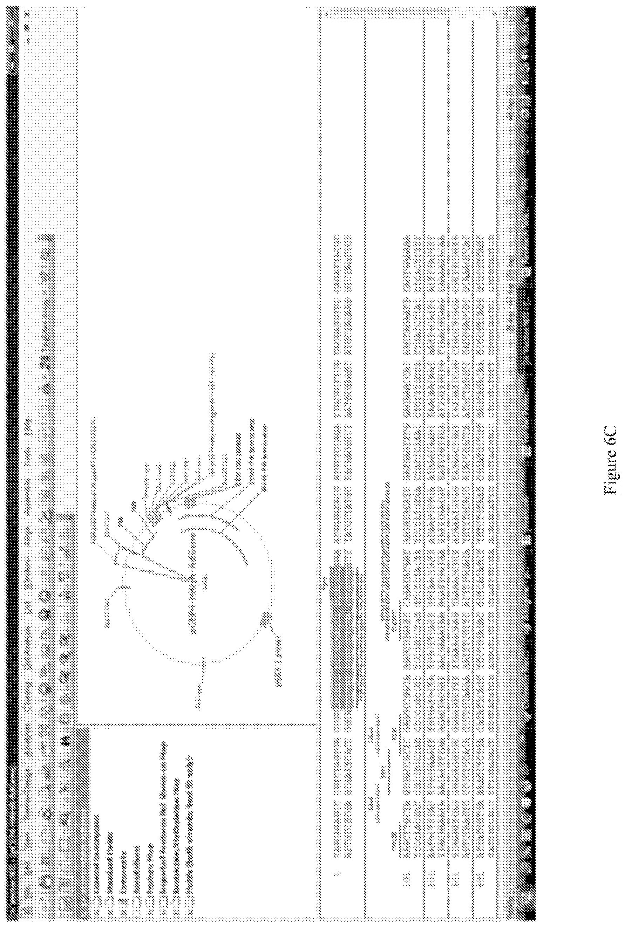

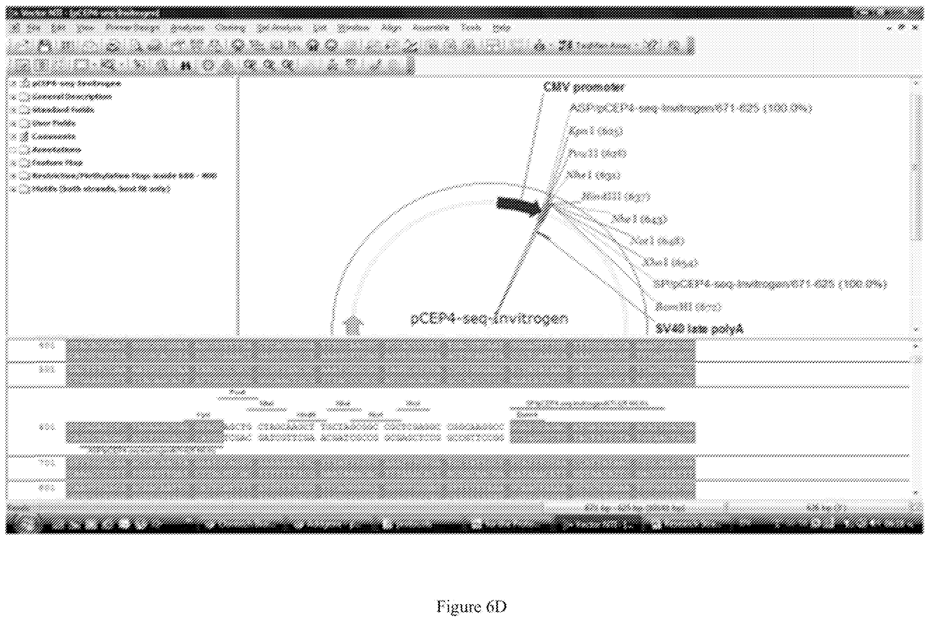

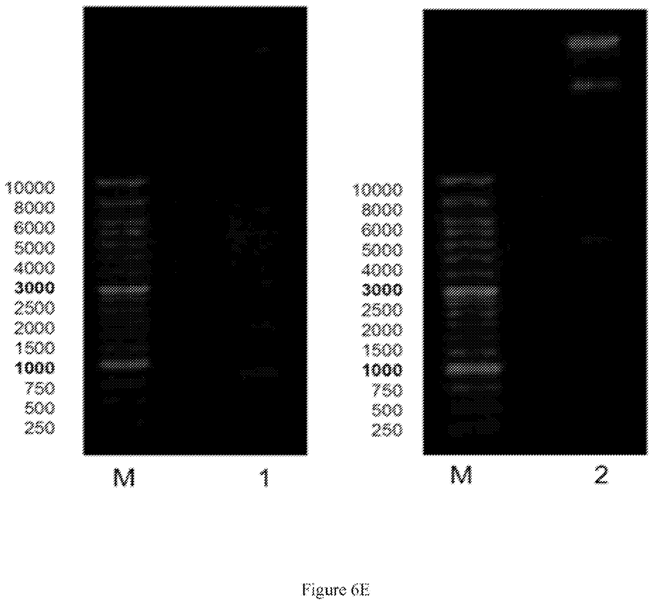

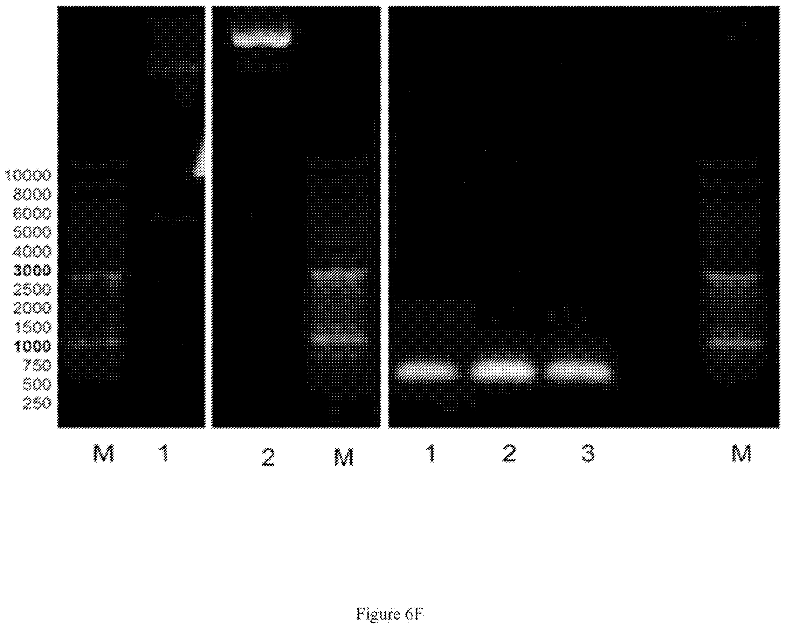

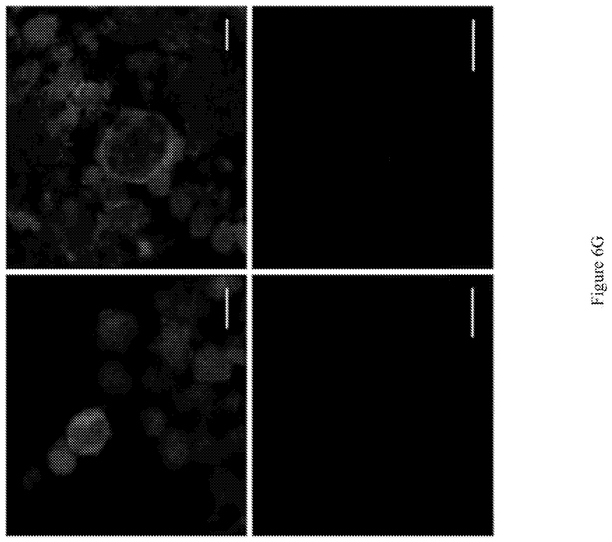

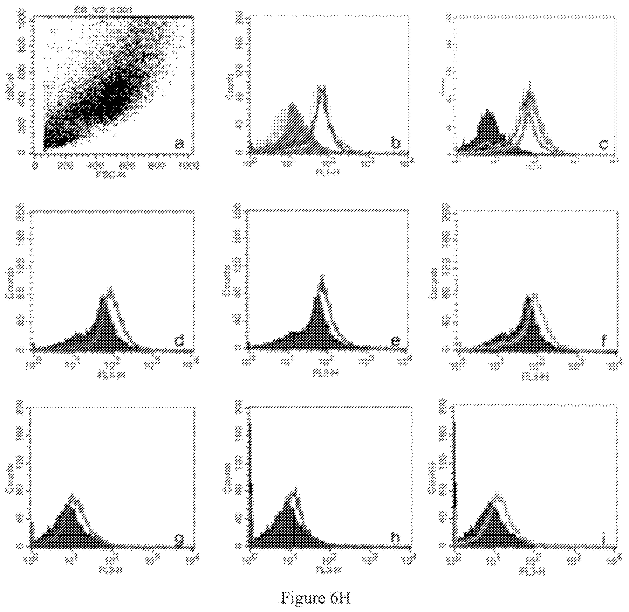

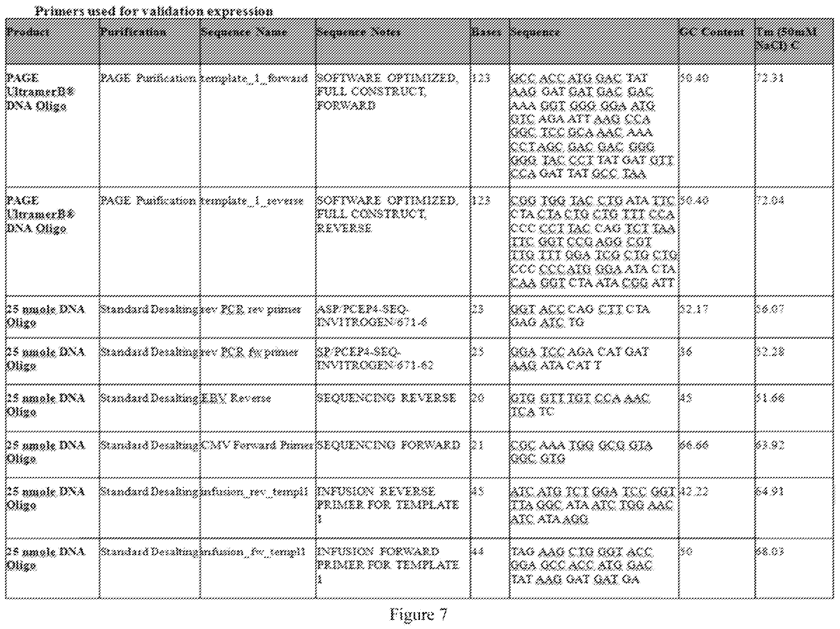

The fusion peptide selected for expression as the most flexible, flanked by FLAG and HA at both sides, was *in silico* encoded using the DNA 2 tool for codon optimization and synthetic expression transcript assembly. The transcript consisted of FLAG tag connected by short linker to the PIF sequence (SEQ ID NO:3; SEQ ID NO: 17) that in turn connected to an HA tag by the same type of short linker. The episomal mammalian expression vector pCEP4 employs a CMV promoter, and SV40polyA sequence. A Kozak sequence was introduced following the rules that regularly apply for Kozak sequence, namely the G or A at position -3 and the G at position +4 (relative to the ATG), which represents the most commonly occurring sequence with strong consensus and an additional Kozak transcription enhancing sequence. The sequence was then introduced into the Vector NTI. Figure 6 shows the Fusion peptide transcript structure, encoding FLAG short linker PIF short linker HA variant, as entered in Vector NTI. The modified pCEP4 vector had double HA tags inserted in the multiple-cloning site (MCS), between *Pvu II* and *Hind III* restriction sites. Using the original pCEP4 full sequence of Invitrogen (see **Figure 6B** showing the original Invitrogen pCEP4 vector diagram, depicting MCS, as entered in Vector NTI for *in silico* cloning) and the data provided by AddGene of sequencing the modified MCS, optimized primer pairs and protocol for reverse PCR were designed in order to discard the modified MCS, except for the selected for insertion restriction sites in it (BamH I and Kpn I). **Figure 6C** shows the Vector NTI screen showing the original MCS and the chosen MCS flanking restriction sites used for linearization primers design. See also **Figure 6D****,** which shows *in silico* reverse PCR: Vector NTI screen, showing 100% alignment between original vector and restriction site sequences of -20bp upstream of Kpn I and +20bp downstream of BamH I in AddGenep sequenced pCEP4-HAHA, where MCS contains two successive HA tags. The reverse PCR linearization primers designed using this alignment are also shown in **Figure 6D****.** **Figure 7** shows the primers used for validation expression.

The designed primers were validated *in silico* using the sequenced pCEP4 modified vector in order to determine if the reverse PCR would be effective using the modified pCEP4 as a template. The Clontech In-Fusion Oligo Design Tool was used for designing the fusion protocol primers, assuming that the reverse PCR linearized primer would have to reconnect at a virtual blunt end restriction site of ACC.GCA. The vector sequence (in bold and underlined are the KpnI and BamHI restriction sequences respectively, following at the same order): 5'... CAGATCTCTAGAAGCTG**GGTACC**AGCTGCTAGCAAGCTTGCTAGCGGC CGCT CGAGGCCGGCAAGGCCGGATCCAGACATGATAAGATACATT... 3' after the reverse PCR becomes linearized as 5'...CAGATCTCTAGAAGCTG**GGTACC**-3', and 5'-**GGATCC**AGACATGATAAGATACATT..3'. This sequence is therefore equivalent to
5'... CAGATCTCTAGAAGCTG**GGT*(ACC.GGA)*TCCA**GACATGATAAGATACATT...3'. As if ACC.GGA is restriction blunt enzyme required for an easier In-Fusion primer design tool. When the In-Fusion reaction takes place, this sequence was replaced by the seamless integration of the insert at the selected positions. See **Figure 6D****.**

The combination of *in silico* cloning design for applying the In-Fusion technology delivers seamless, highly selective directional cloning, removing the MCS and producing restriction-sites-free linearized vector, allowing for an easy peptide assembly. The host expression is further ensured by the crucial Kozak sequence and its enhancer, while the promoter, 5'and 3' UTRs are delivered by the expression pCEP4 vector (obtained from AddGene).

### Validation of PreImplantation Factor Fusion Peptide

The 120 bp synthetic gene template encoding for double tagged PIF fusion peptide, was ordered from IDT and synthesized using their proprietary high-fidelity synthesis system (Ultramer^{®} Oligonucleotides) for up to 200 bp oligos, and PAGE purified. All other oligos used did not require special purification and were ordered only desalted. The PIF synthetic fusion template was PCR amplified using a pair of In-Fusion Primer Design Tool (Clontech) designed primers (See **Figure 7****),** thus adding the flanking sequences required for the subsequent In-Fusion cloning reaction. **Figure 6E** shows the agarose gel electrophoresis of the synthesized in silico designed fusion insert, and initial pCEP4-HAHA cloning vector: **lanes M:** molecular marker; **lane 1:** synthetic gene template of size about 120 bp encoding for double flanking tags fusion showed at a., PCR amplified with fusion primers, adding sequences for the subsequent cloning reaction; **lane 2:** double HA tags containing plasmid, after reverse PCR linearization resulting in HA tags removed. The 17 nt long homology regions (non-annealing 5 ' primer overhangs) needed for integration by In-Fusion cloning are underlined. The pCEP4(+) plasmid with modified MCS was linearized by reverse PCR using the primer pair designed above (see **Figure** 7).

*DpnI* digested spin column purified PCR amplified insert (50 - 200 ng) and *DpnI* digested, spin column purified reverse PCR linearized plasmid (100-250ng) were mixed, volume adjusted with water to a final volume of 10µl, and incubated with dry-down In-Fusion mix (Clontech) at 42°C for 30min. **Figure 6F** shows the agarose gel electrophoresis of cloned products: lane 1: DPN I degradation of the original plasmid DNA, leaving only the reverse PCR amplified plasmid; lane 2: original plasmid DNA before DPN I treatment; lanes 3, 4, 5 PCR amplification products using the cloned PIF fusion gene vector peptide as template and the original fusion primers. The fusion cloning was performed twice and resulted in 80-120 colonies. From each experiment several colonies (4-6) were PCR validated using the cloned column purified plasmid as template and in-fusion template amplification primers (see **Figure 6F****,** lanes 3-5). Negative controls consisted only of linearized plasmid treated with the dry-down In-Fusion mix in one case, and only of insert treated with the dry-down In-Fusion mix in the other case. The control containing only plasmid resulted in only 2-5 colonies. PCR validation was negative. The control containing only insert did not result in any colonies. One of the most important steps in the procedure is to remove template plasmids as effectively as possible to reduce the number of colonies containing parental plasmid.

The product of seamless cloning is validated by the insertion of specific primers and analytical PCR, which was achieved by using fast, high-yield technology. During the process, the initial template DPN I digestion ensures the initial products' contamination-free results.

### Materials and Methods

### PIF Fusion Peptides in silico Modeling and Evaluation

PEP-FOLD server for *de novo* peptide structure prediction of native PIF out of its primary structure (amino-acid sequence (SEQ ID NO:3)) was used. Due to amino acid sequence length limitations of PEP FOLD (n=36), the I-TASSER template based multiple-threading alignments and limited *ab initio* prediction of non-aligned segments service were used for protein structure prediction of double tagged FLAG-HA PIF fusion peptide.

Rigid protein parts and the flexible hinge regions connecting them in the native topology of protein chains was predicted using the HINGEProt Server, which employs single static conformation and elastic network models of movement, utilizing Gaussian Network Model (GNM) for residues fluctuations in the most dominant (slowest) regions and Anisotropic Network models (ANM) for the direction of the fluctuations of the corresponding rigid parts connected by the hinge region. The server output consists of a C-only backbone model.

Using SCRATCH Server secondary structure predictions for the model sequences, following the scheme of DSSP (Define Secondary Structure of Proteins) an algorithm was obtained.

For evaluation of protein-protein interaction interfaces, the DEPTH server (algorithm) was employed. It provided data for the distance of the residue(s) from the protein surface and accurate calculation of cavity volumes in proteins.

In addition to the full molecular model produced by I-Tasser, the DEPTH server was used for depth prediction of the residues accessibility of models predicted by the HingeProt server. The C-only backbone models delivered by HINGEProt were processed using ModRefiner to reconstitute the full amino-acid chain and then were re-submitted to the DEPTH server.

Structure flexibility was further simulated using the CABS-flex server, employing a well-established coarse-grained protein modeling tool. The output protein dynamics is described by a set of protein models (in an all-atom resolution PBD format) reflecting the most dominant structural fluctuations in the near-native ensemble, residue fluctuation profile (showing the relative propensity of protein residues to deviate from an average dynamics structure), and RMSD profiles (displayed for each representative model, showing the deviation of protein model residues from the input structure).

This comprehensive flow allowed for the creation and refinement of several most likely structural models of both native and double fusion tagged PIF scenarios, that were evaluated *in silico* for their secondary structures, ability to bind, forming hinges, their dynamic conformational fluctuations, eventually restrict by weak H-H bonds, and individual residue accessibility for external binding.

### PIF Fusion Peptide Cloning Oligonucleotides

All polymerase chain reactions were carried out on a Stratagene MX3005P qPCR machine. The final volume of typical PCRs was 50 µL. PCR primers were synthesized by IDTDNA, (CA, USA). PAGE purified oligonucleotides were used for the in-Fusion experiments. The PIF fusion was ordered as two separate synthetic PAGE purified lyophilized oligoes using the proprietary Ultramer IDTDNA synthesis technology. Primers for insert amplification were designed to anneal to these sequences. A non-annealing 5'-tail was added to each primer. This tail carried the 15-20 nucleotides long homology to the site of insertion in the plasmid.

The oligonucleotides for vector linearization/amplification did not contain any non-annealing regions. They were designed to anneal to different strands of the plasmid, with their 5'-end being homologous to the 15-20 nucleotides long non-annealing tails of the insert primers. The primers are shown in **Figure 6****.**

### Cloning Vector Reverse PCR Linearization

An initial denaturation step for 3 min at 98°C was followed by 25 cycles of 30 s at 98°C (denaturation), 60 s at 50-70°C (primer annealing) and 60 s/kb template 72°C (elongation). The final elongation step was carried out at 72°C for 10 min. Twenty to sixty nanograms of template plasmid, 1 µM of each primer, 360 µM dNTPs, and 1U of Phusion^{®} Hot Start Flex Polymerase (Cat. No. M0536S) were used for vector linearization. Most vectors used were purchased from AddGene modified pHAHA Invitrogen pCEP4 plasmid. The obtained linearized vector was further used for the In-Fusion Cloning reaction.

### In-Fusion Cloning of PIF Fusion Peptide

Initial denaturation was carried out at 98°C for 90 s, followed by 30 cycles of 98°C for 30 s, 55°C for 30 s, and 30 s/kb at 72°C. The final elongation step was carried out at 72°C for 5 min. 0.5-5 ng of template plasmid DNA, 1 µM of each primer, 250 µM dNTPs, and 1U of Phusion^{®} Hot Start Flex Polymerase (NEB, Cat. No M0536S) was used for insert amplification. PCR products were examined on 1.5% agarose gels (Geneaxxon, Germany). The PCR products were incubated with 20U Dpn I (New England Biolabs, Cat. No. R0176S) at 37°C for at least 2 hrs in presence of NEBuffer 4 (NEB, Cat. No.B7004S) to digest the template DNA. Dpn I specifically cleaves dam methylated DNA, allowing cleavage of the template DNA without affecting unmefhylated PCR products. The PCR products were then spin column purified using the PCR DNA Purification Kit (Cat. No. 740609.10) and eluted in 50 pi of 10 mM Tris, pH 8.5. 50- 200 ng of PCR amplified insert and 100-250 ng of PCR linearized plasmid were mixed, volume-adjusted with water to a final volume of 10 pi, and incubated with dry-down In-Fusion mix (Clontech, Cat. No. 639690) at 42°C for 30 min.

The mixtures were then placed on ice for 5 min. Next, 1 µl of the mixture was used to transform Clontech Stelar chemically competent E. coli cells. The heat shock was carried out at 42°C for 45 s. Two hundred microliters of S.O.C. medium were added to the cells, and the mixtures were incubated for 1 hr at 37°C, 650 rpm in a Memmert Incubator.

The complete transformation reaction mixtures were plated. Plasmid DNA from the resulting colonies was purified using Mini Plasmid Prep Kit (Mo Bio, Cat. No. G-3143-50) standard protocol. Positive clones were identified by analytic PCR using Mini Plasmid Prep isolated DNA as template, with the same amplification protocol as described for insert amplification. The analytical PCR products of positive clones were spin column purified using the NucleoSpin Gel and PCR Clean-Up Kit (Cat. No. 740609.10) and sequenced. The obtained plasmid contained a properly inserted double tagged fusion peptide, which was further validated using PCR. The primers used for analytical PCR of modified pCEP4 plasmid are all shown in **Figure 6****.** The validation of the bacterial clones was based on the expected size of the analytical PCR product, using insert-specific primers.

### PIF Fusion Peptide Plasmid Transfection in HEK293

The selected PIF fusion peptide was transiently transfected in **HEK293** cells seeded in either six well plates or in LabTek slides (Sigma) for 48 hrs, following standard Qiagen protocol using Attractene reagent and 1 ug DNA. The obtained transiently transfected cells expressed the FLAG-HA tagged PIF peptide intra-cellularly and were cultured for more than 72 hrs without signs of toxicity or cell death.

### Antibodies used for PIF Flow Cytometry and Imaging Validation

For this study either Biotin-conjugated mouse anti-PIF monoclonal antibody, (proprietary of Biolncept LLC) raised against solid-phase synthesized PIF sequence or a commercial anti-FLAG (eBioscience, US) was used to detect the tagged PIF fusion peptide . The specificity of the first was confirmed by numerous PIF studies, and the second was confirmed by SIGMA high specificity and acidity antibody.

### Flow Cytometry Assessment of PIF Fusion Peptide in vitro Expression

For the double FLAG-HA tagged PIF fusion peptide expression evaluation, the HEK293 cells transfected for 72 hrs in 6 well plates (Orange Scientific) were subject to intracellular flow cytometry. PIF fusion peptide abundance was assessed either using biotinylated mouse anti-PIF monoclonal antibody (0.4 µg/10⁶ cells) (Biosynthesis, Lewisville Tx), or using a commercial monoclonal anti-DYKDDDDK (FLAG) tag antibody (1 µg/10⁶ cells) (eBioscience, US). The specific binding was subsequently detected by Fluorescein isothiocyanate (FITC)-conjugated Streptavidin (0.25 µg/10⁶ cells), or FITC conjugated anti-mouse antibodies respectively (Santa Cruz). Isotype control antibodies (0.25 µg/10⁶ cells) were raised in the same animal species used to produce the first antibodies (Santa Cruz). Control non-transfected and PIF peptide transiently transfected HEK293 cells were detached using Accutase^{™} (eBioscience, US) and washed with cold 1% BSA-PBS (0.1% (w/v)). After IC Fixation/Permeabilization Solution (eBiosciences) wash, and Flow Cytometry Staining Buffer block (eBiosciences), the specific primer or the appropriate isotype control antibodies were used for 30 min on ice, followed by Flow Cytometry Staining Buffer wash, and respective incubation either using a second antibody or Streptiavidin-FITC for 30 min on ice (in the dark). After washing, cells were gated using forward vs. side scatter to exclude dead cells and debris, collected and analyzed with a BD FACSCalibur flow cytometer (Becton Dickenson). Fluorescence of 10⁴ cells per sample was acquired in logarithmic mode for visual inspection of the distributions and for quantifying the expression of the relevant molecules by calculating the median fluorescence intensity (MFI). This approach allowed for quantitative estimation of expression levels of the fusion peptide after transient transfection in comparison to empty cassette vector transfection or non-transfected conditions, as well as for anti-PIF or anti-FLAG-based detection systems specificity evaluation.

### Immunofluorescent Confocal Imaging of in vitro Expressed PIF Fusion Peptide

The next day, the HEK293 cells seeded on LabTek slides (Sigma) were transiently transfected either using the PIF fusion peptide or an empty pCEP vector cassette. The cells were fixed with 4% (w/v) paraformaldehyde on ice for 20 min and made permeable with 0.2% (v/v) Triton-X 100 in PBS for 15 min. Cells were blocked with 2% (w/v) BSA-PBS for 1 hr. The cells were incubated with the same anti-PIF monoclonal antibody used for flow cytometry at a dilution of 1:100 in PBS overnight, at 4°C, and after 3 PBS washes they were incubated for lhr in the dark, using FITC conjugated anti-mouse IgG antibody at a dilution of 0.25 pg/100 pi. For cell nuclei staining, 4',6-diamidino-2-phenylindole (DAPI) (Sigma) was used at a 1:250 dilution in PBS. The slides were mounted with a Vectashield mounting medium (Vector Labs), covered by coverslips, and sealed with Marabu Fixogum rubber cement. For image acquisition, a Leica TCS SPE laser scanning confocal microscope was used with an apochromatic, violet corrected, 40x, 1.25 numerical aperture oil immersion objective and acquisition software (Leica). Images were acquired in green channel using two-way sequential line scans, providing localization data on the PIF fusion expression. **Results**

The HEK293 cells were transiently transfected with peptides purified from isolated single colonies acquired from each of the two independent transformations. When compared to an empty pCEP4 transfected HEK293 cells, cells transfected with fusion peptide expressing pCEP4-FLAG-PIF-HA expressed PIF. This was demonstrated by probing with an anti-PIF monoclonal antibody which was subsequently visualized by confocal microscopy using a secondary FITC conjugated anti-mouse polyclonal antibody. PIF was expressed predominantly at the cell membrane as observed by the **presence** of bright immunofluorescence. **Figure 6G** shows confocal immunofluorescence validation of PIF fusion transfect expression: background-corrected negative control (non-transfected HEK293, anti-mouse-FITC), compared to transfected HEK293 cells (with the same background settings on the confocal microscope), and reacquisition of the same specimen location using accumulation = 2 (the image is the sum of 2 consecutive acquisitions).

The expression of the double-tagged fusion PIF by flow cytometry (FCS) was validated. Each of the separate transformation-derived peptides was independently **used** for transient transfection of HEK293 cells. Cells that were intact were used for initial gating. For detection of the PIF fusion, either a biotin-conjugated PIF-specific antibody visualized by Strepavidin-FITC or an anti-FLAG-specific monoclonal antibody visualized by an FITC-conjugated anti-mouse polyclonal antibody was used.

The controls were represented by one of the following - empty pCEP4 transfected HEK293 without antibody treatment, or treatment using either secondary detection systems or full detection - specific primary antibody against PIF or against FLAG and secondary detection antibody. All demonstrated an **MFI** lower than the pCEP4-FLAG-PIF-HA-transfected HEK293 cells treated with the full detection system. When only the secondary detection system was applied, the fluorescence channel histogram was similar to the one obtained without transfection. See **Figure 6D****.**

**Figure 6H** shows the flow cytometry validation of PIF fusion transfect expression. **Figure 6H****(a)** shows a 2D scatter of non-transfected HEK293 cells, gated by size and scatter. **Figure 6H**(b) shows a histogram overlay (FL1-H) of non-transfected HEK293 cells, no Ab treatment (light gray shade); non-transfected cells, treated with FITC conjugated anti-mouse Ab (dark gray shade); non-transfected cells, treated with FITC conjugated Streptavidin (black line); non-transfected cells treated with anti-FLAG mAb and FITC conjugated anti-mouse Ab (yellow line); non-transfected cells treated with anti-PIF mAb and FITC conjugated Streptavidin (red line); HEK293 cells transfected with peptide-1, treated with FITC conjugated Streptavidin only (green). **Figure 6H**(c) shows a histogram overlay (FL1-H) of HEK293 cells transfected with peptide-1, treated with anti-FLAG mAb and FITC conjugated anti-mouse Ab **(yellow);** HEK293 cells transfected with peptide-1, treated **with** anti-PIF mAb and FITC conjugated Streptavidin (orange); HEK293 cells transfected with peptide-2, treated with FITC conjugated Streptavidin only (brown); HEK293 cells transfected with peptide-2, treated with anti-FLAG mAb and FITC conjugated anti-mouse Ab (dark brown); HEK293 cells transfected with peptide-2, treated with anti-PIF mAb and FITC conjugated Streptavidin (black); HEK293 cells transfected with both peptides (1 and 2), treated with FITC conjugated Streptavidin only (magenta); HEK293 cells transfected with both peptides (1 and 2), treated with anti-PIF mAb and FITC conjugated Streptavidin (green). **Figure 6H** also shows histogram overlays of (d) HEK293 cells transfected with peptide-1, treated with anti-FLAG mAb and FITC conjugated anti-mouse Ab (magenta); (e) HEK293 cells transfected with peptide-2, treated with anti-FLAG mAb and FITC conjugated anti-mouse Ab (dark red); (f) HEK293 cells transfected with both peptides (1 and 2), treated with anti-FLAG mAb and FITC conjugated anti-mouse Ab (green); histogram overlays of (g) HEK293 cells transfected with peptide-2, treated with anti-PIF mAb and FITC conjugated Streptavidin (magenta); (h) HEK293 cells transfected with peptide-2, treated with anti-PIF mAb and FITC conjugated Streptavidin (dark red); and (i) HEK293 cells transfected with both peptides (1 and 2), treated with anti-PIF mAb and FITC conjugated Streptavidin (green); control (blue shade) represents non-transfected cells either treated with anti-FLAG mAb and anti-mouse FITC conjugated or treated with anti-PIF mAb and FITC conjugated Streptavidin.

Transient expression of PIF was demonstrated in both global cell populations and individual cells, remarkably with no signs of cell death and toxicity. Given the short time post-transfection, and the mixed cell population, without prior antibiotic selection, the expression levels assessed by flow cytometry could be considered sufficient.

The comprehensive strategy for synthetic gene design, sequence and structural evaluation and best tagging scenario selection provides a streamlined, fast and efficient approach for designing a gene sequence lacking known peptides or proteins for single or high-throughput assessment of target of interest peptide-protein binding studies.

### EXAMPLE 2

### Determination of PIF targets, sites of interaction and effect on associated PBMCs gene expression

PIF targets were determined using ProtoArray^{®} followed by *in silico* structure-based analysis. PIF interaction with PBMCs was assessed by using global gene expression analysis. PIF interacts with major targets by binding to their WXiWX₂X3X4REWFX5X₆X₇W (SEQ ID NO: 28) amino acid sequence and PIF exerts a functional auto-regulatory effect on PBMCs FX enzyme expression. Through such multi-targeted, highly adaptive interaction, PIF controls both naive and challenged immune regulation.

### Materials and Methods

### PIF Peptide Synthesis

Synthetic PIF (MVRIKPGSANKPSDD) produced using solid-phase peptide synthesis (Peptide Synthesizer, Applied Biosystems, Foster City, CA) employing Fmoc (9-fluorenylmethoxycarbonyl) chemistry. Final purification was carried out by reversed phase high-pressure liquid chromatography (HPLC), and peptide identity was verified by mass spectrometry. **Alexafluor647-PIF** conjugate was generated (Bio-Synthesis, Inc., Lewisville, TX).

### Aiexa-PIF Interaction with Human Proteome Array

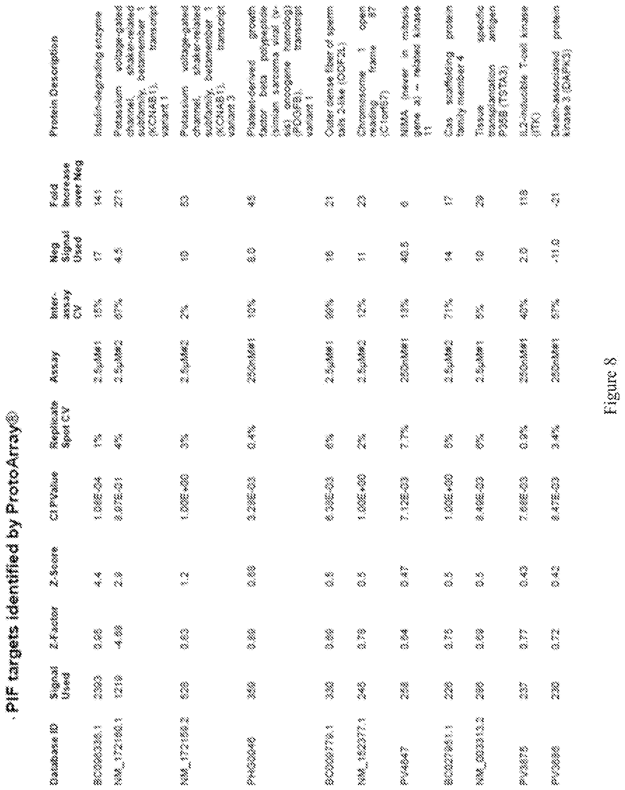

ProtoArray^{®} Human Protein Microarrays v 5.0 (Invitrogen, Carlsbad, CA) with 9000 possible targets were initially blocked followed by exposure to PIF-Alexa 647 (250nM and 2.5pM). Unbound probe was washed off, and arrays were assessed by a fluorescence microarray scanner. See **Figure 8****,** which shows the PIF targets identified by ProtoArray^{®} Human Protein Microarrays v5.0. As a negative control, an array was incubated with wash buffer while calmodulin kinase was used as a positive control detected by Alexa Fluor 647-conjugated anti-V5 antibody. Three stringent conditions determined Alexa-PIF specificity of binding: signal value >200 relative fluorescent units, >6 times higher than the negative control, and Z-Factor > 5. The absolute value of the ratio was used since some negative control features had a negative Signal Used value. The Z-Factor, or signal-to-noise ratio, was greater than 0.5 on the corresponding array, indicating a signal greater than 2-fold above the noise.

### In silico Modeling of the PIF Binding to Protein Targets

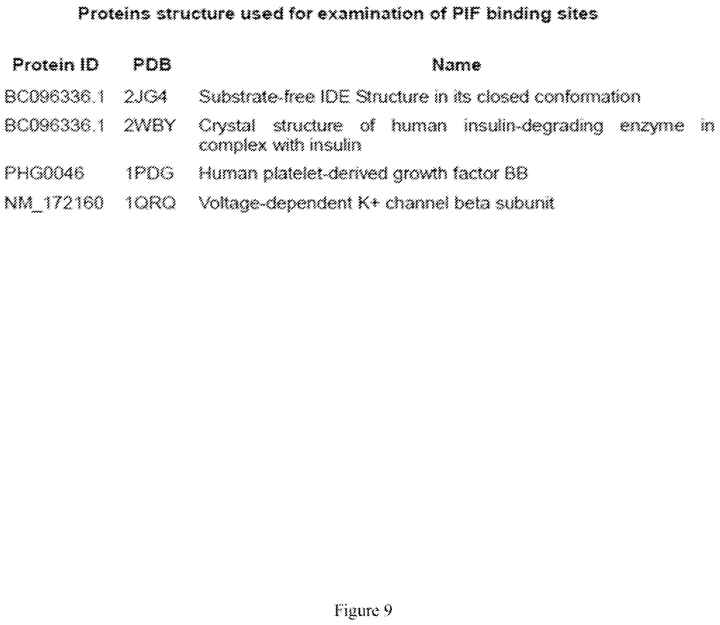

PepSite 2 server was used for *in silico* prediction of PIF peptide binding sites on protein surfaces, referred to herein as PepSite. The following PDBs of the protein targets were used. **Figure 9** shows the protein structures used for the examination of PIF binding sites. As the PepSite server is limited to 10 amino acids per peptide sequence, only the first 10 amino acids were used, as it was shown previously that the first 9 amino acids are shared between the two biologically active PIF forms, both having the same actions. Rosetta FlexPepDock server for high-resolution peptide docking was used, having as input, a PDB file, consisting of the PIF protein target as a first chain - the voltage dependent K+ 110 ion channel, model PDB 1QRQ and PIF peptide conformation predicted *ab initio* using the Pep-Fold server as a second chain. FlexPepDock allows full flexibility to the peptide and side-chain flexibility to the target protein, thus providing accurate refinement of the peptide structure, starting from up to 5.5A RMSD (root-mean-square deviation, the measure of the average distance between the atoms (usually the backbone atoms) of superimposed proteins) of the native conformation.

All protein image rendering and the multiple sequence alignment (MSA) following multiple protein structural alignment (superposition) was performed using Chimera v. 1.8c (UCSD, US). A local Smith-Waterman alignment algorithm, BLOSUM 35 matrix, 8090% secondary structure score inclusion, and 3 A iterative matching, were all used for the structural alignments. MSA was performed using superposition and circular permutation, with residue-residue distance cutoff of 5 A. MultiProt server was used for partial multiple alignment of the protein targets identified by ProtoArray^{®}, using an algorithm for the local multiple structural alignment of common geometrical cores.

### PBMCs Culture

Blood samples without identifiers were obtained from males and nonpregnant females after obtaining consent at MDSPS, WA. The studies were approved by the ethical committee at the institute. From the excess specimen, PBMCs were isolated using standard Ficoll Hypaque, a density gradient method. PBMCs in duplicate (200,000/well) were cultured with +/-PIF 50nM for up to 48 hrs and were compared with PBMCs stimulated with plate-bound anti-CD3 and anti-CD28 MAb 10µg/ml, +/- 50nM PIF and cultured for up to 48 hrs in a serum-free medium (GIBCO^{™} AIM-V Medium, Invitrogen Corp., Carlsbad, CA). Samples were collected in duplicate at 24 and 48 hrs and used for global mRNA analysis.

### PIF Effect on PBMCs Global mRNA Analysis

After culture, PBMCs were collected and the chemically fragmented cRNA was then hybridized on Affymetrix HGU133 Plus 2.0 human chips in a GeneChip Hybridization Oven 640 (Affymetrix) and screened for 18,400 human genes and expressed-sequence tags, followed by fluorescence labeling with Fluidics Station 450 (Affymetrix) and optical scanning with an Affymetrix GeneChip Scanner 3000 (Codon Biosciences, LP, Houston, Texas). PO.05 and > 2-fold changes were considered as significant. Data is deposited at NCBI Geo (GSE18291).

### PIF Interaction with Kinases: ITK and NEK11 Activity Analysis

ITK human origin enzyme was incubated with 8 mM MOPS pH 7.0, 0.2 mM EDTA, 0.33 mg/mL myelin basic protein, 10 mM MgAcetate and [y-33PATP] (specific activity approx. 500 cpm/pmol, concentration as required). PIF at different concentrations was added and the reaction was initiated by the addition of the MgATP mix. After incubation for 40min at room temperature the reaction was stopped by the addition of 3% phosphoric acid solution. lOµL of the reaction was then spotted onto a P30 filtermat and washed three times for 5min in 75mM phosphoric acid and once in methanol prior to drying and scintillation counting.

### ZIPK Activity Analysis

ZIPK human origin was incubated with 8mM MOPS pH 7.0, 0.2 mM EDTA, 250 µM KKLNRTLSFAEPG, lOmM MgAcetate and [y-33PATP] (specific activity approx. 500 cpm/pmol, concentration as required). PIF at different concentrations was added and the reaction was initiated by the addition of the MgATP mix. After incubation for 40min at room temperature, the reaction was stopped by the addition of 3% phosphoric acid solution. 10 µL of the reaction was then spotted onto a P30 filter mat and washed three times for 5min in 75 mM phosphoric acid and once in methanol prior to drying and scintillation counting. Studies were carried out by Millipore (UK).

### Statistical Analysis

For gene experiments, data was first analyzed using student's t-test, with p<0.05 results, followed by fold-change testing, with a cutoff 2- fold only reported.

### Results PIF

### Specifically Binds to Limited Protein Partners (11)

Using Alexa-PIF as a ligand, binding to 9000 human protein targets by ProtoArray^{®} was examined. PIF binds only to limited number of proteins, reflecting high binding specificity. A total of 11 candidates were identified. Beyond PIF binding to IDE and Kvl.3b, two variants which was previously reported, one major and seven minor candidates were additionally identified. These proteins met the threshold criteria and were visually confirmed on at least one array. **Figure 8** shows the additional values calculated by the ProtoArray^{®} Prospector software were incorporated into the data analysis, including the Z-Score and CI P-value. The Z-Score, or normalized signal, indicates the number of standard deviations above or below the median signal value for all human proteins featured on the array. Of the 11 proteins identified as interactors with PIF-Alexa 647, IDE and Kvl.3b two variants had a Z score greater than 2 and one had a Z-Score value greater than 3. The CI PI72 Value assigns a probability that an observed signal is derived from distribution of signals arising from a set of defined negative controls. A CI P-Value O.05 correlated with a visually confirmed signal on the array. Seven of the 11 candidate binding proteins identified on arrays probed with PIF-Alexa 647 had CI P< 0.05.

### PIF Targets Linearized Consensus Amino Acid Residues Equivalent to WX₁WX₂X₃X₄REWFX₅X₆X₇W (SEQ ID NO: 28) of its Structural Target Potential (Putative) Binding Pockets Using its R-I-K-P sequence with Highest Probability -Structural Consensus and Probability Analysis

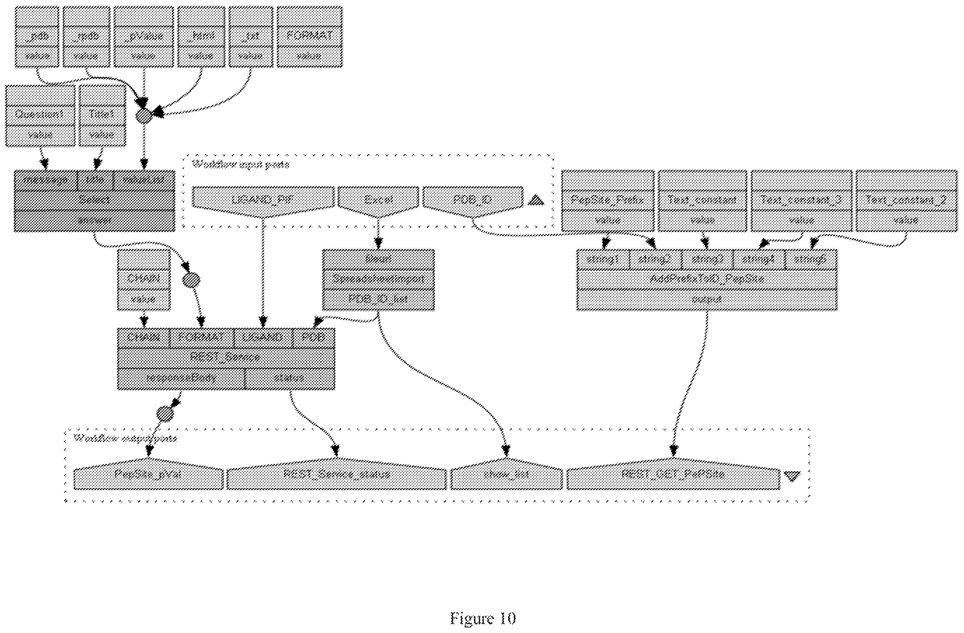

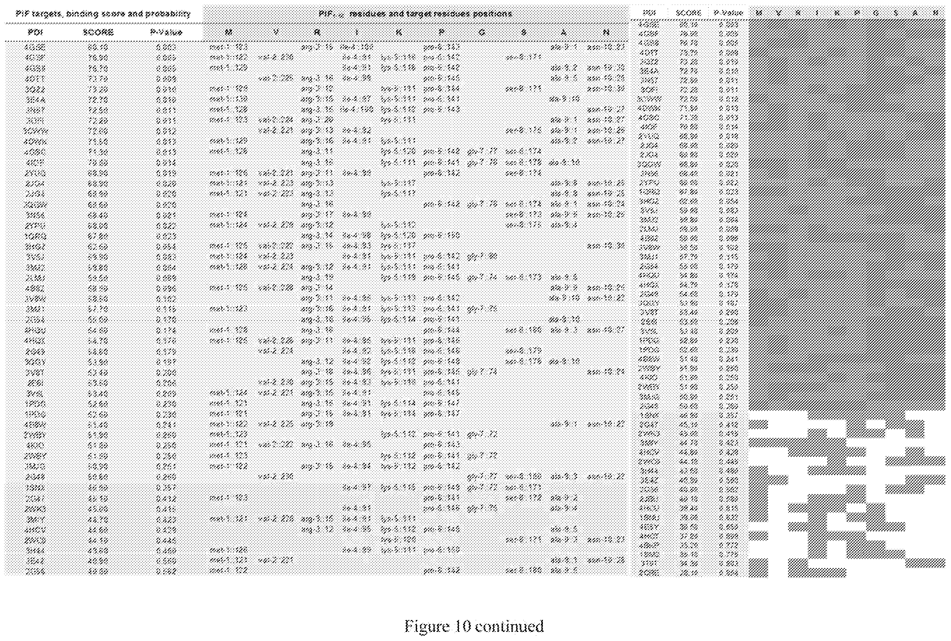

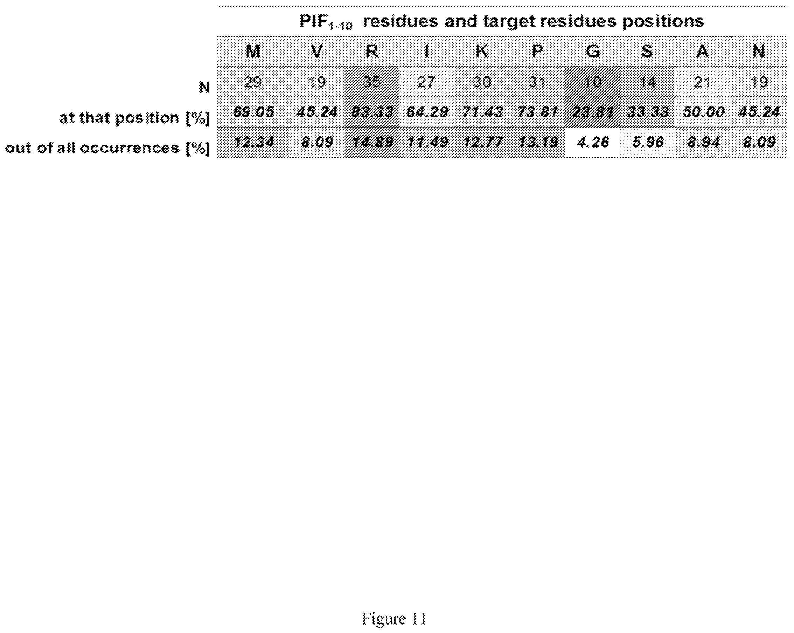

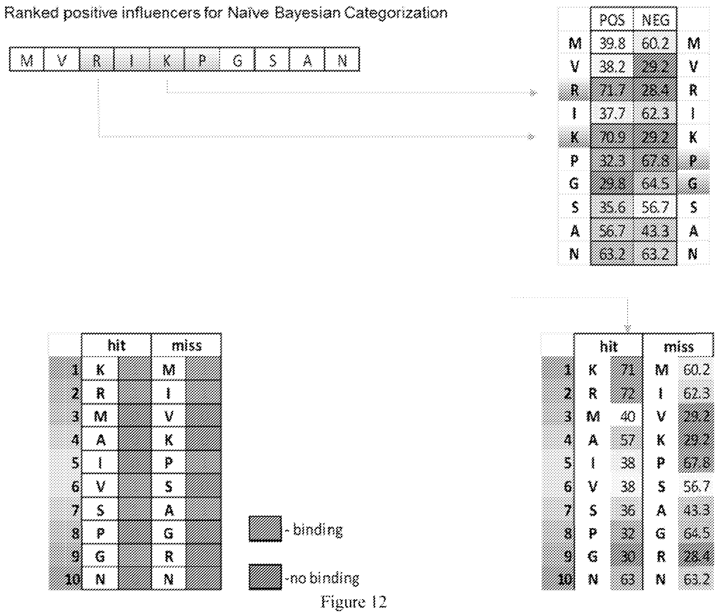

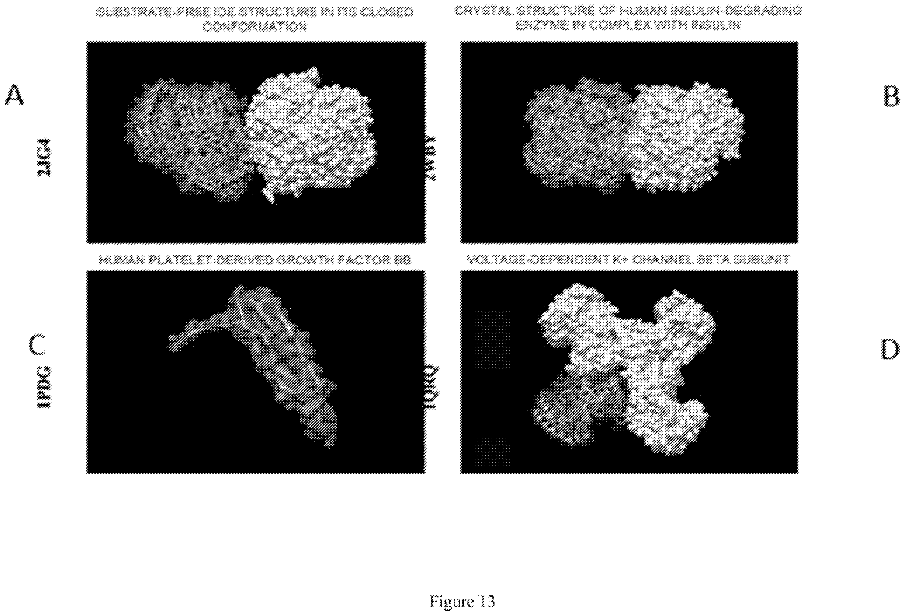

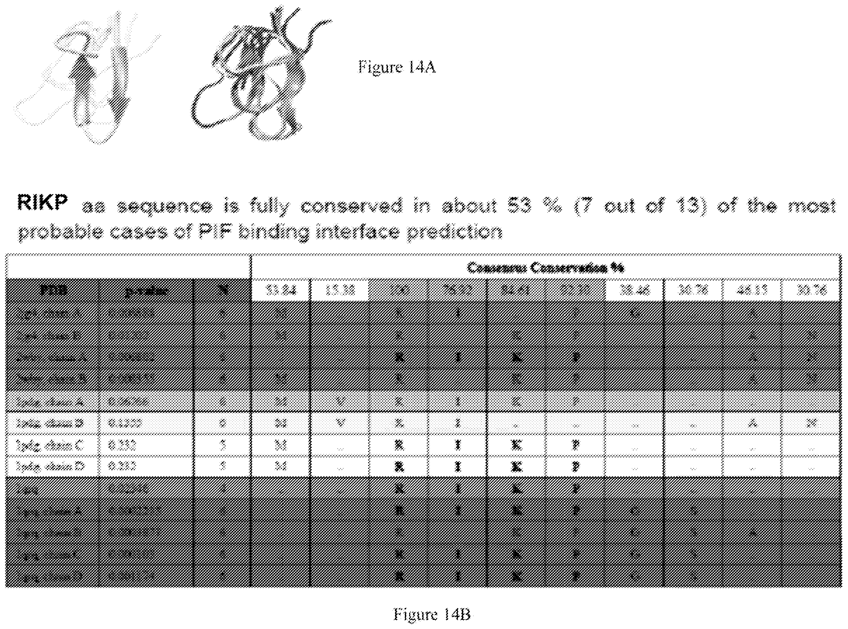

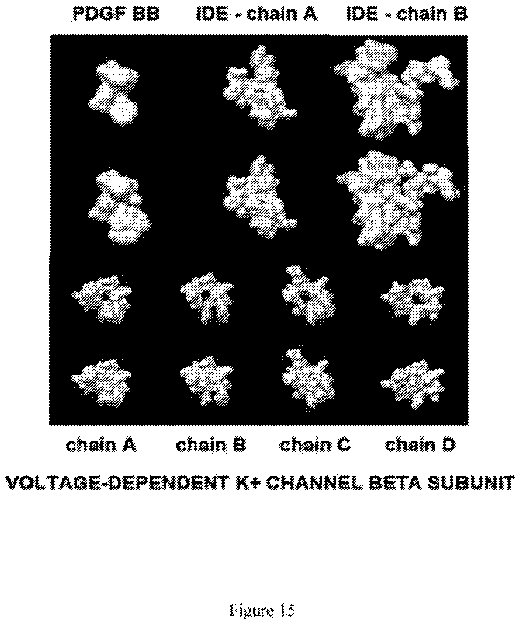

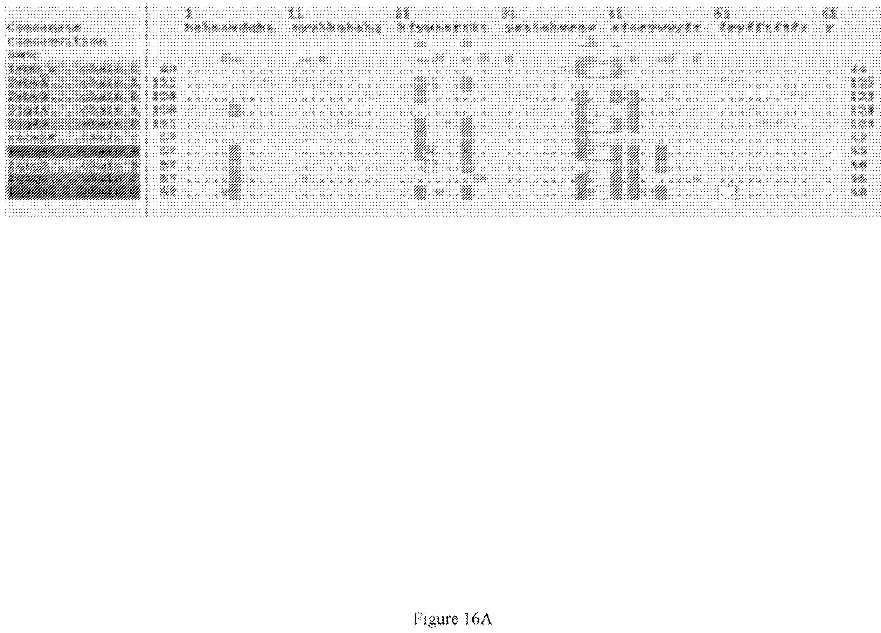

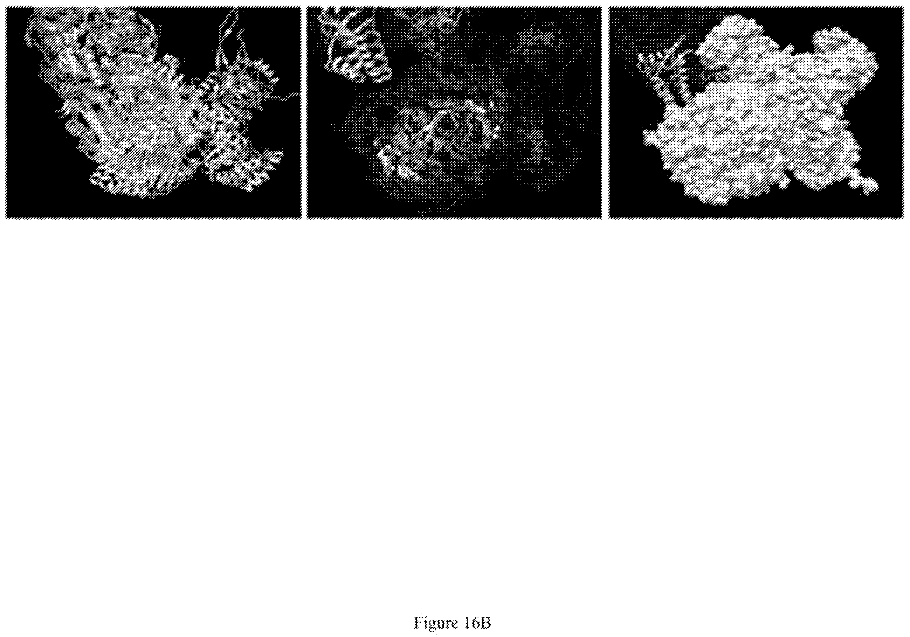

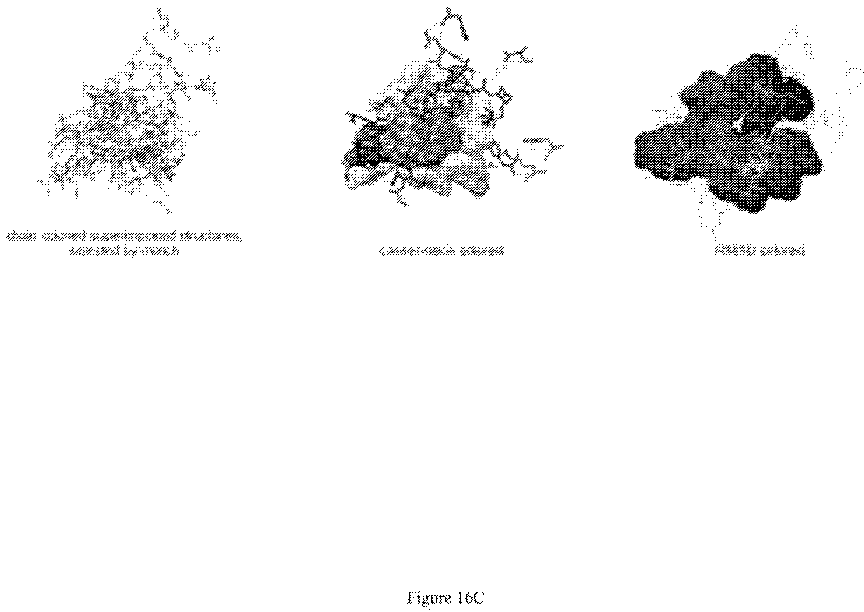

To determine PIF binding to these multiple diverse targets, the first ten most probable targets based on their CI p-value from the ProtoArray^{®} screening were selected for further analysis; of them, four were used for structural analysis of the PIF binding pocket, and all ten were used for structural binding screening. Using data from crystal structural analysis available at the PDB database (see **Figure 9****),** the ten positive PIF targets and 8990 negative targets' corresponding PDB structural data files and generated models **(****Figure 10****)** were obtained. Of the ten positive targets, 200 PDBs were retrieved. Of the negative targets, to the 14000 PDB files, only 2500 PDB models were unique. These 2500 PDB models were used further in the screening of PIF binding amino acid core. Taverna bioinformatics workbench was used to automate REST web service requests to PepSite 2 for scanning multiple crystallography models for peptide binding **(****Figure 10****).** According to this simple pattern of binding probabilities model, the PIF sequence participating in the binding was M¹ "R³I⁴K⁵P⁶ **(****Figure 11****).** Then a probabilistic definition of the peptide core residues supporting the binding interface was built **(****Figure 12****).** The probabilistic interface accounted R³K⁴ and A⁹N¹⁰ as the most important PIF sequence in positive vs. negative hits of PIF. Further, a structurally-based approach was used to predict the PIF binding interface characteristics. **Figure 13** shows PIF targets structures A-D, wherein cartoon ribbon and surface rendering of the four most prominent PIF targets were identified using ProtoArray^{®} Human Protein Microarrays v5.0. Orange represents alpha spiral, violet represents beta sheet, and gray tubes represent coiled coils stmctures. **Figure 13A** shows the IDE-free format. **Figure 13B** shows IDE complexed with insulin. **Figure 13C** shows PDGFb. **Figure 13D** shows Kvl.3b. **Figure 14** shows that PIF binds to its targets using its R-I-K-P sequence. **Figure 14A** shows two synthetic images of PIF PDB stmctures modeled using PepSite, superimposed in transparent mode emphasizing the solid beta sheet PIF structure. The green beta-sheet corresponds to conserved consensus PIF sequence (aligned). **Figure 14B** shows a table representing PIF amino acid residue configuration with the highest probability for four targets binding out of 11 putative targets. Color coding is based on the p-value of the structure. Consensus conservation greater than 70% is highlighted in green. The models were used together with putative PIF peptide tertiary structure models **(****Figure 14A****)** and the PepSite server to *in silico* predict the putative binding interfaces where these molecules interact with PIF proteins. The generated PIF peptide stmctures had one variant with defined beta-sheet structure and multiple others represented as coiled coil stmctures. In almost all cases, PIF would possibly have a coil at its intermediate part of the sequence. The PIF peptide binding scenarios estimated by the PepSite server were downloaded as PDB files describing the PIF residues and the target amino-acid residues that together would form a binding interface. Aligning the PIF peptide residues of the highest probability binding interface models **(****Figure 14B****)** revealed the PIF consensus sequence R-I-K-P (arg3, ile4, lyss, pro6) that was represented in full in more than 53% of the models. **Figure 15** shows PIF peptide binding surfaces represented by amino acid residues identified using peptide to protein binding modeling by the PepSite server of the top 4 protein targets identified using ProtoArray^{®} Human Protein Microarrays v5.0. The images represent the consensus empty or PIF binding residues clefts of 11 putative binding targets. Comparing the targets binding surface interfaces, cleft-like stmctures that were contained by all four possible targets were identified. To reveal a putative structural similarity that would explain the PIF multiverse effects, we performed structural alignment of the target molecules (using MultiPept server) and found that most of them co-align with the chain A of the voltage-dependent potassium ion channel regulatory beta-subunit tetramer(lQRQ). **Figure 16** shows PIF alignment with protein targets. **Figure 16A** shows multiple protein sequence alignment after multiple protein structural alignment and superposition (Chimera 1.8). **Figure 16B** shows (from left to right) multiple protein structural alignment of all four targets imaged as cartooned ribbons colored by protein model id; only matching structures are in opaque, while non-matching structures are colored dark-gray and transparent; the surface renderings of the superimposed protein structures are colored by model. Superposition of the residue atom structures and their molecular surfaces revealed binding pocket (cleft) having relatively conserved bottom and similar among the different targets boundaries. **Figure 16C** shows (from left to right) superimposed atom chains of the structures that have the highest level of structural alignment; the same structures are superimposed by the surfaces of those residues that match and are colored by the level of conservation, blue being the lowest; the same structures are colored by the RMSD, blue being the lowest distance, and red the highest distance.

The PIF peptide binds diverse structures that could be summarized by the linearized version of their binding pockets using the following consensus sequence: WX₁WX₂X₃X₄REWFX₅X₆X₇W (Trp-X-Trp-X-X-X-Arg-Glu-Trp-Phe-X-X-X-Trp; SEQ ID NO:28). This sequence is result of the structural superposition of the different binding pockets predicted by the PepSite 2 sever for the following targets: Kvl.3 potassium channel (chain A, chain B, chain C, chain D, regulatory subunit), Insulin Degrading Enzyme (open state bound to Insulin and free closed state), Protein disulfide-isomerase (reduced and oxidized, chains A and B), and HSP70 (chains A and B). Each chain was evaluated separately and 10 predictive models were created. Using intermediate structural and sequence alignment (Smith-Waterman algorithm, BLOSUM62 matrix, secondary structure consideration of 80 %) 2 out of every 10 models were selected to cover for all amino acid residues potentially binding to PIF in the PepSite 2 predictions. A structural alignment using Smith-Waterman algorithm with the same parameters over the entire set of models representing all the targets was done **(****Figures 17A** **and** 17B) using Match-Maker algorithm of UCSF Chimera 1.9. The structural representation is too diverse as it resembled distinct non-homologous proteins that do not share the same amino acid sequences, but rather have similar binding pockets properties in terms of hydrophobicity/electrostatic potential/H-bonds formation/van der Waals forces, etc. We ran on top of this structural interface alignment a sequence alignment using MUSCLE algorithm and obtained a linearized version of each structurally distinct and differently distributed in space amino acid PIF target interface residue. Thus we obtained the following signature for the maximally 23 amino acids participating in the PIF binding pocket: (1)--------w-w---rewf---w-(23), where their constellation could be represented at the following relative linear positions: Trp-9, Trp-11, Arg-15, Glu-16, Trp-17, Phe-18, Trp-22 **(****Figures** 17C **and** **D).** **Figure** 17C represents this signature and the corresponding per target model amino acids cored in ClustalX alignment format, while Figure 17D represents the same amino acids, but colored in Residue charge color coding. The analysis demonstrates similar in polarity (within structurally closer targets) residues to form the amino acid pattern of the binding pocket.

This target interface signature is referred to herein as WX₁WX₂X₃X₄REWFX₅X₆X₇W (Trp-X-Trp-X-X-X-Arg-Glu-Trp-Phe-X-X-X-Trp; SEQ ID NO:28, wherein each X can independently be any amino acid) and corresponds to the linearized representation of the summarized sterical binding pocket of the PIF targets.

### PIF Peptide Binds to Cortisol Binding Site, Possibly Competing with Cortisol Binding Site

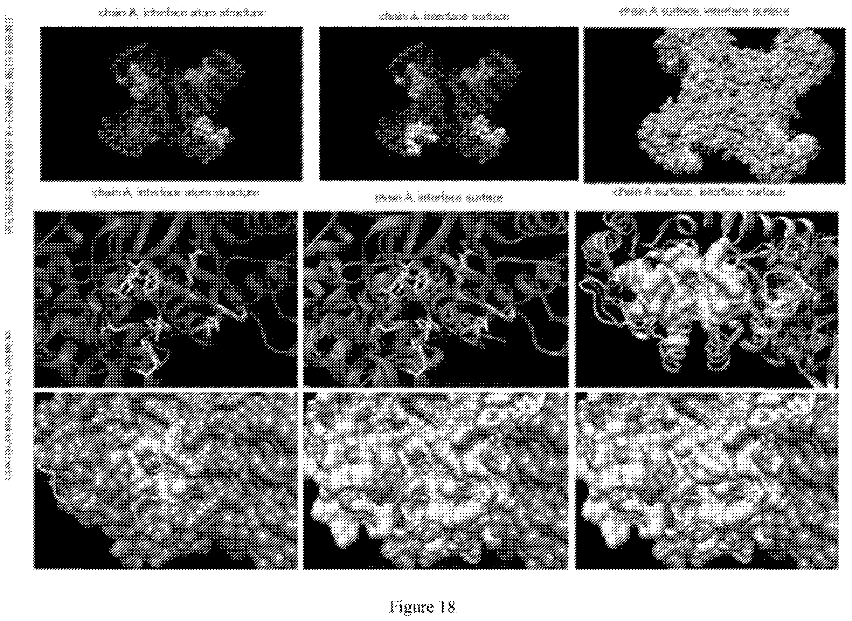

To *in silico* validate this phenomenon a cortisol-bound beta-subunit (PDB 3EB4) crystal structure data obtained from rat protein studies was selected and compared to the chain A of the voltage dependent potassium ion channel subunit (PDB 1QRQ). **Figure 18** shows a PIF peptide binding validation scenario. There is a cartoon ribbon diagram of chain A of the voltage-dependent K ion channel subunit (PDB 1QRQ), with PIF peptide binding residues depicted as interface surface and target-site binding residues depicted as atom stmctures/ribbons (1), surfaces (2), and the entire four-chain molecule is represented as surface render (3). The same molecule ribbon diagram and PIF peptide binding residues atom stmctures (1) were aligned with the K ion channel bound to Cortisol (PDB 3EB4) (2) and its residue's binding surface rendered in pink (3). This binding surface represented the bottom of putative PIF peptide binding cleft, colored in cyan (4), that was intersected with part of the surface of the cortisol-bound ion channel (3EB4), colored in light blue (5). The cortisol-bound channel crystal structure derived full surface render revealed that part of the PIF peptide binding cleft is occupied by residues that change its conformation (6). By modeling the binding surface **(****Figure 18****),** it was established that Cortisol binding actually deforms the PIF binding pocket **(****Figure 18****)** and underlines the structural difference between the unbound and Cortisol bound regulatory b subunit of the channel.

### Validation of the R-I-K-P Consensus of the PIF Peptide Binding Sequence

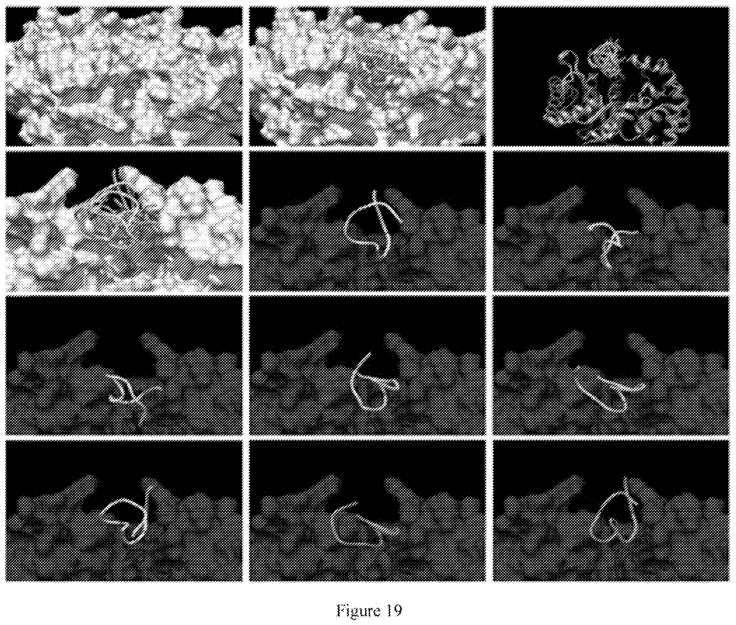

The validation of the consensus PIF peptide binding interface was continued by using flexible peptide-to-protein docking that estimated the PIF peptide near to real binding conformation and refined docking to the cleft. **Figure 19** shows a PIF peptide binding validation scenario, flexible peptide docking using FlexPepDock. There is a surface render diagram of PIF peptide binding cleft in chain A of the voltage-dependent K ion channel subunit (PDB 1QRQ), non-occupied (1), or all PIF peptide structures superimposed occupied (2). The same chain is represented as secondary structure color coded ribbon diagram and PIF peptides coiled-coil tubes of all flexible docking models in (3). There is a close view of the binding surface (4), and a representation of every individual model of PIF peptides (5-12) is also shown. The green contour over PIF peptides represents the consensus binding sequence. PIF peptide conformation is determined *in silico* not by its sequence alone, but rather obtained after flexible transformations in conditions of pocket binding, demonstrated characteristic form, identifying a specific coil that is always present and facing the bottom of the binding pocket surface. Mapping of the consensus R-I-K-P sequence obtained using PepSite to this model revealed that it is always present and binds the pocket bottom.

### Validation of IDE as proof-of-concept of PIF binding interface

### Validation through in silico mutagenesis and flexible docking

FlexPepDock provides the opportunity to model the native peptide flexibility and fluctuation when presented near the protein binding groove(s) detected by PepSite 2. Further, *in silico* mutagenesis of the refined peptide-protein binding interface allows us to predict with some probability the putative amino acids that upon mutation would break the interface or make it stronger *(minimally active motif* definition). To increase the probability of the prediction, we generated new models reflecting the *in silico* predicted mutation. These models were further subject to re-docking by FlexPepDock. The mutation changes the binding and ultimately result in higher energy models. By analyzing the energies of binding of *high-resolution flexible peptide docking* models of wild type (wt) peptide vs. those of *in silico* mutagenesis predicted loss-of-function peptides, we validated the importance of the core peptide sequence, further defining the most important amino acids in the lead peptide (wt) necessary for its biological function.

High-resolution peptide docking of PIF to its potential targets was done using the Rosetta FlexPepDock server. A PDB encoded model of the *de novo* PIF model was supplied in close proximity and its location was specified by the PepSite 2. For the protein targets PDI and HSP binding PIF structure was predicted *ab initio* using the Pep-Fold server. FlexPepDock allows full flexibility to the peptide and side-chain flexibility to the target protein, thus providing accurate refinement of the peptide structure, starting from up to 5.5 RMSD of the native conformation. (RMSD - root-mean-square deviation, is the measure of the average distance between the atoms [usually the backbone atoms] of superimposed proteins).

An analysis of PIF specificity of target binding interface was done using the BeAtMuSiC server. We used a single aa *in silico* mutagenesis and validation to estimate interface energy change generated by mutated PIF *de novo* models (PepFold). Re-docking by FlexPepDock was compared with the initial PIF-target docking model as a reference. The estimated bbRBMS (Root Mean Square Deviation of peptide backbone heavy atoms only) change between wt PIF and mutated PIF docking models and their interface energy were compared for the highest Total Rosetta energy per model. This determined the total energy of the PIF-protein complex less the energy of the binding partners when separated.

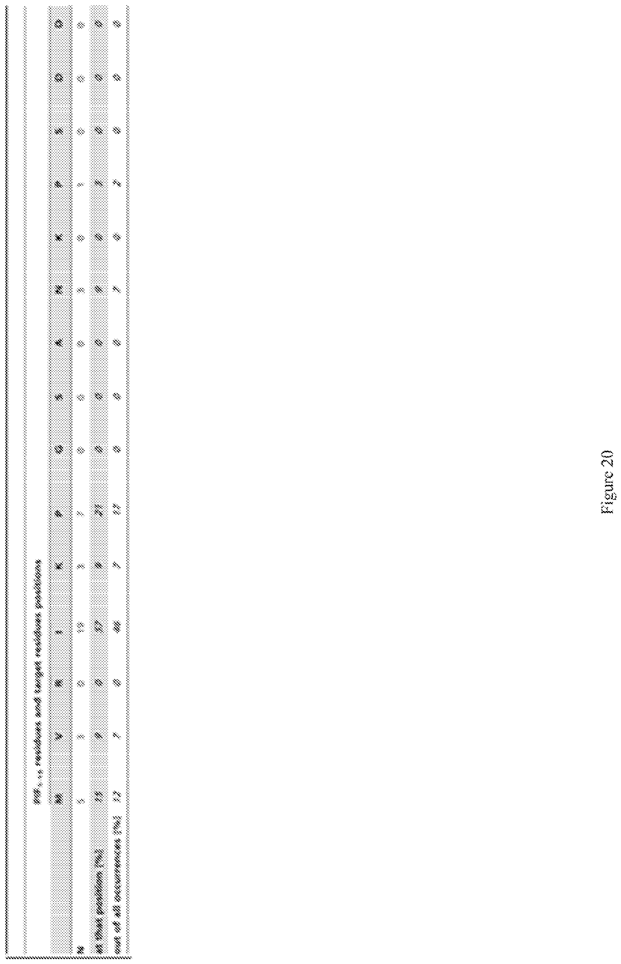

The high-precision docking models obtained were further used for *in silico* mutagenesis. We virtually mutated every single amino acid from the entire PIF1-15 sequence participating in the model interface and assessed the change in free energy of the ligand-receptor bond, thus obtaining the amino acids having the highest importance for the binding of PIF to its protein targets. This was achieved using the algorithm implementation of BeaTMusiC server. We statistically evaluated the frequency of residue positions having highest energy impact (Figure 20). Here again the R-I-K-P sequence had the most frequent employment in the binding interfaces assessed through their *in silico* mutations (data not shown).

The FlexPepDock modeled flexible docking to human IDE suggested that PIF mutation resulted in reduced interface and model energy, but also that the stabilization of the models came into place only when mutated PIF carbon backbone increased its root mean square distance (as represented by the root mean square of the carbon backbone - rmsBB) in the model from 4.5Å to more than 6 Å. The distance between the first amino acid in docked wild type PIF sequence - Met¹ₘᵤₜ₋₁ and the mutated PIF (mutant - 1) Met¹ₘᵤₜ₋₁, where Pro⁶_{wt} is mutated into a different amino acid is 13 Å.

### PIF Peptides Exert Antagonist Effects on IDE-Dependent Pathways in Naive and Coactivated PBMCs

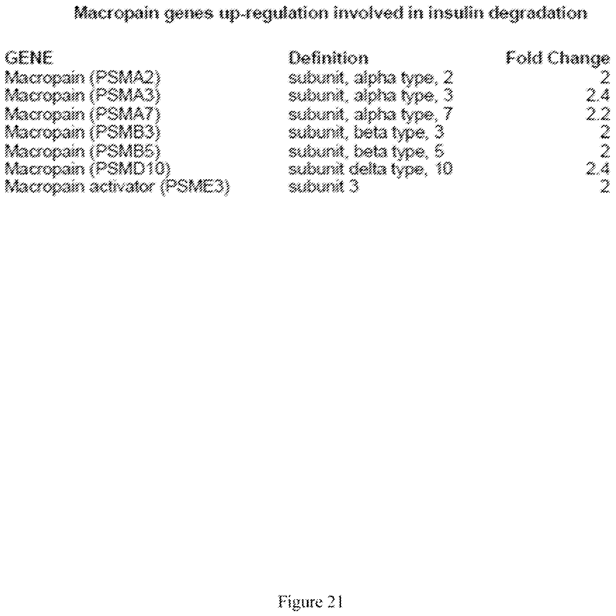

ProtoArray^{®} demonstrated that IDE is the prime PIF peptide target; however, the encoding gene was not affected by exposure to PIF peptides. In PBMCs several insulin-related genes were affected by PIF peptides, their expression increased in naive cells and decreased following co-activation. In naive cells, PIF peptides upregulated insulin-like growth factor-1 (IGF1) (2.8 fold) where the encoded protein interacts and upregulates IDE activity. The IGF-binding protein-3 (IGF219 BP3) was upregulated (4.6 fold) which amplifies IGFs activity. In contrast, following co-activation, PIF downregulated the insulin receptor (INSR) (-2.8 fold) and interacting gene IGFBP6 (-2.4 fold), while macropain 26S subunit, non-ATPase, 10 (PSMD10)was upregulated (2.4 fold). The encoded protein is involved in ATP-dependent degradation of ubiquitinated proteins by UDP-glucose dehydrogenase (UGDH) (-2 fold) as well as insulin beta subunit degradation. Evidence of PIF's significant role in this pathway is shown in **Figure 21****,** where the PIF peptide upregulated six additional macropain genes family members supporting negative insulin regulation and insulin degradation.

### PIF Modulates Differently K+ and Na+ Channels Related to Gene Expression in PBMCs

ProtoArray^{®} studies demonstrated that PIF targets Kvl.3b channels that control the alpha pore. However, these genes expression in PBMCs were only minimally affected by exposure to PIF peptides. Both K+ and Na+ are prime ions that regulate cellular function. The only K+ channel gene significantly upregulated is in naive cells K+ channel (KCNK4) (2.8 fold) a K(2P) WIK-related arachidonic acid-activated channel. The counter-ion genes SCNA1, (Na+ voltage-gated Al) which promote Na+ flux increased (7.8-fold) and SCN12 (5.4-fold). The former is expressed by B lymphocytes. In contrast, following activation, ATP1B1 expression decreased ( -2.4-fold) a Na+,K+-ATPase ion pump which maintains their flux across the cell membrane.

### PIF Targets Platelet-Derived Growth Factor-B(PDGFP)

This peptide is a potent mitogen that binds the PDGFa receptor in PBMC which interacts avidly with osteonectin decreased (-2.2 fold) by PIF peptide in activated cells. Osteonectin binds calcium, copper, collagen, and thrombospondin.

### PIF Both Targets and Upregulates Tissue-Specific Transplantation Antigen P35

### Expression (FX enzyme)

FX is a multi-functional amino acid size enzyme involved in two step NADP-dependent conversion of GDP-4-dehydro-6- deoxy-D-mannose to GDP-fucose, through epimerase and reductase reactions. This complex enzyme has an NADP as well as a polypeptide binding site. Notably, in activated cells PIF upregulates TSTA3 (FX), (3.6 fold) and downstream MCM2 gene expression (2.8 fold) which initiates DNA replication enabling entry to the S phase. PIF may control the NOTCH pathway by lowering ADAM 17, a tumor necrosis factor-alpha converting enzyme (-2.2-fold). Of note, that PBMC co-activation alone (without PIF) did not increase the same gene expression. Data generated support intimate interaction between PIF's effect on peptide binding and gene expression.

### PIF Targets and May Upregulate ITK Expression in PBMC

ITK is an IL2-induced tyrosine kinase which promotes T-cell proliferation. PIF mildly upregulates ITK (1.6-fold) in naive cells. However, in activated cells downstream CDC42 (-2.2-fold) decreased as well as the ITK effector gene which also decreased SPEC 1 (-2.2-fold). In contrast, in naive cells ITK expression mildly decreased.

### Additional PIF Minor Binding Targets (Cass4, NEK11, DAPK3, ODF2L Clorf87).

Cass4, another PIF peptide target, is a member of the Cass family. It regulates PTK2/FAK1 activity, acting as a scaffold to regulate protein complexes controlling migration and chemotaxis, apoptosis, cell cycle, invasion and survival. PIF peptides also bind the kinase NEK11, involved in DNA replication significant for response to environmental stress. In activated cells, PIF upregulates the cell division cycle 25A (CDC25A) (6-fold), a phosphatase, essential for cyclin-dependent kinase activation and cell-cycle progression. PIF further targets another kinase, DAPK3, (ZIPK), serine/threonine kinase, an apoptosis regulator and tumor suppressor. DAPKs share high homology in their catalytic site where Histone H3 protein is the major substrate. The histone octamer is composed of pairs of four core histones (H2A, H2B, H3, and H4). PIF decreases H2A (4.2-fold) O/L (2-fold) and H3F3A (-3-fold). Binding of PIF peptide to the outer dense fiber of the sperm tail-2 (ODF2L) part of the cytoskeleton was also observed. The protein is necessary for mother centriole distal/subdistal appendages and primary cilia generation. It may interact with R2 ErbB-3 R2 (c-erbB-3) which was (4.8-fold) upregulated by PIF in naive cells. The chromosome fragment (Clorf87) is a PIF peptide target for which the encoded protein is likely a calcium-binding protein. This data further reveals the diversity of PIF peptide binding targets.

### PIF Peptide Targets are Mainly Intracellular Proteins and Expressed by Immune Cells and Platelets

Due to the diversity of the PIF peptide targets identified, it was important to evaluate which cells express them and in which cell compartments they are located. Gene cards databases were used for this analysis. IDE is expressed mainly in immune cells, microglia, platelets and astrocytes. It is an intracellular enzyme which is also present in a secreted form. Kvl.3b channels are mainly present in immune cells and platelets at a cytoplasmic location. PDGFb is a platelet-secreted protein found in the circulation. It may also be secreted by the liver and heart. FX enzyme is present in immune cells' cytoplasm. ITK and DAPK3 are kinases found in immune cells and platelets' cytoplasm and nuclei. NEK11 kinase is found in platelets' nuclei. ODF2L location is platelets' centrioles. Clorf87 is expressed in platelets; its calcium binding effect is likely intracellular. Such data substantiates that PIF's primary interaction is with immune cells/platelets targeting mostly intracellular proteins.

### EXAMPLE 3

### Pump Administration of PIF Peptides Prevents Anesthesia-Induced Abortion and Decreases LPS-Induced Abortion

30 Swiss female mice and 30 Swiss male mice, 8-10 weeks old, were matched overnight for three consecutive days. The next morning, the presence of a vaginal plug was verified. 20 female Swiss mice positive for the vaginal plug were selected for the experiment.

Mice at days 1-3 post-insemination (pi) were anesthetized with ketamine and domitor 200 µl. 20 Alzet pumps filled with 100 pi of PBS or PIF 4.2 mg/ml (1 mg/kg/die) were inserted subcutaneously in the back at shoulder blade level. The mice were located singularly in separated cages. 2 mice with PBS pumps died after the insertion. At days 6-8 pi, the mice were treated with LPS 0,lpg/gr or with PBS. At days 14-16 pi, the mice were euthanized and investigated for vital fetuses and for aborts. Mice with no vital fetuses nor signs of abortion were considered to carry 10 aborts for statistical purposes. The groups were compared with Fisher exact test, p<0.05 was considered significant (Figure 22). The results demonstrated that pump administration of PIF peptides prevents anesthesia-induced abortion and decreases LPS-induced abortion.

## Claims

1. A cell comprising an exogenous deoxyribonucleic acid (DNA) sequence encoding a preimplantation factor (PIF) peptide, wherein the PIF peptide consists of the sequence of MX₁RIKPX₂X₃A, wherein X₁, X₂ and X₃ are independently any amino acid, provided that said PIF peptide is not SEQ ID NO: 1, wherein the cell is obtained by a method of modifying the cell to express the exogenous DNA.

2. The cell of claim 1, wherein the DNA sequence comprises SEQ ID NO:17.

3. The cell of claim 1 or 2, wherein the DNA sequence further encodes for at least one fusion tag.

4. The cell of claim 3, wherein the at least one fusion tag is connected to the PIF peptide by a linker.

5. The cell of claim 3, wherein the fusion tag is selected from the group consisting of FLAG, human influenza hemagglutinin (HA) and an myc tag.

6. The cell of claim 1 or 2 modified to express the preimplantation factor (PIF) peptide, for use in the treatment of a condition selected from: a pregnancy-related disorder, an immune disorder, an autoimmune disease, an inflammatory disorder, a disorder caused by neuroinflammation, diabetes, exposure to radiation, an intracellular disorder, atherosclerosis or complications thereof, brain injury and combinations thereof.

7. The cell of claim 1 or 2 modified to express the preimplantation factor (PIF) peptide, for use in the treatment according to claim 6, wherein said condition is a pregnancy-related disorder and the treatment requires one or more of: enhanced implantation of one or more embryos, increased endometrial receptivity, promoted trophoblast invasion, promoted *in vitro* fertilization, decreased incidence of miscarriage, or maintenance of a pregnancy through term, and combinations thereof.

8. The cell of claim 1 or 2 modified to express the preimplantation factor (PIF) peptide, for use in the treatment according to claim 6, wherein said condition is an immune disorder selected from the group consisting of HIV, AIDS, SCIDS, bacterial peritonitis, chemical peritonitis, allergy, asthma, pollinosis, and atopy.

9. The cell of claim 1 or 2 modified to express the preimplantation factor (PIF) peptide, for use in the treatment according to claim 6, wherein the condition is exposure to radiation and the treatment requires prevented development of acute radiation syndrome; prevented or reversed changes in chemokines, cytokines, and other inflammatory agents after exposure to radiation; improved immune cell function and recovery following exposure to radiation; normalized hepatic, colon and other organs' function following exposure to radiation; prevented stochastic effect of radiation; and combinations thereof.

10. The cell of claim 1 or 2 modified to express the preimplantation factor (PIF) peptide, for use in the treatment according to claim 6, wherein the condition is neuroinflammation and the treatment requires treated brain inflammation; reversed advanced brain ischemia and inflammation following a stroke; and combinations thereof.

11. The cell of claim 1 or 2 modified to express the preimplantation factor (PIF) peptide, for use in the treatment according to claim 6, wherein the condition is atherosclerosis or said complications thereof and the treatment requires prevented or reduced atheromatous plaque hemorrhage or rupture; inhibited or prevented complications of aortic diseases or vascular aneurysms; inhibited or prevented vascular stenosis or occlusion; and combinations thereof.

12. The cell of claim 1 or 2 modified to express the preimplantation factor (PIF) peptide, for use in the modulation of the immune system, wherein modulation is selected from: decreased immune response after a transplantation; increased engraftment of any one of solid organs, heart, liver, bowel, lung, and kidney, transplanted bone marrow, and combinations thereof; improved acceptance of transplanted bone marrow without deleterious graft-versus-host disease while the beneficial graft-versus-leukemia effect is maintained; improved acceptance and function of transplanted stem cells; preserved graft-versus leukemia effect of transplanted bone marrow and reduced graft-versus-host disease; cancer treatment; and combinations thereof.

13. A pharmaceutical composition comprising the cell of claim 1 or 2 and a pharmaceutically acceptable carrier.

14. The cell of claim 1 or 2 for use as a medicament.

## Patentansprüche

1. Zelle, umfassend eine exogene Desoxyribonukleinsäure-(DNA)-Sequenz, die ein Präimplantationsfaktor-(PIF)-Peptid codiert, wobei das PIF-Peptid aus der Sequenz MX₁RIKPX₂X₃A besteht, wobei X₁, X₂ und X₃ unabhängig voneinander für eine beliebige Aminosäure stehen, mit der Maßgabe, dass das PIF-Peptid nicht SEQ ID NO: 1 ist, wobei die Zelle durch ein Verfahren zur Modifizierung der Zelle zur Expression der exogenen DNA erhalten wird.

2. Zelle nach Anspruch 1, wobei die DNA-Sequenz SEQ ID NO:17 umfasst.

3. Zelle nach Anspruch 1 oder 2, wobei die DNA-Sequenz zudem mindestens eine Fusions-Markierung codiert.

4. Zelle nach Anspruch 3, wobei die mindestens eine Fusions-Markierung durch einen Linker mit dem PIF-Peptid verbunden ist.

5. Zelle nach Anspruch 3, wobei die Fusions-Markierung ausgewählt ist aus der Gruppe bestehend aus FLAG, menschlichem Influenza-Hämagglutinin (HA) und einer myc-Markierung.

6. Zelle nach Anspruch 1 oder 2, die so modifiziert ist, dass sie das Präimplantationsfaktor-(PIF)-Peptid exprimiert, zur Verwendung bei der Behandlung eines Zustands, der ausgewählt ist aus einer schwangerschaftsbedingten Störung, einer Immunstörung, einer Autoimmunerkrankung, einer entzündlichen Störung, einer durch Neuroinflammation verursachten Störung, Diabetes, Strahlenexposition, einer intrazellulären Störung, Atherosklerose oder deren Komplikationen, Hirnverletzung und Kombinationen davon.

7. Zelle nach Anspruch 1 oder 2, die so modifiziert ist, dass sie das Präimplantationsfaktor-(PIF)-Peptid exprimiert, zur Verwendung bei der Behandlung nach Anspruch 6, wobei der Zustand eine schwangerschaftsbedingte Störung ist und die Behandlung eine oder mehrere Maßnahmen von verbesserter Implantation eines oder mehrerer Embryonen, erhöhter endometrialer Empfänglichkeit, geförderter Trophoblasteninvasion, geförderter In-vitro-Fertilisation, verringerter Inzidenz von Fehlgeburten oder Aufrechterhaltung einer Schwangerschaft bis zum Ende der Schwangerschaft und Kombinationen davon erfordert.

8. Zelle nach Anspruch 1 oder 2, die so modifiziert ist, dass sie das Präimplantationsfaktor-(PIF)-Peptid exprimiert, zur Verwendung bei der Behandlung nach Anspruch 6, wobei der Zustand eine Immunstörung ist, die ausgewählt ist aus der Gruppe bestehend aus HIV, AIDS, SCIDS, bakterieller Peritonitis, chemischer Peritonitis, Allergie, Asthma, Pollinose und Atopie.

9. Zelle nach Anspruch 1 oder 2, die so modifiziert ist, dass sie das Präimplantationsfaktor-(PIF)-Peptid exprimiert, zur Verwendung bei der Behandlung nach Anspruch 6, wobei der Zustand eine Strahlenexposition ist und die Behandlung die verhinderte Entwicklung eines akuten Strahlensyndroms, die verhinderte oder umgekehrte Veränderungen bei Chemokinen, Zytokinen und anderen Entzündungsstoffen nach der Strahlenexposition, die verbesserte Funktion der Immunzellen und Erholung nach der Strahlenexposition; die normalisierte Funktion der Leber, des Dickdarms und anderer Organe nach der Strahlenexposition; die verhinderte stochastische Wirkung der Strahlung; und Kombinationen davon erfordert.

10. Zelle nach Anspruch 1 oder 2, die so modifiziert ist, dass sie das Präimplantationsfaktor-(PIF)-Peptid exprimiert, zur Verwendung bei der Behandlung nach Anspruch 6, wobei der Zustand eine Neuroinflammation ist und die Behandlung eine behandelte Gehirnentzündung; die umgekehrte fortgeschrittene Gehirnischämie und -Entzündung nach einem Schlaganfall; und Kombinationen davon erfordert.

11. Zelle nach Anspruch 1 oder 2, die so modifiziert ist, dass sie das Präimplantationsfaktor-(PIF)-Peptid exprimiert, zur Verwendung bei der Behandlung nach Anspruch 6, wobei es sich bei dem Zustand um Atherosklerose oder deren Komplikationen handelt und die Behandlung eine verhinderte oder reduzierte atheromatöse Plaque-Blutung oder -Ruptur, gehemmte oder verhinderte Komplikationen von Aortenerkrankungen oder vaskulären Aneurysmen, gehemmte oder verhinderte vaskuläre Stenose oder Okklusion und Kombinationen davon erfordert.

12. Zelle nach Anspruch 1 oder 2, die so modifiziert ist, dass sie das Präimplantationsfaktor-(PIF)-Peptid exprimiert, zur Verwendung bei der Modulation des Immunsystems, wobei die Modulation ausgewählt ist aus verringerter Immunreaktion nach einer Transplantation; erhöhter Transplantation von festen Organen, Herz, Leber, Darm, Lunge und Niere, transplantiertem Knochenmark und Kombinationen davon; verbesserter Akzeptanz von transplantiertem Knochenmark ohne schädliche Transplantat-gegen-Wirt-Krankheit, während der vorteilhafte Transplantat-gegen-Leukämie-Effekt erhalten bleibt; verbesserter Akzeptanz und Funktion von transplantierten Stammzellen; erhaltenem Transplantat-gegen-Leukämie-Effekt von transplantiertem Knochenmark und verringerter Transplantat-gegen-Wirt-Krankheit; Krebsbehandlung; und Kombinationen davon.

13. Pharmazeutische Zusammensetzung, umfassend die Zelle nach Anspruch 1 oder 2 und einen pharmazeutisch akzeptablen Träger.

14. Zelle nach Anspruch 1 oder 2 zur Verwendung als Medikament.

## Revendications

1. Cellule comprenant une séquence d'acide désoxyribonucléique (ADN) exogène codant pour un peptide de facteur de préimplantation (PIF), le peptide PIF étant constitué de la séquence de MX₁RIKPX₂X₃A, X₁, X₂ et X₃ étant indépendamment un quelconque acide aminé, à la condition que ledit peptide PIF ne soit pas la SEQ ID NO: 1, la cellule étant obtenue par un procédé de modification de la cellule pour exprimer l'ADN exogène.

2. Cellule selon la revendication 1, la séquence d'ADN comprenant la SEQ ID NO: 17.

3. Cellule selon la revendication 1 ou 2, la séquence d'ADN codant en outre pour au moins une étiquette de fusion.

4. Cellule selon la revendication 3, l'au moins une étiquette de fusion étant connectée au peptide PIF par un lieur.

5. Cellule selon la revendication 3, l'étiquette de fusion étant choisie dans le groupe constitué par FLAG, l'hémagglutinine de la grippe humaine (HA) et une étiquette myc.

6. Cellule selon la revendication 1 ou 2 modifiée pour exprimer le peptide de facteur de préimplantation (PIF), pour une utilisation dans le traitement d'une affection choisie parmi : un trouble lié à une grossesse, un trouble immun, une maladie auto-immune, un trouble inflammatoire, un trouble causé par une neuroinflammation, le diabète, une exposition à un rayonnement, un trouble intracellulaire, l'athérosclérose ou des complications de celle-ci, une lésion cérébrale et des combinaisons correspondantes.

7. Cellule selon la revendication 1 ou 2 modifiée pour exprimer le peptide de facteur de préimplantation (PIF), pour une utilisation dans le traitement selon la revendication 6, ladite affection étant un trouble lié à une grossesse et le traitement requérant l'un ou plusieurs parmi : l'implantation accrue d'un ou de plusieurs embryons, une réceptivité endométriale accrue, une invasion trophoblastique favorisée, une fertilisation *in vitro* favorisée, une incidence de fausse couche diminuée ou le maintien d'une grossesse à terme, et des combinaisons correspondantes.

8. Cellule selon la revendication 1 ou 2 modifiée pour exprimer le peptide de facteur de préimplantation (PIF), pour une utilisation dans le traitement selon la revendication 6, ladite affection étant un trouble immun choisi dans le groupe constitué par le VIH, le SIDA, SCIDS, une péritonite bactérienne, une péritonite chimique, une allergie, l'asthme, une pollinose et une atopie.

9. Cellule selon la revendication 1 ou 2 modifiée pour exprimer le peptide de facteur de préimplantation (PIF), pour une utilisation dans le traitement selon la revendication 6, ladite affection étant une exposition à un rayonnement et le traitement requérant l'empêchement du développement d'un syndrome d'irradiation aiguë ; des changements empêchés ou inversés de chimiokines, de cytokines et d'autres agents inflammatoires après une exposition à un rayonnement ; une fonction cellulaire immunitaire et une récupération améliorées à la suite d'une exposition à un rayonnement ; une fonction hépatique, du côlon et d'autres organes normalisée à la suite d'une exposition à un rayonnement ; un effet stochastique du rayonnement empêché ; et des combinaisons correspondantes.

10. Cellule selon la revendication 1 ou 2 modifiée pour exprimer le peptide de facteur de préimplantation (PIF), pour une utilisation dans le traitement selon la revendication 6, ladite affection étant une neuroinflammation et le traitement requérant une inflammation cérébrale traitée ; une ischémie et une inflammation cérébrales avancées inversées à la suite d'un accident vasculaire ; et des combinaisons correspondantes.

11. Cellule selon la revendication 1 ou 2 modifiée pour exprimer le peptide de facteur de préimplantation (PIF), pour une utilisation dans le traitement selon la revendication 6, ladite affection étant l'athérosclérose ou lesdites complications de celle-ci et le traitement requérant l'empêchement ou la réduction d'une hémorragie ou une rupture de plaque athéromateuse ; des complications inhibées ou empêchées de maladies aortiques ou d'anévrismes vasculaires ; une sténose ou une occlusion vasculaire inhibée ou empêchée ; et des combinaisons correspondantes.

12. Cellule selon la revendication 1 ou 2 modifiée pour exprimer le peptide de facteur de préimplantation (PIF), pour une utilisation dans la modulation du système immunitaire, la modulation étant choisie parmi : une réponse immunitaire diminuée après une transplantation ; une prise de greffe accrue de l'un quelconque parmi des organes solides, le cœur, le foie, l'intestin, le poumon et le rein, une moelle osseuse transplantée, et des combinaisons correspondantes ; une acceptance accrue de moelle osseuse transplantée sans maladie de greffon contre l'hôte délétère tandis que l'effet de greffon contre leucémie bénéfique est maintenu ; une acceptance et une fonction accrues de cellules souches transplantées ; un effet de greffon contre leucémie de moelle osseuse transplantée préservé et une maladie du greffon contre l'hôte réduite ; un traitement d'un cancer ; et des combinaisons correspondantes.

13. Composition pharmaceutique comprenant la cellule selon la revendication 1 ou 2 et un support pharmaceutiquement acceptable.

14. Cellule selon la revendication 1 ou 2 pour une utilisation en tant que médicament.
